(19) Europäisches Patentamt / European Patent Office / Office européen des brevets

(11) EP 1 261 585 B1

(12) EUROPÄISCHE PATENTSCHRIFT

(45) Veröffentlichungstag und Bekanntmachung des Hinweises auf die Patenterteilung:
**26.09.2007 Patentblatt 2007/39**

(21) Anmeldenummer: **00991219.7**

(22) Anmeldetag: **20.12.2000**

(51) Int Cl.:
***C07D 209/42*** (2006.01) ***A61P 25/04*** (2006.01)
***A61K 31/40*** (2006.01) ***C07D 209/14*** (2006.01)

(86) Internationale Anmeldenummer:
**PCT/EP2000/012974**

(87) Internationale Veröffentlichungsnummer:
**WO 2001/047885 (05.07.2001 Gazette 2001/27)**

# (54) SUBSTITUIERTE INDOL-MANNICHBASEN

SUBSTITUTED INDOLE MANNICH BASES

COMPOSES INDOL/BASES DE MANNICH SUBSTITUES

(84) Benannte Vertragsstaaten:
**AT BE CH CY DE DK ES FI FR GB GR IE IT LI LU MC NL PT SE TR**
Benannte Erstreckungsstaaten:
**LT LV SI**

(30) Priorität: **27.12.1999 DE 19963178**

(43) Veröffentlichungstag der Anmeldung:
**04.12.2002 Patentblatt 2002/49**

(73) Patentinhaber: **Grünenthal GmbH**
**52078 Aachen (DE)**

(72) Erfinder:
- **GERLACH, Matthias**
  **63636 Brachttal (DE)**
- **MAUL, Corinna**
  **52066 Aachen (DE)**

(74) Vertreter: **Kutzenberger, Helga et al**
**Kutzenberger & Wolff,**
**Theodor-Heuss-Ring 23**
**50668 Köln (DE)**

(56) Entgegenhaltungen:

| | |
|---|---|
| **EP-A- 0 768 301** | **WO-A-94/14770** |
| **WO-A-94/14771** | **WO-A-99/43654** |
| **DE-A- 2 329 430** | **FR-A- 2 181 559** |
| **US-A- 3 957 288** | **US-A- 4 341 402** |
| **US-A- 4 785 016** | **US-A- 4 855 448** |

- **CHEMICAL ABSTRACTS, vol. 98, no. 17, 25. April 1983 (1983-04-25) Columbus, Ohio, US; abstract no. 143226, VLASOVA, M. I. ET AL: "New synthesis of substituted gramines" XP002166632 & KHIM. GETEROTSIKL. SOEDIN. (1983), (1), 49-54 , 1983,**
- **HUFFMAN, ROBERT W. ET AL: "Reaction of indolenine salts with nucleophiles" J. AM. CHEM. SOC. , Bd. 89, Nr. 24, 1967, Seiten 6243-6251, XP000996698**
- **CHEMICAL ABSTRACTS, vol. 99, no. 3, 18. Juli 1983 (1983-07-18) Columbus, Ohio, US; abstract no. 22260, TABIDZE, D. M. ET AL: "Bisindoles. 15. Acid condensation of 2,2'-ethoxycarbonyl derivatives of bis(5-indolyl) oxide and bis(5-indolyl)methane with aromatic aldehydes" XP002166633 & KHIM. GETEROTSIKL. SOEDIN. (1983), (3), 365-8 , 1983,**

EP 1 261 585 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft substituierte Indol-Mannichbasen, Verfahren zu deren Herstellung, Arzneimittel enthaltend diese Verbindungen sowie die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie z.B. das Morphin, sind bei der Therapie starker bis sehr starker Schmerzen wirksam. Als unerwünschte Begleiterscheinungen weisen sie jedoch unter anderem Atemdepression, Erbrechen, Sedierung, Obstipation sowie eine Toleranzentwicklung auf.

Tramadolhydrochlorid - (1RS, 2RS) - 2-[(Dimethylamino)methyl]-1-(3-methoxyphenyl)-cyraohexanot - nimmt unter den zentral wirksamen Analgetika eine Sonderstellung ein, da dieser Wirkstoff eine starke Schmerzhemmung ohne die für Opioide bekannten Nebenwirkungen hervorruft. (J. Pharmacol. Exptl. Ther. 267, 33 (1993)). Nach weiteren schmerzhemmenden Mitteln wird weltweit geforscht.

In der Veröffentlichung Khim. Geterotsikl. Soedin. 1983 (1), 49 - 54, wird die Synthese substituierter Gramine beschrieben.

US 3 957 288 offenbart unter anderem die Verwendung von Indolen als Farbstoffe.

In WO 94/14770 und WO 94/14771 wird jeweils die Verwendung von Tryptamin-Analoga als 5-$HT_1$-Rezeptor-Agonisten gezeigt.

US 4 855 448 beschreibt 2-Amino-3-aroyl-γ-oxo-benzolbuttersäuren und -ester mit entzündungshemmender Aktivität.

In EP 0 768 301 werden N-(2-substituierte-3-(2-aminoethyl)-1H-indol-5-yl)-amide und deren Verwendung als 5-$HT_{1F}$-Agonisten offenbart.

Die Veröffentlichung J. Am. Chem. Soc. 1967, 243-251 beschreibt die Umsetzung von Indolenin-Salzen mit Nukleophilen.

Die Veröffentlichungen Synthesis 1996 , 883 - 887; Synthesis 1980, 728 - 730; J. Am. Chem. Soc. 1959, 2239-2242 Chem. Ber. 1913, 2144 und Tezisy Doul. - Simp. Khim. Tekhnol. Geterotsihl. Soedin. Gorguch. Iskop., 1973, 95-96 beschreiben jeweils die Synthese von substituierten Indolen.

Aufgabe der vorliegenden Erfindung war es daher, neue Verbindungen zur Verfügung zu stellen, die sich insbesondere als Wirkstoffe in Arzneimitteln eignen.

Diese Wirkstoffe sollen sich insbesondere zur Schmerztherapie und zur Behandlung von inflammatorischen und allergischen Reaktionen, Drogen-und/oder Alkoholmißbrauch, Diarrhoe, Gastritis, Ulcera, cardiovaskulären Erkrankungen, Harninkontinenz, Depressionen, Schockzuständen, Migräne, Narkolepsie, Übergewicht, Asthma, Glaukom, hyperkinetischem Syndrom, Antriebslosigkeit, Bulimie, Anorexie, Katalepsie, zur Anxiolyse, zur Vigilanzsteigerung und/oder zur Libidosteigerung eignen.

Erfindungsgemäß wird diese Aufgabe durch die Bereitstellung substituierter Indol-Mannichbasen der nachstehenden allgemeinen Formel 1 gelöst, die eine ausgeprägte analgetische Wirkung, insbesondere auch bei chronischen Schmerzen aufweisen, und die sich darüber hinaus zur Behandlung/Bekämpfung von inflammatorischen und allergischen Reaktionen, Drogen- und/oder Alkoholmißbrauch, Diarrhoe, Gastritis, Ulcera, cardiovaskulären Erkrankungen, Harninkontinenz, Depressionen, Schockzuständen, Migräne, Narkolepsie, Übergewicht, Asthma, Glaukom, hyperkinetischem Syndrom, Antriebslosigkeit, Bulimie, Anorexie, Katalepsie, zur Anxiolyse, zur Vigilanzsteigerung und/oder zur Libidosteigerung eignen.

Gegenstand der Erfindung sind daher substituierte Indol-Mannichbasen der allgemeinen Formel I,

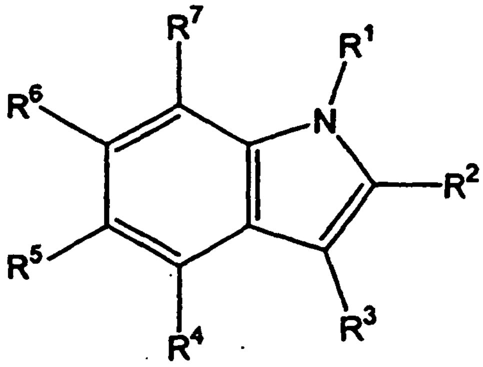

I

worin

$R^1$ = H, einen $C_{1-10}$-Alkyl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest, vorzugsweise H, einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, besonders bevorzugt H oder einen $C_{1-2}$-Alkyl-Rest, bedeutet,

$R^2$ = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$Alkyl-, einem $C_{1-6}$Alkoxy- oder einem Phenyl-Rest substituierten Phenyl- oder Naphthyl-Rest, der gegebenenfalls mit weiteren Ringen kondensiert ist, oder einen über eine $C_{1-6}$Alkylen-Gruppe gebundenen, unsubstituierten oder mit einem Halogen-, CN-, $CF_3$- oder OH-Rest substituierten Thiophen-, Pyrrolyl- oder Furfuryl-Rest , vorzugsweise H, F, Cl, $NO_2$, $OR^{10}$, $CO(OR^{11})$, einen $C_{1-6}$-Alkyl- oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, besonders bevorzugt H, einen $C_{1-2}$-Alkyl- oder einen unsubsituierten Phenyl-Rest, bedeutet,

$R^3$ = $CH(R^{13})N(R^{14})(R^{15})$ bedeutet,

$R^4$, $R^5$, $R^6$, $R^7$, gleich oder verschieden, = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$-oder OH-Rest substituierten Thiophen-, Pyrrolyl- oder Furfuryl-Rest, vorzugsweise H, F, Cl, $NO_2$, $OR^{10}$, $CO(OR^{11})$, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen $C_{1-6}$-Alkyl- oder einen über eine $C_{1-2}$-Alkylen-Gruppe gebundenen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, besonders bevorzugt H, $NO_2$, einen $C_{1-2}$-Alkyl- oder einen unsubsituierten Phenyl-Rest, bedeuten,

$R^8$ = H, $COR^{16}$, einen $C_{1-10}$-Alkyl- oder einen unsubstituierten oder wenigstens einfach mit einem OH-, einem Halogen-,

einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl-Rest, bedeutet,
$R^9$ = H, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl-Rest oder einen Phenyl-Rest bedeutet,
$R^{10}$ = H, $COR^{17}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen über eine $C_{1-6}$-AlkylenGruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest, vorzugsweise H, einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl-oder Naphthyl-Rest, der gegebenenfalls mit weiteren Ringen kondensiert ist, bedeutet,
$R^{11}$ = H, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise H oder einen $C_{1-6}$-Alkyl-Rest, bedeutet,
$R^{12}$ = H, einen $C_{1-10}$-Alkyl-Rest oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl-Rest, bedeutet,
$R^{13}$ = einen unsubstituierten Phenyl-Rest oder einen wenigstens einfach mit $C_{1-4}$-Alkyl-, Halogen-, $CF_3$-, CN- oder OH substituierten Phenyl-Rest, vorzugsweise einen unsubstituierten Phenyl-Rest oder einen wenigstens einfach mit Methyl-, tert-Butyl-, Methoxy-, F-, Cl-, Br- oder $CF_3$-substituierten Phenyl-Rest, besonders bevorzugt einen unsubstituierten Phenyl-Rest oder einen oder einen 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluor-phenyl-, 2-Chlor-4-Fluor-phenyl-, 2-Chlor-5-Fluor-phenyl-, 2-Chlor-6-Fluor-phenyl-, 4-Brom-2-Fluor-phenyl-, 3-Brom-4-Fluor-phenyl-, 3-Brom-2-Fluor-phenyl-, 2,3-Dichlor-phenyl-, 2,4-Dichlor-phenyl-, 2,5-Dichlorphenyl-, 3,4-Dichlor-phenyl-, 2,3-Dimethyl-phenyl-, 2,4-Dimethyl-phenyl-, 2,5-Dimethylphenyl-, 2-Trifluormethyl-phenyl, 3-Trifluormethyl-phenyl oder einen 4-Trifluormethyl-phenyl-Rest, ganz besonders bevorzugt einen unsubstituierten Phenyl-Rest, bedeutet,
$R^{14}$, $R^{15}$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest, vorzugsweise einen gesättigten, unsubsituierten oder wenigstens einfach substituierten $C_{1-6}$-Alkyl-Rest, besonders bevorzugt einen $CH_3$-Rest,
oder $R^{14}$ und $R^{15}$ zusammen $(CH_2)_n$ mit n = eine ganze Zahl von 3 bis 6 oder $(CH_2)_2O(CH_2)_2$, vorzugsweise $(CH_2)_n$ mit n = 4 oder 5, bedeuten,
$R^{16}$ = einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy-oder einem Phenyl-Rest substituierten Phenyl- oder Naphthyl-Rest, der gegebenenfalls mit weiteren Ringen kondensiert ist, oder einen unsubstituierten oder mit einem Halogen-, CN-, $CF_3$- oder OH-Rest substituierten Thiophen-, Pyrrolyl- oder Furfuryl-Rest, vorzugsweise einen $C_{1-6}$-Alkyl-Rest, bedeutet,
$R^{17}$ = einen $C_{1-10}$-Alkyl-Rest, vorzugsweise einen $C_{1-6}$-Alkyl-Rest, bedeutet,
$R^{18}$ = einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy-oder einem Phenyl-Rest substituierten Phenyl- oder Naphthyl-Rest, der gegebenenfalls mit weiteren Ringen kondensiert ist, oder einen unsubstituierten oder mit einem Halogen-, CN-, $CF_3$- oder OH-Rest substituierten Thiophen-, Pyrrolyl- oder Furfuryl-Rest, vorzugsweise einen $C_{1-6}$-Alkyl-Rest, bedeutet,
$R^{19}$ = H, $NHNH_2$, $NHR^{20}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylengruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylengruppe gebundenen, unsubstituierten oder mit einem Halogen-, CN-, $CF_3$- oder OH-Rest substituierten Thiophen-, Pyrrolyl- oder Furfuryl-Rest, vorzugsweise einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist,

bedeutet,
$R^{20}$ = H, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest bedeutet, einen über eine $C_{1-6}$-Alkylengruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylengruppe gebundenen, unsubstituierten oder mit einem Halogen-, CN-, $CF_3$- oder OH-Rest substituierten Thiophen-, Pyrrolyl- oder Furfuryl-Rest, vorzugsweise einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,
$R^{21}$ = H, $COR^{17}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen über eine $C_{1-6}$-Alkylengruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy-oder einem Phenyl-Rest substituierten Phenyl- oder Naphthyl-Rest, der gegebenenfalls mit weiteren Ringen kondensiert ist, oder einen über eine $C_{1-6}$-Alkylengruppe gebundenen, unsubstituierten oder mit einem Halogen-, CN-, $CF_3$-oder OH-Rest substituierten Thiophen-, Pyrrolyl- oder Furfuryl-Rest, vorzugsweise H, einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet, und/oder deren Racemate, Enantiomere, Diastereomere und/oder entsprechende Basen und/oder entsprechende Salze von physiologisch verträglichen Säuren,
wobei die Racemate der Verbindungen, in denen der Rest $R^3$ = $CH(R^{13})N(R^{14})(R^{15})$ bedeutet und jeweils
die Reste $R^4$ bis $R^7$ und $R^2$ = H, der Rest $R^{13}$ = Phenyl, die Reste $R^{14}$ und $R^{15}$ zusammen = $(CH_2)_5$ und der Rest $R^1$ = H, $CH_3$ oder Benzyl bedeuten
oder
die Reste $R^4$ bis $R^7$ und $R^2$ = H, der Rest $R^{13}$ = Phenyl, die Reste $R^{14}$, $R^{15}$ jeweils = $CH_3$, und der Rest $R^1$ = H, $CH_3$ oder Benzyl bedeuten
oder
die Reste $R^4$ bis $R^7$ und $R^1$ = H, der Rest $R^{13}$ = 2-Chlor-phenyl, die Reste $R^{14}$ und $R^{15}$ jeweils = $CH_3$ oder die Reste $R^{14}$ und $R^{15}$ zusammen = $(CH_2)_2O(CH_2)_2$, der Rest $R^2$ = $COOR^{11}$ und der Rest $R^{11}$ = $C_2H_5$ bedeuten sowie die entsprechenden Hydrochloride
oder
die Reste $R^4$ bis $R^7$ und $R^1$ = H, der Rest $R^{13}$ = Phenyl, die Reste $R^{14}$ und $R^{15}$ zusammen = $(CH_2)_5$, der Rest $R^2$ = $COOR^{11}$ und der Rest $R^{11}$ = $CH_3$ bedeuten und das entsprechende Hydrochlorid
oder
die Reste $R^4$ bis $R^7$ und $R^2$ = H, der Rest $R^{13}$ = Phenyl, die Reste $R^{14}$ und $R^{15}$ zusammen = $(CH_2)_4$, der Rest $R^1$ = H, $CH_3$, $CH_2$-$CH$=$CH_2$ oder 4-Chlorbenzyl sowie für $R^1$ = $CH_3$ auch das entsprechende Hydrochlorid der Verbindung
oder
die Reste $R^4$ bis $R^7$, $R^1$ und $R^2$ = H, der Rest $R^{13}$ = Phenyl, der Rest $R^{14}$ = Phenyl und der Rest $R^{15}$ = 4-Ethoxyphenyl bedeuten
oder
die Reste $R^4$ bis $R^7$ und $R^1$ = H, der Rest $R^{13}$ = Phenyl, 2-Hydroxyphenyl oder 2-Hydroxy-5-methylphenyl, die Reste $R^{14}$ und $R^{15}$ zusammen = $(CH_2)_5$ und der Rest $R^2$ = $CH_3$ bedeuten, für $R^{13}$ = Phenyl auch das entsprechende Hydrogensulfat
oder
die Reste $R^4$ bis $R^7$ und $R^1$ = H, der Rest $R^{13}$ = Phenyl, die Reste $R^{14}$ und $R^{15}$ zusammen = $(CH_2)_2O(CH_2)_2$ und der Rest $R^2$ = $CH_3$ bedeuten und das entsprechende Hydrogensulfat der Verbindung
ausgenommen sind.

Unter Alkyl-Resten werden vorzugsweise mindestens einfach mit Halogen-, OH-, CN-, $CF_3$-, besonders bevorzugt mit F-, Cl-, Br- oder OH-, substituierte Kohlenwasserstoff-Reste verstanden. Enthalten diese mehr als einen Substituenten, so können diese Substituenten gleich oder verschieden sein. Die Alkyl-Reste können verzweigt, unverzweigt oder cyclisch sein. Besonders bevorzugt sind die Alkyl-Reste Methyl, Ethyl, Propyl, 1-Methylethyl, Butyl, 1-Methylpropyl, 2-Methylpropyl, 1,1-Dimethylpropyl, 1,2-Dimethylpropyl, 2,2-Dimethylpropyl, Hexyl, 1-Methylpentyl, Heptyl, Nonyl oder Decanyl.

Unter einem Aryl-Rest werden mindestens einfach, mit einem OH-, einem Halogen-, vorzugsweise F-, Br- oder

Cl-, einem $CF_3$-, einem CN-, einem $C_{1-6}$Alkyl-, einem $C_{1-6}$-Alkoxy- oder einem Phenyl-Rest substituierte Phenyl- oder Naphthyl-Reste verstanden. Die unsubstituierten oder substituierten Phenyl-Reste können auch mit weiteren Ringen kondensiert sein. Besonders bevorzugt sind die Aryl-Reste 2-, 3-, 4-Bromphenyl, 4-Brom-2-fluorphenyl, 5-Brom-2-fluorphenyl, 3-Brom-4-fluorphenyl, 4-tert.-Butylphenyl, 2-Chlor-4-fluorphenyl, 2-Chlor-6-fluorphenyl, 4-Cyanophenyl, 2,3-Dichlorphenyl, 2,4-Dichlorphenyl, 2,6-Dichforphenyl, 3,4-Dichlorphenyl, 2,3-Dimethoxyphenyl, 3,4-Dimethoxyphenyl; 2,4-Dimethylphenyl, 2,5-Dimethylphenyl, 2-, 3-, 4-Fluorphenyl, 2-Methoxyphenyl, 2-,3-,4-Methylphenyl, 3-Phenoxyphenyl, 2-,4-Trifluormethylphenyl oder 3,4,5-Trimethoxypheny.

Unter einem Heteroaryl-Rest wird ein Thiophen-, Pyrrolyl- oder Furfuryl-Rest verstanden, der unsubstituiert oder mit einem Halogen-, CN-, $CF_3$- oder OH-Rest substituiert sein kann.

Besonders bevorzugt sind folgende substituierte Indol-Mannichbasen:

3-(Dimethylaminophenylmethyl)-1H-indol-2-carbonsäureethylester
3-(Dimethylaminophenylmethyl)-1H-indol-2-carbonsäure
[(5-Fluoro-1H-IndoJ-3-yl)-phenylmethyl]-dimethylamin
[(1-Ethyt-2-phenyl-1H-indol-3-yl)-phenylmethyl]-dimethylamin
[(5-Methoxy-1H-indol-3-yl)-phenylmethyl]-dimethylamin
3-(Dimethylaminophenylmethyl)-5-hydroxy-1H-indol-2-carbonsäure
Dimethyl-[(2-methyl-1H-indol-3-yl)-phenylmethyl]-amin
3-(Dimethylaminophenylmethyl)-1H-indol-4-ol
Dimethyl-[(4-methyl-1H-indol-3-yl)-phenylmethyl]-amin
[(5-Chloro-1H-indol-3-yl)-phenylmethyl]-dimethylamin
[(5-Benzyloxy-1H-indol-3-yl)-phenylmethyl]-dimethylamin
Essigsäure-3-(dimethylaminophenylmethyl)-1H-indol-4-yl-ester
{[2-(4-Chlorophenyl)-1H-indol-3-yl]-phenylmethyl}-dimethylamin
5-Benzyloxy-3-(dimethylaminophenylmethyl)-1H-indol-2-carbonsäure
Dimethyl-[(2-methyl-5-nitro-1H-indol-3-yl)-phenylmethyl]-amin
Dimethyl-[(2-methyl-6-nitro-1H-indol-3-yl)-phenylmethyl]-amin
[(6-Fluoro-2-methyl-1H-indol-3-yl)-phenylmethyl]-dimethylamin
{[2-(4-Fluorophenyl)-1H-indol-3-yl]-phenylmethyl}-dimethylamin
{[2-(3-Chloro-4-fluorophenyl)-1H-indol-3-yl]-phenylmethyl}-dimethylamin
[(7-Ethyl-1H-indol-3-yl)-phenylmethyl]-dimethylamin
3-(Dimethylaminophenylmethyl)-1H-indol-6-carbonsäure
Dimethyl-[(1-methyl-1H-indol-3-yl)-phenylmethyl]-amin
1-Methyl-3-(morpholin-4-yl-phenylmethyl)-1H-indol
1-Ethyl-2-phenyl-3-(pyrrolidin-1-yl-o-tolylmethyl)-1H-indol
3-[(2-Chlorophenyl)-piperidin-1-yl-methyl]-1-ethyl-2-phenyl-1H-indol
1-Ethyl-2-phenyl-3-(phenylpyrrolidin-1-yl-methyl)-1H-indol
2-Phenyl-3-(pyrrolidin-1-yl-*o*-tolylmethyl)-1H-indol
2-Phenyl-3-(piperidin-1-yl-*o*-tolylmethyl)-1H-indol
3-[(2-Chlorophenyl)-piperidin-1-yl-methyl]-2-phenyl-1H-indol
Dimethyl-[(2-phenyl-1H-indol-3-yl)-o-tolyl-methyl]-amin
2-Phenyl-3-(phenylpyrrolidin-1-yl-methyl)-1H-indol
4-Methyl-3-(piperidin-1-yl-o-totyl-methyl)-1H-indol
5-Benzyloxy-3-(phenyl-piperidin-1-yl-methyl)-1H-indol
[(5-Benzyloxy-1H-indol-3-yl)-o-tolylmethyl]-dimethylamin
{[1-(4-Methoxybenzyl)-1H-indol-3-yl]-phenyl-methyl}-dimethylamin
3-(Phenylpiperidin-1-yl-methyl)-1H-indol
1-Methyl-3-(phenylpiperidin-1-yl-methyl)-1H-indol
3-(Phenylpyrrolidin-1-yl-methyl)-1H-indol
[(1H-Indol-3-yl)-phenylmethyl]-dimethylamin
5-Benzyloxy-3-(phenyl-pyrrolidin-1-yl-methyl)-1H-indol
[(5-Brom-2-fluor-phenyl)-(4-nitro-1H-indol-3-yl)-methyl]-dimethyl-amin
[(5-Brom-2-fluor-phenyl)-(1-methyl-1H-indol-3-yl)-methyl]-dimethyl-amin
[(2-Chlor-6-fluor-phenyl)-(2-phenyl-1H-indol-3-yl)-methyl]-dimethylamin
[(2-Chlor-6-fluor-phenyl)-(1-ethyl-2-phenyl-1H-indol-3-yl)-methyl]-dimethylamin
[(2-Chlor-6-fluor-phenyl)-(4-nitro-1H-indol-3-yl)-methyl]-dimethyl-amin
[(2-Chlor-6-fluor-phenyl)-(1-methyl-1H-indol-3-yl)-methyl]-dimethyl-amin
[(2-Brom-phenyl)-(4-nitro-1H-indol-3-yl)-methyl]-dimethyl-amin

[(2-Brom-phenyl)-(1-methyl-1H-indol-3-yl)-methyl]-dimethyl-amin
[(3-Brom-phenyl)-(7-ethyl-1H-indol-3-yl)-methyl]-dimethyl-amin
[(3-Brom-phenyl)-(1-methyl-1H-indol-3-yl)-methyl]-dimethyl-amin
[(4-tert-Butyl-phenyl)-(2-methyl-1H-indol-3-y-)-methyl]-dimethyl-amin
[(4-tert-Butyl-phenyl)-(1-ethyl-2-phenyl-1H-indol-3-yl)-methyl]-dimethyl-amin
[(4-tert-Butyt-phenyl)-(1-methyl-1H-indol-3-yl)-methyl]-dimethyl-amin
[(2-Chlor-4-fluor-phenyl)-(4-nitro-1H-indol-3-yl)-methyl]-dimethyl-amin
[(2-Chlor-4-fluor-phenyl)-(4-methyl-1H-indol-3-yl)-methyl]-dimethyl-amin
[(2-Chlor-6-fluor-phenyl)-(2-phenyl-1H-indol-3-yl)-methyl]-dimethyl-amin
{(2-Chlor-6-fluor-phenyl)-[2-(4-chlor-phenyl)-1H-indol-3-yl]-methyl}-dimethylamin
[(2-Chlor-6-fluor-phenyl)-(1-methyl-1H-indol-3-yl)-methyl]-dimethyl-amin
[(3-Chlor-phenyl)-(1-methyl-1H-indol-3-yl)-methyl]-dimethyl-amin
[(2,3-Dichlor-phenyl)-(1-methyl-1H-indol-3-yl)-methyl]-dimethyl-amin
[(2,4-Dichlor-phenyl)-(1-methyl-1H-indol-3-yl)-methyl]-dimethyl-amin.
[(4-tert-Butyl-phenyl)-(1-ethyl-2-phenyl-1H-indol-3-yl)-methyl]-dimethyl-amin
[(2-Chlor-4-fluor-phenyl)-(2-methyl-1H-indol-3-yl)-methyl]-dimethyl-amin
[(2-Chlor-6-fluor-phenyl)-(2-methyl-1H-indol-3-yl)-methyl]-dimethyl-amin
[(5-Chlor-1H-indol-3-yl)-(2-fluor-phenyl)-methyl]-dimethyl-amin
[(4-Fluor-phenyl)-(4-methyl-1H-indol-3-yl)-methyl]-dimethyl-amin
Dimethyl-[(2-methyl-1H-indol-3-yl)-o-tolyl-methyl]-amin
[(7-Ethyl-1H-indol-3-yl)-o-tolyl-methyl]-dimethyl-amin
Dimethyl-[(1-methyl-1H-indol-3-yl)-o-tolyl-methyl]-amin
[(5-Chlor-1H-indol-3-yl)-o-tolyl-methyl]-dimethyl-amin
[(5-Chlor-1H-indol-3-yl)-m-tolyl-methyl]-dimethyl-amin
Dimethyl-[(2-methyl-1H-indol-3-yl)-p-tolyl-methyl]-amin
[(5-Chlor-1H-indol-3-yl)-p-tolyl-methyl]-dimethyl-amin
[(5-Chlor-1H-indol-3-yl)-(2-trifluormethyl-phenyl)-methyl]-dimethyl-amin
Dimethyl-[(2-methyl-1H-indol-3-yl)-(4-trifluormethyl-phenyl)-methyl]-amin.

Ein weiterer Gegenstand der Erfindung sind Verfahren zur Herstellung von substituierten Indol-Mannichbasen der allgemeinen Formel I, die dadurch gekennzeichnet sind, daß man aromatische Aldehydverbindungen der allgemeinen Formel II,

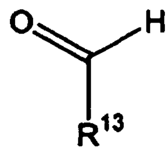

II

worin $R^{13}$ die Bedeutung gemäß der allgemeinen Formel 1 hat, in Lösung, vorzugsweise in einem organischen Lösungsmittel, besonders bevorzugt in Toluol, in Gegenwart einer Base, vorzugsweise Kaliumcarbonat oder Borsäureanhydrid bei einer Temperatur von vorzugsweise -10 bis 110 °C mit sekundären Aminen der allgemeinen Formel III,

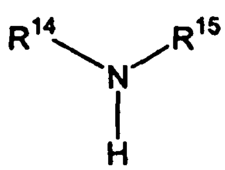

III

in denen $R^{14}$ und $R^{15}$ die Bedeutung gemäß der allgemeinen Formel I haben, zu Aminalverbindungen der allgemeinen Formel IV,

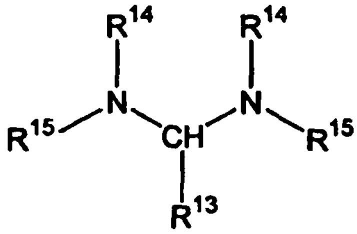

IV

umsetzt, und diese Aminalverbindungen der allgemeinen Formel IV ohne weitere Aufreinigung mit einem Säurechlorid, vorzugsweise mit Acetylchlorid, in absolutem Lösungsmittel, vorzugsweise in Diethylether zu Iminiumsalzen der allgemeinen Formel V

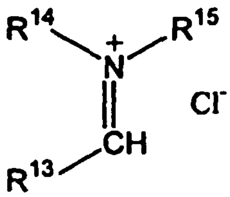

V

umsetzt, und diese Iminiumsalze der allgemeinen Formel V ohne weitere Aufreinigung in Lösung, vorzugsweise in Acetonitril und/oder Toluol mit Indol und/oder substituierten Indolverbindungen der allgemeinen Formel VI,

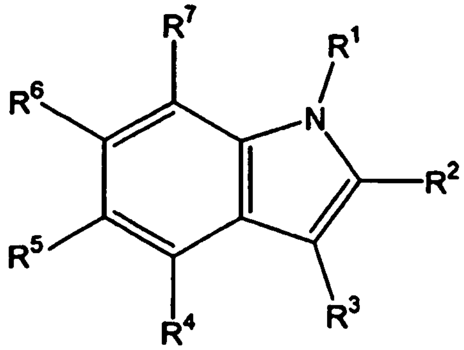

VI

in denen der Rest $R^3$ = H bedeutet und die Reste $R^1$, $R^2$, $R^4$ bis $R^{12}$ und $R^{16}$ bis $R^{21}$ die Bedeutung gemäß der allgemeinen Formel I haben, umsetzt, und die so erhaltenen Indol-Mannichbasen der allgemeinen Formel I durch Waschen und/oder Extraktion, vorzugsweise durch Waschen mit Aceton reinigt und nach den üblichen Methoden isoliert.

Ein weiterer Gegenstand der Erfindung sind auch alternative Verfahren zur Herstellung von substituierten Indol-Mannichbasen der allgemeinen Formel I, in denen der Rest $R^1 \neq$ H ist und die Reste $R^2$ bis $R^{21}$ die Bedeutung gemäß

der allgemeinen Formel I haben, die dadurch gekennzeichnet sind, daß man aromatische Aldehydverbindungen der allgemeinen Formel II,

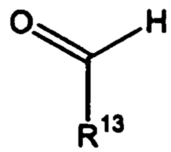

II

worin $R^{13}$ die Bedeutung gemäß der allgemeinen Formel I hat, in Lösung, vorzugsweise in einem organischen Lösungsmittel, besonders bevorzugt in Toluol, in Gegenwart einer Base, vorzugsweise Kaliumcarbonat oder Borsäureanhydrid bei einer Temperatur von vorzugsweise -10 bis 110 °C mit sekundären Aminen der allgemeinen Formel III.

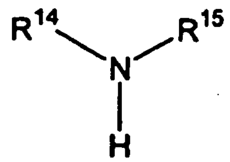

III

in denen die Reste $R^{14}$ und $R^{15}$ die Bedeutung gemäß der allgemeinen Formel I haben, zu Aminalverbindungen der allgemeinen Formel IV

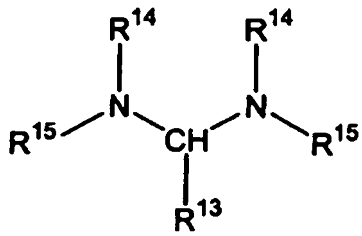

IV

umsetzt, und diese Aminalverbindungen der allgemeinen Formel IV ohne weitere Aufreinigung mit einem Säurechlorid, vorzugsweise mit Acetylchlorid, in absolutem Lösungsmittel, vorzugsweise in Diethylether, zu Iminiumsalzen der allgemeinen Formel V

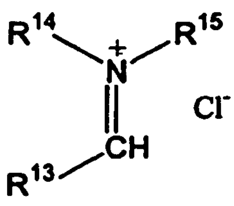

V

umsetzt, und diese Iminiumsalze der allgemeinen Formel V ohne weitere Aufreinigung in Lösung, vorzugsweise in Acetonitril und/oder Toluol, mit Indol und/oder substituierten Indolverbindungen der allgemeinen Formel VI,

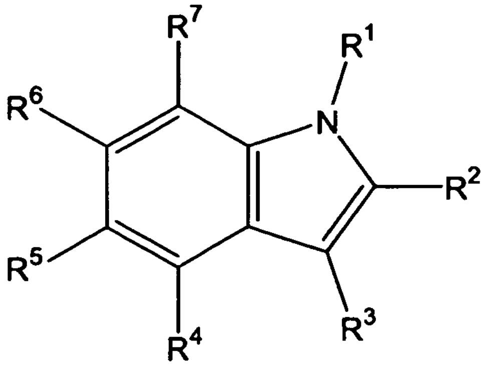

VI

in denen die Reste $R^1$ und $R^3$ jeweils = H bedeuten und die Reste $R^2$, $R^4$ bis $R^{12}$ und $R^{16}$ bis $R^{21}$ die Bedeutung gemäß der allgemeinen Formel I haben, umsetzt, und die so erhaltenen Verbindungen der allgemeinen Formel VI, in denen $R^1$ = H ist und die Reste $R^2$ bis $R^{21}$ die Bedeutung gemäß der allgemeinen Formel I haben, in Lösung mit Verbindungen der allgemeinen Formel $XR^{22}$, in denen $R^{22}$ einen $C_{1-10}$-Alkyl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest steht, der gegebenenfalls mit weiteren Ringen kondensiert ist, oder einen über eine $C_{1-6}$-AlkylenGruppe gebundenen, unsubstituierten oder mit einem Halogen-, CN-, $CF_3$- oder OH-Rest substituierten Thiophen-, Pyrrolyl- oder Furfuryl-Rest bedeutet und X = Cl, Br oder I, vorzugsweise Cl, bedeutet, bei einer Temperatur von vorzugsweise 10 bis 150°C in Gegenwart einer Base, vorzugsweise Kaliumhydroxid umsetzt, und die so erhaltenen Verbindungen der allgemeinen Formel I, in denen $R^1$ für einen $C_{1-10}$-Alkyl-, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$ Alkoxy- oder einem Phenyl-Rest substituierten Phenyl- oder Naphthyl-Rest steht, der gegebenenfalls mit weiteren Ringen kondensiert ist, oder einen über eine $C_{1-6}$-AlkylenGruppe gebundenen, unsubstituierten oder mit einem Halogen-, CN-, $CF_3$- oder OH-Rest substituierten Thiophen-, Pyrrolyl- oder Furfuryl-Rest bedeutet und die übrigen Reste $R^2$ bis $R^{21}$ die Bedeutung gemäß der allgemeinen Formel I haben, durch

Filtration, vorzugsweise durch Filtration über ein Scavenger-Harz, besonders bevorzugt durch Filtration über polymergebundenes Tris(2-aminoethyl)-amin (Novabiochem, Bad Soden) und/oder 3-(3-Mercaptophenyl)propan-amidomethylpolystylrol (Argonaut, Muttenz, Schweiz) reinigt und nach den üblichen Methoden isoliert.

Vorzugsweise erfolgt die Synthese der erfindungsgemäßen substituierten Indol-Mannichbasen auf einer automatischen Anlage der Firma Zymark gemäß Figur 1 und Figur 2 wie untenstehend beschrieben.

Die erfindungsgemäßen substituierten Indol-Mannichbasen der allgemeinen Formel I lassen sich mit physiologisch verträglichen Säuren, vorzugsweise mit Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in einer dem Fachmann an sich bekannten Weise in ihre Salze überführen. Vorzugsweise wird die Salzbildung in einem Lösungsmittel, besonders bevorzugt in Diethylether, Diisopropylether, Essigsäurealkylester, Aceton und/oder 2-Butanon durchgeführt. Ganz besonders bevorzugt erfolgt die Salzbildung mit Trimethylchlorsilan in Methylethylketon.

Die erfindungsgemäßen substituierten Indol-Mannichbasen der allgemeinen Formel I sind toxikologisch unbedenklich und stellen daher geeignete pharmazeutische Wirkstoffe dar.
Ein weiterer Gegenstand der Erfindung sind daher auch Arzneimittel, die als Wirkstoff wenigstens eine substituierte Indol-Mannichbase der allgemeinen Formel I gemäß Anspruch 56 und gegebenenfalls weitere Wirkstoffe und/oder Hilfsstoffe enthalten.

Vorzugsweise kann das Arzneimittel als Wirkstoff auch ein Gemisch aus Enantiomeren wenigstens einer substituierten Indol-Mannichbase der allgemeinen Formel I enthalten, wobei das Gemisch die Enantiomere vorzugsweise nicht in äquimolaren Mengen aufweist. Besonders bevorzugt beträgt der relative Anteil eines der Enantiomere 5 bis 45 Mol-%, ganz besonders bevorzugt 10 bis 40 Mol-%, bezogen auf das Gemisch der Enantiomere.

Vorzugsweise werden die Arzneimittel zur Behandlung/Bekämpfung von Schmerzen, insbesondere chronischen Schmerzen, und/oder inflammatorischen Reaktionen und/oder allergischen Reaktionen und/oder Drogenmißbrauch und/oder Alkoholmißbrauch und/oder Diarrhoe und/oder Gastritis und/oder Ulcera und/oder cardiovaskulären Erkrankungen und/oder Harninkontinenz und/oder Depressionen und/oder Schockzuständen und/oder Migräne und/ oder Narkolepsie und/oder Übergewicht und/oder Asthma und/oder Glaukom und/oder hyperkinetischem Syndrom und/oder Antriebslosigkeit und/oder Bulimie und/oder Anorexie und/oder Katalepsie und/oder zur Anxiolyse und/oder zur Vigilanzsteigerung und/oder zur Libidosteigerung eingesetzt.

Die Verwendung wenigstens einer erfindungsgemäßen substituierten Indol-Mannichbase der allgemeinen Formel I gemäß Anspruch 62 zur Hertellung eines Arzneimittels zur Behandlung/Bekämpfung von Schmerzen, insbesondere von chronischen Schmerzen, und/oder inflammatorischen Reaktionen und/oder allergischen Reaktionen und/oder Drogenmißbrauch und/oder Alkoholmißbrauch und/oder Diarrhoe und/oder Gastritis und/oder Ulcera und/oder cardiovaskulären Erkrankungen und/oder Harninkontinenz und/oder Depressionen und/oder Schockzuständen und/oder Migräne und/ oder Narkolepsie und/oder Übergewicht und/oder Asthma und/oder Glaukom und/oder hyperkinetischem Syndrom und/oder Antriebslosigkeit und/oder Bulimie und/oder Anorexie und/oder Katalepsie und/oder zur Anxiolyse und/oder zur Vigilanzsteigerung und/oder zur Libidosteigerung ist ebenfalls ein Gegenstand der vorliegenden Erfindung.

Zur Zubereitung entsprechender pharmazeutischer Formulierungen werden neben mindestens einer substituierten Indol-Mannichbase der allgemeinen Formel I Trägermaterialien, Füllstoffe, Lösungsmittel, Verdünnungsmittel, Farbstoffe und/oder Bindemittel eingesetzt. Die Auswahl der Hilfsstoffe hängt davon ab, ob das Arzneimittel oral, intravenös, intraperitoneal, intradermal, intramuskulär, intranasal, buccal oder örtlich, zum Beispiel auf Infektionen an der Haut, der Schleimhäute und an den Augen, appliziert werden soll. Für die orale Applikation eignen sich die Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays.

Die erfindungsgemäßen Indol-Mannichbasen der allgemeinen Formel I in einem Depot in gelöster Form oder in einem Pflaster, gegebenenfalls unter Zusatz von die Hautpenetration fördernden Mittel, stellen geeignete perkutane Applikationszubereitungen dar. Aus oralen oder perkutanen Zubereitungsformen können die erfindungsgemäßen Verbindungen der allgemeinen Formel 1 verzögert freigesetzt werden.

**Pharmakologische Untersuchungen:**

1.) In vitro-Tests

Die breite Austestung der erfindungsgemäßen Indol-Mannichbasen auf ihre Wirksamkeit erfolgte nach den üblichen High Throughput Screening Methoden, wie sie in John P. Devlin, High Throughput Screening, 1997, Marcel Dekker Inc. beschrieben sind. Sie werden hiermit als Referenz eingeführt und gelten somit als Teil der Offenbarung.

Die Wirkung der erfindungsgemäßen Indol-Mannichbasen wird insbesondere durch die Affinität zur N-Methyl-D-Aspartat-(NMDA) Rezeptorfamilie, zu $\alpha$-adrenergen Rezeptoren und opioiden Rezeptoren bestimmt.

Die Untersuchungen zur Serotonin-Wiederaufiahmehemmung (5-HT-Uptake-Hemmung) erfolgte nach den Methoden, wie sie in M.Ch. Frink, H.-H.-Hennies, W. Englberger, M. Haurand und B. Wilffert, Arzneim.-Forsch./Drug. Res. 46 (III), 11, 1996, Seiten 1029-1036 beschrieben sind. Sie werden hiermit als Referenz eingeführt und gelten somit als Teil der Offenbarung.

Zur Durchführung dieser Untersuchungen wurden Synaptosomen aus Rattenhimarealen frisch isoliert. Es wurde jeweils die sogenannte "P2"-Fraktion verwendet, die gemäß der Vorschrift in E.G. Gray und V.P. Whittaker, J. Anat. 76, Seiten 79-88, 1962 präpariert wird. Diese Literatur wird hiermit als Referenz eingeführt und gilt somit als Teil der Offenbarung. Für die Bestimmung des 5-HT-Uptake wurden diese vesikulären Partikel aus dem Pons- und Medulla oblongata-Bereich des männlichen Rattengehims isoliert.

Folgende Kenndaten wurden für den 5-HT-Transporter ermittelt:

5-HT-Uptake: $K_m = 0{,}084 \pm 0.011\ \mu M$

$V_{max}$: $38{,}13 \pm 4{,}52$ pmo/min/mg Protein.

Die Ergebnisse der Untersuchungen sind jeweils als Mittelwerte aus 2 Parallelversuchen angegeben.

2.) Analgesieprüfung im Writhing-Test an der Maus

Die vertiefte Untersuchung der erfindungsgemäßen Verbindungen auf ihre analgetische Wirksamkeit wurde im Phenylchinon-induzierten Writhing an der Maus (modifiziert nach I.C. Hendershot, J. Forsaith, J. Pharmacol. Exp. Ther. 125, 237-240 (1959)) durchgeführt. Dazu wurden männliche NMRI-Mäuse im Gewicht von 25-30 g verwendet. Gruppen von 10 Tieren pro Substanzdosis erhielten 10 Minuten nach intravenöser Gabe der Prüfsubstanzen 0,3 ml/ Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden einzeln in Beobachtungskäfige gesetzt. Mittels eines Drucktastenzähler wurde die Anzahl der Schmerz-induzierten Streckbewegungen (sogenannte Writhingreaktionen = Durchdrücken des Körpers mit Abstrecken der Hinterextremitäten) 5-20 Minuten nach der Phenylchinon-Gabe ausgezählt. Als Kontrolle wurden Tiere mitgeführt, die nur physiologische Kochsalzlösung erhielten.

Die Substanzen wurden in der Standarddosis von 10 mg/kg getestet. Die Hemmung der Writhingreaktionen durch eine Substanz wurde nach folgender Formel berechnet

$$\%\ \text{Hemmung} = 100 - \left[ \frac{\text{Writhingreaktion behan. Tiere}}{\text{Writhingreaktion Kontrolle}} \times 100 \right]$$

Die folgenden Beispiele dienen der Erläuterung der Erfindung.

Beispiel 1:

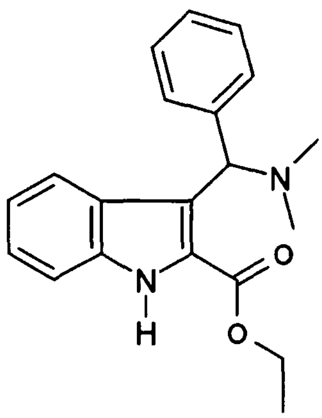

3-(Dimethylaminophenylmethyl)-1H-indol-2-carbonsäureethylester

1. Stufe

Benzyliden-dimethyl-ammoniumchlorid

Die Umsetzung von 32,0 ml (0,213 mol) Dimethylaminlösung und 8,0 ml (0,079 mol) Benzaldehyd gemäß der allgemeinen Synthesevorschrift 1 und nachfolgende Reaktion mit 4,7 ml (0,079 mol) Acetylchlorid gemäß der allgemeinen Synthesevorschrift 3 ergaben 9,5 g (entsprechend 70.7 % der theoretisch berechneten Ausbeute) an Benzyliden-dimethyl-ammoniumchlorid.

2. Stufe

3-(Dimethylaminophenylmethyl)-1H-indol-2-carbonsäureethylester

Die Herstellung erfolgte gemäß der allgemeinen Synthesevorschrift 4 aus 1H-Indol-2-carbonsäureethylester und Benzyliden-dimethyl-ammoniumchlorid.
Zur Charakterisierung wurde ein ESI-MS aufgenommen:
MS (EI) m/z: 323.2, 278.4 ($M^*$).

Beispiel 2:

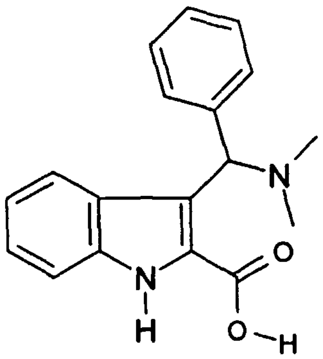

3-(Dimethylaminophenylmethyl)-1H-indol-2-carbonsäure
Die Herstellung erfolgte gemäß der allgemeinen Synthesevorschrift 4 aus 1H-Indol-2-carbonsäure und Benzyliden-dimethyl-ammoniumchlorid, welches gemäß Beispiel 1 hergestellt worden ist.
Zur Charakterisierung wurde ein ESI-MS aufgenommen:
MS (EI) m/z: 295.2, 250.4, 206.6 (M*).

Beispiel 3:

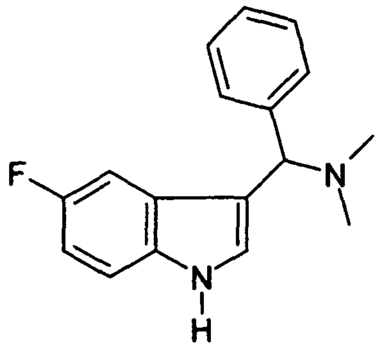

[(5-Fluoro-1H-indol-3-yl)-phenylmethyl]-dimethylamin

Die Herstellung erfolgte gemäß der allgemeinen Synthesevorschrift 4 aus 5-Fluoro-1H-indol und Benzyliden-dimethyl-ammoniumchlorid, welches gemäß Beispiel 1 hergestellt worden ist.

Zur Charakterisierung wurde ein ESI-MS aufgenommen:

MS (EI) m/z: 313.3, 268.3, 224.6 ($M^*$).

Beispiel 4:

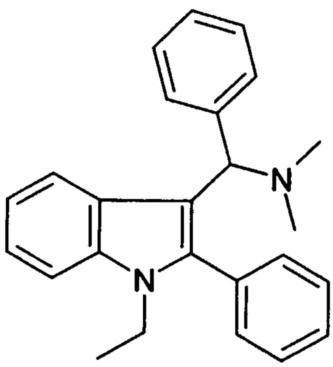

[(1-Ethyl-2-phenyl-1H-indol-3-yl)-phenylmethyl]-dimethylamin

Die Herstellung erfolgte gemäß der allgemeinen Synthesevorschrift 4 aus 1-Ethyl-2-phenyl-1H-indol und Benzyliden-dimethyl-ammoniumchlorid, welches gemäß Beispiel 1 hergestellt worden ist.

Zur Charakterisierung wurde ein ESI-MS aufgenommen:

MS (EI) m/z: 310.5 ($M^*$).

Beispiel 5:

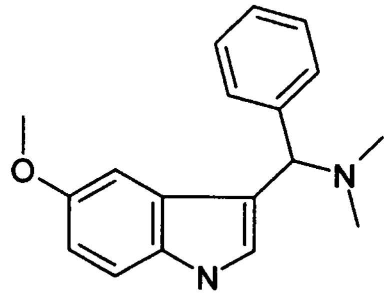

[(5-Methoxy-1H-indol-3-yl)-phenylmethyl]-dimethylamin

Die Herstellung erfolgte gemäß der allgemeinen Synthesevorschrift 4 aus 5-Methoxy-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 325.1, 280.3(M*).

Beispiel 6:

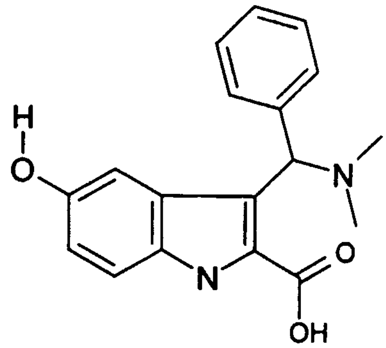

3-(Dimethylaminophenylmethyl)-5-hydroxy-1H-indol-2-carbonsäure
Die Herstellung erfolgte gemäß der allgemeinen Synthesevorschrift 4 aus 5-Hydroxy-1H-indol-2-carbonsäure und Benzyliden-dimethyl-ammoniumchlorid, welches gemäß Beispiel 1 hergestellt worden ist.

Zur Charakterisierung wurde ein ESI-MS aufgenommen:
MS (EI) m/z: 310.6, 266.4, 222.5(M*).

Beispiel 7:

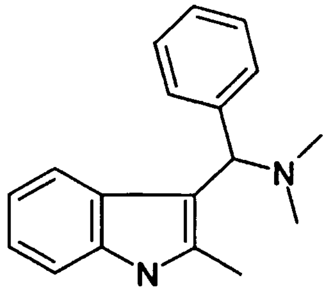

Dimethyl-[(2-methyl-1H-indol-3-yl)-phenylmethyl]-amin

Die Herstellung erfolgte gemäß der allgemeinen Synthesevorschrift 4 aus 2-Methyl-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 220.5(M*).

Beispiel 8:

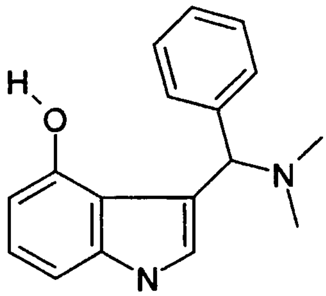

3-(Dimethylaminophenylmethyl)-1H-indol-4-ol

Die Herstellung erfolgte gemäß der allgemeinen Synthesevorschrift 4 aus 4-Hydroxy-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 267.1, 222.5(M*).

Beispiel 9:

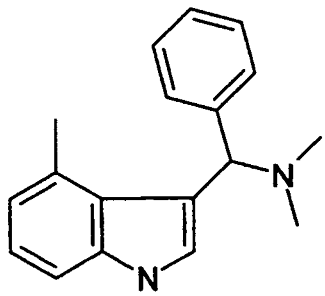

Dimethyl-[(4-methyl-1H-indol-3-yl)-phenylmethyl]-amin

Die Herstellung erfolgte gemäß der allgemeinen Synthesevorschrift 4 aus 4-Methyl-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 220.5(M*).

Beispiel 10:

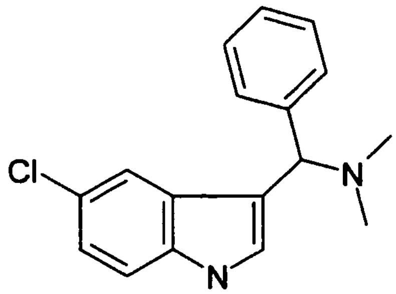

[(5-Chloro-1H-indol-3-yl)-phenylmethyl]-dimethylamin

Die Herstellung erfolgte gemäß der allgemeinen Synthesevorschrift 4 aus 5-Chloro-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 240.6 ($M^*$).

Beispiel 11:

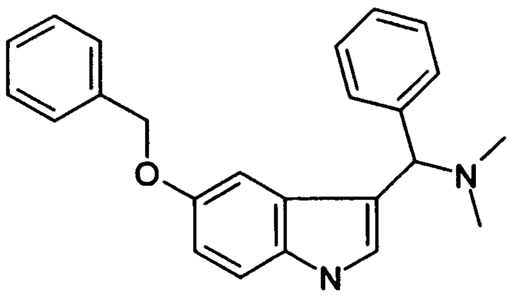

[(5-Benzyloxy-1H-indol-3-yl)-phenylmethyl]-dimethylamin

Die Herstellung erfolgte gemäß der allgemeinen Synthesevorschrift 4 aus 5-Benzyloxy-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 312.4 ($M^*$).

Beispiel 12:

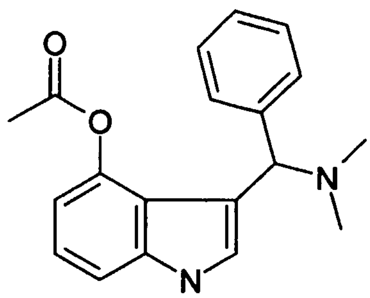

Essigsäure-3-(dimethylaminophenylmethyl)-1H-indol-4-yl-ester

Die Herstellung erfolgte gemäß der allgemeinen Synthesevorschrift 4 aus Essigsäure-1H-indol-4-yl-ester und Benzyliden-dimethyl-ammoniumchlorid, welches gemäß Beispiel 1 hergestellt worden ist.

Zur Charakterisierung wurde ein ESI-MS aufgenommen:
MS (EI) m/z: 264.3, 222.5 (M*).

Beispiel 13:

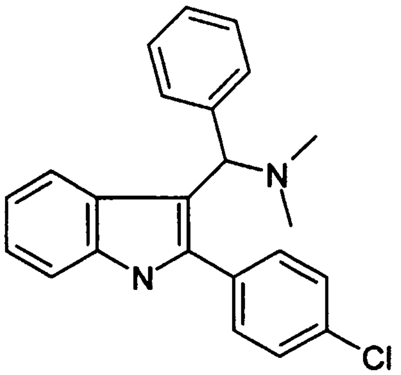

{[2-(4-Chlorophenyl)-1H-indol-3-yl]-phenylmethyl}-dimethylamin

Die Herstellung erfolgte gemäß der allgemeinen Synthesevorschrift 4 aus 2-(4-Chlorophenyl)-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 316.3(M*).

Beispiel 14:

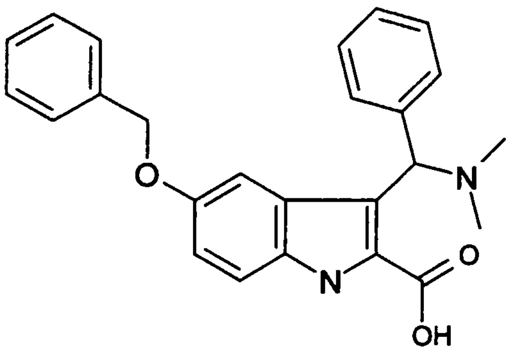

5-Benzyloxy-3-(dimethylaminophenylmethyl)-1H-indol-2-carbonsäure

Die Herstellung erfolgte gemäß der allgemeinen Synthesevorschrift 4 aus 5-Benzyloxy-1H-indol-2-carbonsäure und Benzyliden-dimethyl-ammoniumchlorid, welches gemäß Beispiel 1 hergestellt worden ist.

Zur Charakterisierung wurde ein ESI-MS aufgenommen:
MS (EI) m/z: 401.1, 356.3, 312.4 (M*).

Beispiel 15:

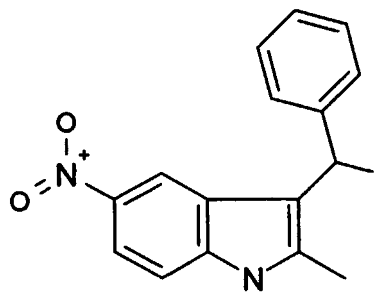

Dimethyl-[(2-methyl-5-nitro-1H-indol-3-ylrphenylmethyl)-amin

Die Herstellung erfolgte gemäß der allgemeinen Synthesevorschrift 4 aus 2-Methyl-5-nitro-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 265.4, 235.6 ($M^*$).

Beispiel 16:

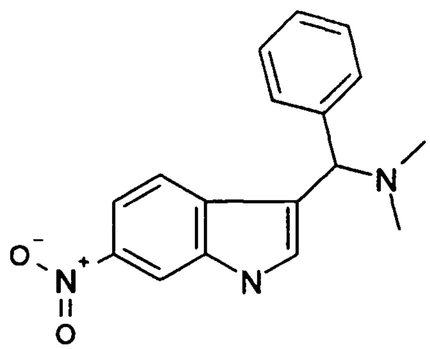

Dimethyl-[(6-nitro-1H-indol-3-yl)-phenylmethyl]-amin

Die Herstellung erfolgte gemäß der allgemeinen Synthesevorschrift 4 aus 6-Nitro-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 251.4 ($M^*$).

Beispiel 17:

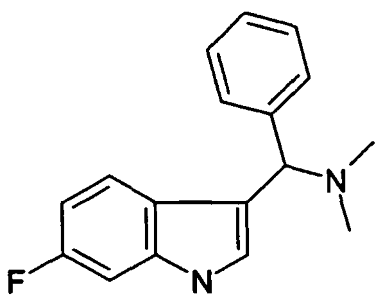

[(6-Fluoro-1H-indol-3-yl)-phenylmethyl]-dimethylamin

Die Herstellung erfolgte gemäß der allgemeinen Synthesevorschrift 4 aus 6-Fluoro-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 224.4 ($M^*$).

Beispiel 18:

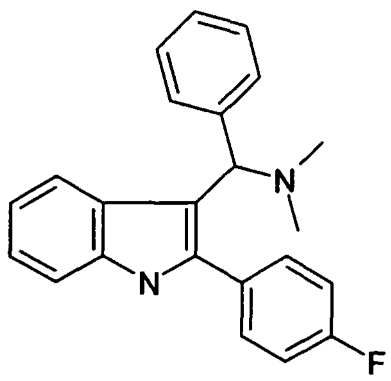

{[2-(4-Fluoro-phenyl)-1H-indol-3-yl]-phenylmethyl}-dimethylamin

Die Herstellung erfolgte gemäß der allgemeinen Synthesevorschrift 4 aus 2-(4-Fluorophenyl)-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 300.5, 224.5 ($M^*$).

Beispiel 19:

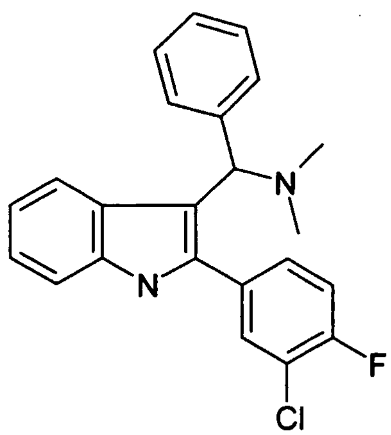

{[2-(3-Chloro-4-fluorophenyl)-1H-indol-3-yl]-phenylmethyl}-dimethylamin

Die Herstellung erfolgte gemäß der allgemeinen Synthesevorschrift 4 aus 2-(3-Chloro-4-fluorophenyl)-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 334.4 ($M^*$).

Beispiel 20:

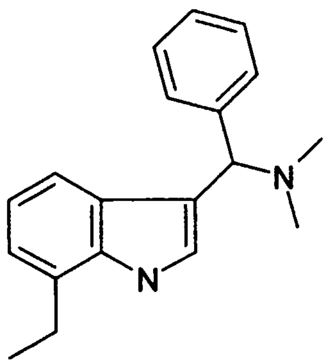

[(7-Ethyl-1H-indol-3-yl)-phenylmethyl]-dimethylamin

Die Herstellung erfolgte gemäß der allgemeinen Synthesevorschrift 4 aus 7-Ethyl-1H-indol und Benzyliden-dimethyl-ammoniumchlorid, welches gemäß Beispiel 1 hergestellt worden ist.

Zur Charakterisierung wurde ein ESI-MS aufgenommen:
MS (EI) m/z: 234.5 ($M^*$).

Beispiel 21:

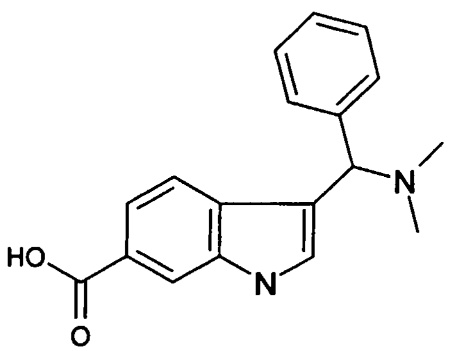

3-(Dimethylaminophenylmethyl)-1H-indol-6-carbonsäure

Die Herstellung erfolgte gemäß der allgemeinen Synthesevorschrift 4 aus 1H-indol-6-carbonsäure und Benzyliden-dimethyl-ammoniumchlorid, welches gemäß Beispiel 1 hergestellt worden ist.

Zur Charakterisierung wurde ein ESI-MS aufgenommen:
MS (EI) m/z: 264.4, 250.5 ($M^*$).

Beispiel 22:

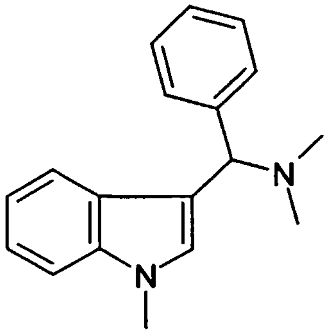

Dimethyl-[(1-methyl-1H-indol-3-yl)-phenylmethyl]-amin Die Herstellung erfolgte gemäß der allgemeinen Synthesevorschrift 4 aus 1-Methyl-1H-indol und Benzyliden-dimethyl-ammoniumchlorid, welches gemäß Beispiel 1 hergestellt worden ist.
Zur Charakterisierung wurde ein ESI-MS aufgenommen:
MS (EI) m/z: 265.1 ($M^*$).

Beispiel 23:

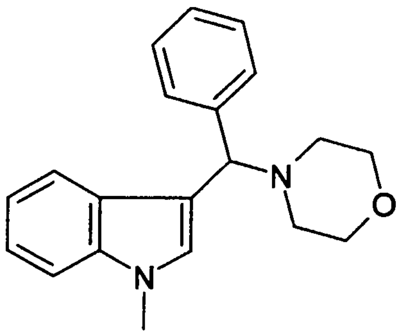

1-Methyl-3-(morpholin-4-yl-phenylmethyl)-1H-indol

1. Stufe

4-Benzyliden-morpholin-4-ium-chlorid

Die Umsetzung von 17,9 ml (0,200 mol) Morpholin und 10,1 ml (0,100 mol) Benzaldehyd gemäß der allgemeinen Synthesevorschrift 2 und nachfolgende Reaktion mit 6,0 ml (0,100 mol) Acetylchlorid gemäß der allgemeinen Synthesevorschrift 3 ergaben 10,1 g (entsprechend 48 % der theoretisch berechneten Ausbeute) an 4-Benzyliden-morpholin-4-ium-chlorid.

2. Stufe

1-Methyl-3-(morpholin-4-yl-phenylmethyl)-1H-indol

Die Herstellung erfolgte gemäß der allgemeinen Synthesevorschrift 4 aus 1-Methyl-1H-indol und 4-Benzyliden-morpholin-4-ium-chlorid.
Zur Charakterisierung wurde ein ESI-MS aufgenommen:
MS (EI) m/z: 220.4 ($M^*$).

Beispiel 24:

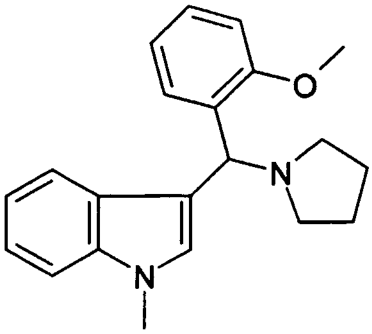

3-[(2-Methoxyphenyl)-pyrrolidin-1-yl-methyl]-1-methyl-1H-indol

1. Stufe

1-(2-Methoxy-benzyliden)-pyrrolidinium-chlorid

Die Umsetzung von 6,9 ml (0,084 mol) Pyrrolidin und 4,8 g (0,035 mol) 2-Methoxybenzaldehyd gemäß der allgemeinen Synthesevorschrift 2 und nachfolgende Reaktion mit 2,1 ml (0,035 mol) Acetylchlorid gemäß der allgemeinen Synthesevorschrift 3 ergaben 6,2 g (entsprechend 78 % der theoretisch berechneten Ausbeute) an 1-(2-Methoxy-benzyliden)-pyrrolidinium-chlorid.

2. Stufe

3-[(2-Methoxyphenyl)-pyrrolidin-1-yl-methyl]-1-methyl-1H-indol

Die Herstellung erfolgte gemäß der allgemeinen Synthesevorschrift 4 aus 1-Methyl-1H-indol und 1-(2-Methoxy-benzyliden)-pyrrolidinium-chlorid.
Zur Charakterisierung wurde ein ESI-MS aufgenommen:
MS (EI) m/z: 250.4 ($M^*$).

Beispiel 25:

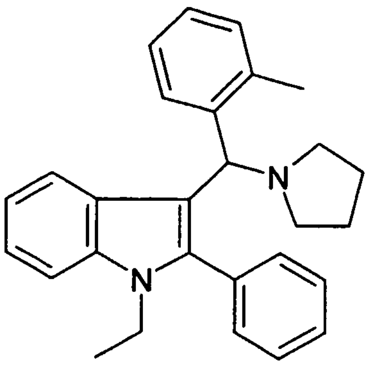

1-Ethyl-2-phenyl-3-(pyrrolidin-1-yl-o-tolylmethyl)-1H-indol

1. Stufe

1-(2-Methyl-benzyliden)-pyrrolidinium- chlorid

Die Umsetzung von 8,2 ml (0,100 mol) Pyrrolidin und 7,0 g (0,050 mol) 2-Methylbenzaldehyd gemäß der allgemeinen Synthesevorschrift 2 und nachfolgende Reaktion mit 3,9 g (0,050 mol) Acetylchlorid gemäß der allgemeinen Synthesevorschrift 3 ergaben 6,6 g (entsprechend 63 % der theoretisch berechneten Ausbeute) an 1-(2-Methyl-benzyliden)-pyrrolidinium-chlorid.

2. Stufe

1-Ethyl-2-phenyl-3-(pyrrolidin-1-yl-*o*-tolylmethyl)-1H-indol

Die Herstellung erfolgte gemäß der allgemeinen Synthesevorschrift 4 aus 1-Ethyl-2-phenyl-1H-indol und 1-(2-Methyl-benzyliden)-pyrrolidinium-chlorid.

Zur Charakterisierung wurde ein ESI-MS aufgenommen:
MS (EI) m/z: 324.4 ($M^*$).

Beispiel 26:

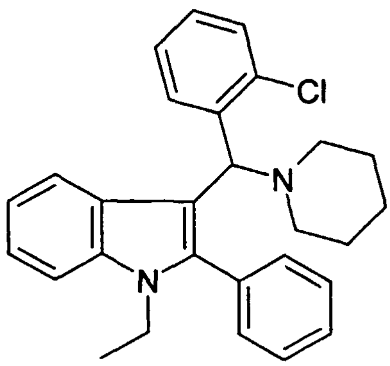

3-[(2-Chlorophenyl)-piperidin-1-yl-methyl]-1-ethyl-2-phenyl-1H-indol

1. Stufe

1-(2-Chlor-benzyliden)-piperidinium-chlorid

Die Umsetzung von 8,5 g (0,100 mol) Piperidin und 7,0 g (0,050 mol) 2-Chlorbenzaldehyd gemäß der allgemeinen Synthesevorschrift 2 und nachfolgende Reaktion mit 3,9 g (0,050 mol) Acetylchlorid gemäß der allgemeinen Synthesevorschrift 3 ergaben 7,1 g (entsprechend 58 % der theoretisch berechneten Ausbeute) an 1-(2-Chlor-benzyliden)-piperidinium-chlorid.

2. Stufe

3-[(2-Chlorophenyl)-piperidin-1-yl-methyl]-1-ethyl-2-phenyl-1H-indol

Die Herstellung erfolgte gemäß der allgemeinen Synthesevorschrift 4 aus 1-Ethyl-2-phenyl-1H-indol und 1-(2-Chlor-benzyliden)-piperidinium-chlorid.

Zur Charakterisierung wurde ein ESI-MS aufgenommen:
MS (EI) m/z: 344.4 ($M^*$).
Beispiel 27:

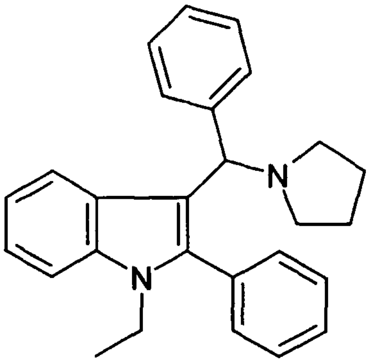

1-Ethyl-2-phenyl-3-(phenylpyrrolidin-1-yl-methyl)-1H-indol

1. Stufe

1-Benzyliden-pyrrolidinium-chlorid

Die Umsetzung von 16,4 ml (0,200 mol) Pyrrolidin und 10,1 ml (0,100 mol) Benzaldehyd gemäß der allgemeinen Synthesevorschrift 2 und nachfolgende Reaktion mit 6,0 ml (0,100 mol) Acetylchlorid gemäß der allgemeinen Synthesevorschrift 3 ergaben 14,1 g (entsprechend 72 % der theoretisch berechneten Ausbeute) an 1-Benzyliden-pyrrolidinium-chlorid.

2. Stufe

1-Ethyl-2-phenyl-3-(phenylpyrrolidin-1-yl-methyl)-1H-indol

Die Herstellung erfolgte gemäß der allgemeinen Synthesevorschrift 4 aus 1-Ethyl-2-phenyl-1H-indol und 1-Benzyliden-pyrrolidinium-chlorid.
Zur Charakterisierung wurde ein ESI-MS aufgenommen:
MS (EI) m/z: 310.5 ($M^*$).
Beispiel 28:

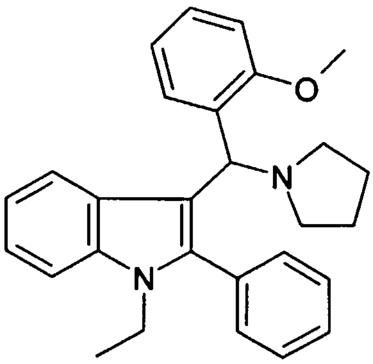

1-Ethyl-3-[(2-methoxyphenyl)-pyrrolidin-1-yl-methyl]-2-phenyl-1H-indol
Die Herstellung erfolgte gemäß der allgemeinen Synthesevorschrift 4 aus 1-Ethyl-2-phenyl-1H-indol und 1-(2-Methoxy-benzyliden)-pyrrolidinium-chlorid, welches gemäß Beispiel 24 hergestellt worden ist.
Zur Charakterisierung wurde ein ESI-MS aufgenommen:
MS (EI) m/z: 340.4 ($M^*$).

Beispiel 29:

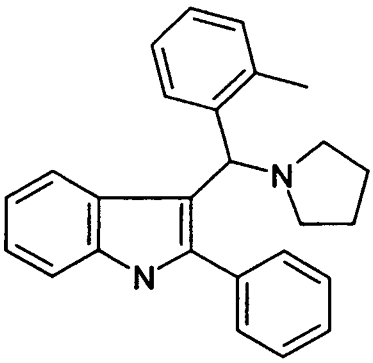

2-Phenyl-3-(pyrrolidin-1-yl-*o*-tolylmethyl)-1H-indol

Die Herstellung erfolgte gemäß der allgemeinen Synthesevorschrift 4 aus 2-Phenyl-1H-indol und 1-(2-Methyl-benzyliden)-pyrrolidinium-chlorid, welches gemäß Beispiel 25 hergestellt worden ist.

Zur Charakterisierung wurde ein ESI-MS aufgenommen:
MS (EI) m/z: 296.5 ($M^*$).

Beispiel 30:

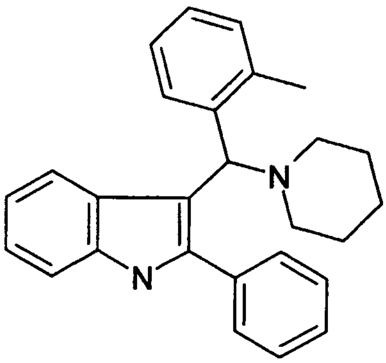

1. Stufe

1-(2-Methyl-benryliden)-piperidinium-chlorid

Die Umsetzung von 9,5 ml (0,096 mol) Piperidin und 4,7 ml (0,040 mol) 2-Methylbenzaldehyd gemäß der allgemeinen Synthesevorschrift 2 und nachfolgende Reaktion mit 2,4 ml (0,040 mol) Acetylchlorid gemäß der allgemeinen Synthesevorschrift 3 ergaben 5,8 g (entsprechend 65 % der theoretisch berechneten Ausbeute) an 1-(2-Methyl-benzyliden)-piperidinium-chlorid.

2. Stufe

2-Phenyl-3-(piperidin-1-yl-*o*-tolylmethyl)-1H-indol

Die Herstellung erfolgte gemäß der allgemeinen Synthesevorschrift 4 aus 2-Phenyl-1H-indol und 1-(2-Methyl-benzyliden)-piperidinium-chlorid.

Zur Charakterisierung wurde ein ESI-MS aufgenommen:
MS (EI) m/z: 296.5 ($M^*$).
Beispiel 31:

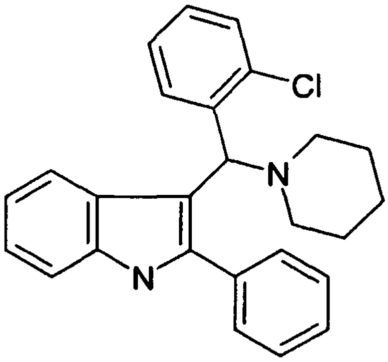

3-[(2-Chlorophenyl)-piperidin-1-yl-methyl]-2-phenyl-1H-indol
Die Herstellung erfolgte gemäß der allgemeinen Synthesevorschrift 4 aus 2-Phenyl-1H-indol und 1-(2-Chlorbenzyliden)-piperidinium-chlorid, welches gemäß Beispiel 26 hergestellt worden ist.
Zur Charakterisierung wurde ein ESI-MS aufgenommen:
MS (EI) m/z: 316.5 ($M^*$).

Beispiel 32:

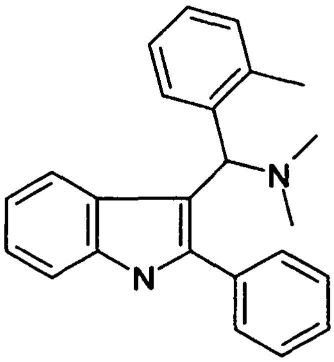

Dimethyl-[(2-phenyl-1H-indol-3-yl)-*o*-tolylmethyl]-amin

1. Stufe

Dimethyl-(2-methyl-benzyliden)-ammonium-chlorid

Die Umsetzung von 14,0 ml (0,108 mol) Dimethylaminlösung und 4,6 ml (0,040 mol) 2-Methylbenzaldehyd gemäß der allgemeinen Synthesevorschrift 1 und nachfolgende Reaktion mit 2,4 ml (0,040 mol) Acetylchlorid gemäß der allgemeinen Synthesevorschrift 3 ergaben 5,3 g (entsprechend 73 % der theoretisch berechneten Ausbeute) an Dimethyl-(2-methyl-benzyliden)-ammonium-chlorid.

2. Stufe

Dimethyl-[(2-phenyl-1H-indol-3-yl)-*o*-tolylmethyl]-amin

Die Herstellung erfolgte gemäß der allgemeinen Synthesevorschrift 4 aus 2-Phenyl-1H-indol und Dimethyl-(2-methyl-benzyliden)-ammonium-chlorid.
Zur Charakterisierung wurde ein ESI-MS aufgenommen:
MS (EI) m/z: 296.6 ($M^*$).

Beispiel 33:

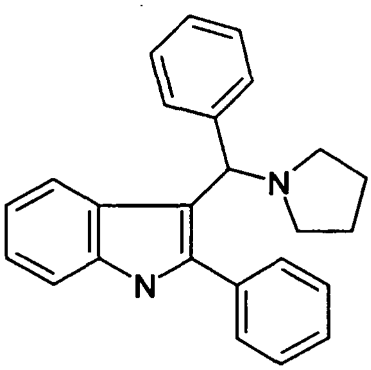

2-Phenyl-3-(phenylpyrrolidin-1-yl-methyl)-1H-indol
Die Herstellung erfolgte gemäß der allgemeinen Synthesevorschrift 4 aus 2-Phenyl-1H-indol und 1-Benzyliden-pyrrolidinium-chlorid, welches gemäß Beispiel 27 hergestellt worden ist.
Zur Charakterisierung wurde ein ESI-MS aufgenommen:
MS (EI) m/z: 282.5 ($M^*$).

Beispiel 34:

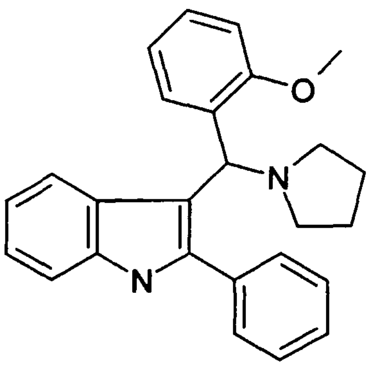

3-[(2-Methoxyphenyl)-pyrrolidin-1-yl-methyl]-2-phenyl-1H-indol
Die Herstellung erfolgte gemäß der allgemeinen Synthesevorschrift 4 aus 2-Phenyl-1H-indol und 1-(2-Methoxy-benzyliden)-pyrrolidinium-chlorid, welches gemäß Beispiel 24 hergestellt worden ist.
Zur Charakterisierung wurde ein ESI-MS aufgenommen:
MS (EI) m/z: 312.3 ($M^*$).

Beispiel 35:

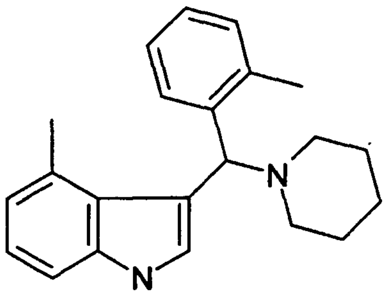

4-Methyl-3-(piperidin-1-yl-o-tolylmethyl)-1H-indol

Die Herstellung erfolgte gemäß der allgemeinen Synthesevorschrift 4 aus 4-Methyl-1H-indol und 1-(2-Methylbenzyliden)-piperidinium-chlorid, welches gemäß Beispiel 30 hergestellt worden ist.

Zur Charakterisierung wurde ein ESI-MS aufgenommen:
MS (EI) m/z: 234.5 ($M^*$).

Beispiel 36:

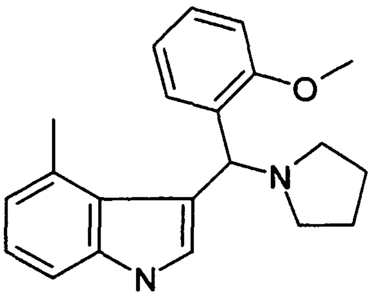

3-[(2-Methoxyphenyl)-pyrrolidin-1-yl-methyl]-4-methyl-1H-indol

Die Herstellung erfolgte gemäß der allgemeinen Synthesevorschrift 4 aus 4-Methyl-1H-indol und 1-(2-Methoxybenzyliden)-pyrrolidinium-chlorid, welches gemäß Beispiel 24 hergestellt worden ist.

Zur Charakterisierung wurde ein ESI-MS aufgenommen:
MS (EI) m/z: 250.4 ($M^*$).

Beispiel 37:

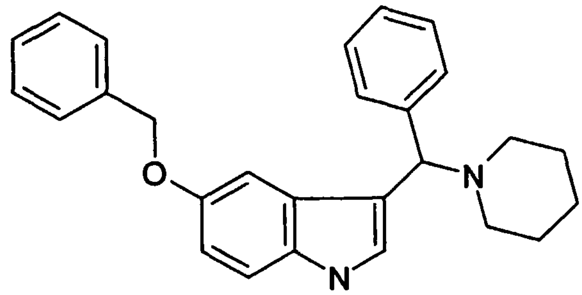

5-Benzyloxy-3-(phenylpiperidin-1-yl-methyl)-1H-indol

1. Stufe

1-Benzyliden-piperidinium-chlorid

Die Umsetzung von 19,8 ml (0,200 mol) Piperidin und 10,1 ml (0,100 mol) Benzaldehyd gemäß der allgemeinen Synthesevorschrift 2 und nachfolgende Reaktion mit 6,0 ml (0,100 mol) Acetylchlorid gemäß der allgemeinen Synthesevorschrift 3 ergaben 11,7 g (entsprechend 56 % der theoretisch berechneten Ausbeute) an 1-Benzyliden-piperidinium-chlorid.

2. Stufe

5-Benzyloxy-3-(phenylpiperidin-1-yl-methyl)-1H-indol

Die Herstellung erfolgte gemäß der allgemeinen Synthesevorschrift 4 aus 5-Benzyloxy-1H-indol und 1-Benzyliden-piperidinium-chlorid.
Zur Charakterisierung wurde ein ESI-MS aufgenommen:
MS (EI) m/z: 397.3, 312.5 ($M^*$).

Beispiel 38:

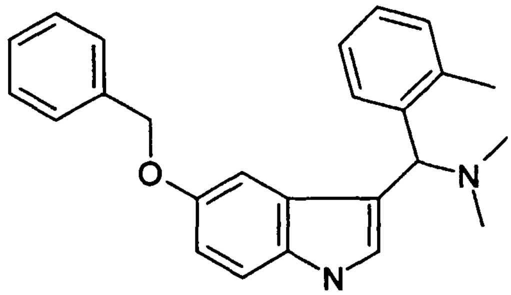

[(5-Benzyloxy-1H-indol-3-yl)-*o*-tolylmethyl]-dimethylamin
Die Herstellung erfolgte gemäß der allgemeinen Synthesevorschrift 4 aus 5-Benzyloxy-1H-indol und Dimethyl-(2-methyl-benzyliden)-ammonium-chlorid, welches gemäß Beispiel 32 hergestellt worden ist.
Zur Charakterisierung wurde ein ESI-MS aufgenommen:
MS (EI) m/z: 326.5 ($M^*$).

Beispiel 39:

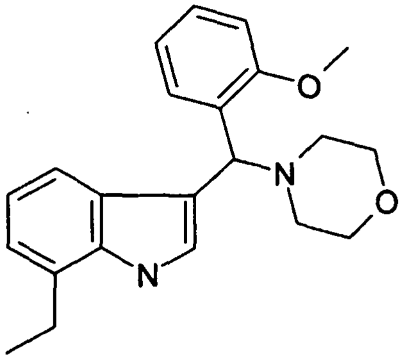

7-Ethyl-3-[(2-methoxyphenyl)-morpholin-4-yl-methyl]-1H-indol

1. Stufe

4-(2-Methoxy-benzyliden)-morpholin-4-ium-chlorid

Die Umsetzung von 18,8 ml (0,216 mol) Morpholin und 12,4 g (0,09 mol) 2-Methoxybenzaldehyd gemäß der allgemeinen Synthesevorschrift 2 und nachfolgende Reaktion mit 5,3 ml (0,110 mol) Acetylchlorid gemäß der allgemeinen Synthesevorschrift 3 ergaben 7,61 g (entsprechend 38 % der theoretisch berechneten Ausbeute) an 4-(2-Methoxy-benzyliden)-morpholin-4-ium-chlorid.

2. Stufe

7-Ethyl-3-[(2-methoxyphenyl)-morpholin-4-yl-methyl]-1H-indol

Die Herstellung erfolgte gemäß der allgemeinen Synthesevorschrift 4 aus 7-Ethyl-1H-indol und 4-(2-Methoxy-benzyliden)-morpholin-4-ium-chlorid.

Zur Charakterisierung wurde ein ESI-MS aufgenommen:
MS (EI) m/z: 264.3 ($M^*$).

Beispiel 40:

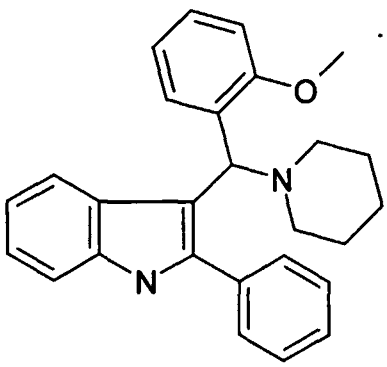

3-[(2-Methoxyphenyl)-piperidin-1-yl-methyl]-2-phenyl-1H-indol

1. Stufe

1-(2-Methoxy-benzyliden)-piperidinium-chlorid

Die Umsetzung von 18,4 g (0,216 mol) Piperidin und 25,9 g (0,090 mol) 2-Methoxybenzaldehyd gemäß der allgemeinen Synthesevorschrift 2 und nachfolgende Reaktion mit 5,3 ml (0,11 mol) Acetylchlorid gemäß der allgemeinen Synthesevorschrift 3 ergaben 13,4 g (entsprechend 62 % der theoretisch berechneten Ausbeute) an 1-(2-Methoxy-benzyliden)-piperidinium-chlorid.

2. Stufe

3-[(2-Methoxyphenyl)-piperidin-1-yl-methyl]-2-phenyl-1H-indol

Die Herstellung erfolgte gemäß der allgemeinen Synthesevorschrift 4 aus 2-Phenyl-1H-indol und 1-(2-Methoxy-benzyliden)-piperidinium-chlorid.
Zur Charakterisierung wurde ein ESI-MS aufgenommen:
MS (EI) m/z: 312.3 ($M^*$).

Beispiel 41:

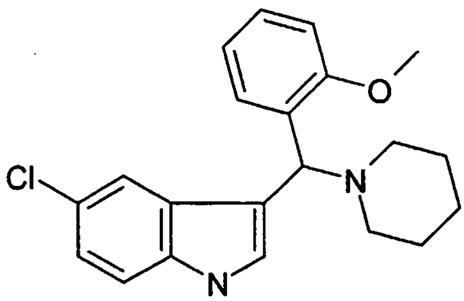

5-Chloro-3-[(2-methoxyphenyl)-piperidin-1-yl-methyl]-1H-indol
Die Herstellung erfolgte gemäß der allgemeinen Synthesevorschrift 4 aus 5-Chloro-1H-indol und 1-(2-Methoxy-benzyliden)-piperidinium-chlorid, welches gemäß Beispiel 40 hergestellt worden ist.
Zur Charakterisierung wurde ein ESI-MS aufgenommen:
MS (EI) m/z: 355.0, 270.3 ($M^*$).

Beispiel 42:

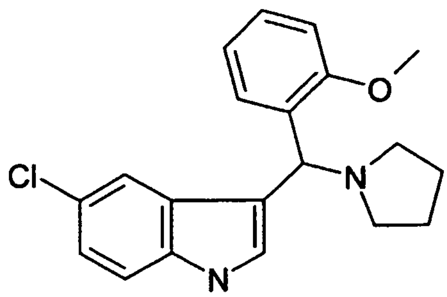

5-Chloro-3-[(2-methoxyphenyl)-pyrrolidin-1-yl-methyl]-1H-indol

Die Herstellug erfolgte gemäß der allgemeinen Synthesevorschrift 4 aus 5-Chloro-1H-indol und 1-(2-Methoxybenzyliden)-pyrrolidinium-chlorid, welches gemäß Beispiel 24 hergestellt worden ist.

Zur Charakterisierung wurde ein ESI-MS aufgenommen:
MS (EI) m/z: 340.9, 270.2 (M*).

Beispiel 43:

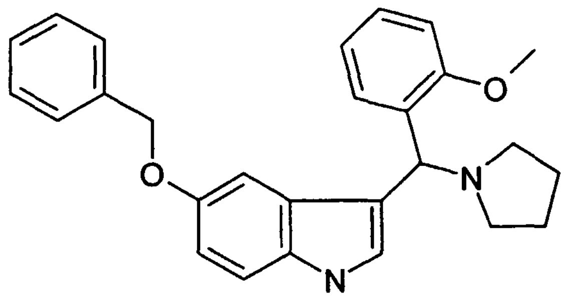

5-Benzyloxy-3-[(2-methoxyphenyl)-pyrrolidin-1-yl-methyl]-1H-indol

Die Herstellung erfolgte gemäß der allgemeinen Synthesevorschrift 4 aus 5-Benzyloxy-1H-indol und 1-(2-Methoxy-benzyliden)-pyrrolidinium-chlorid, welches gemäß Beispiel 24 hergestellt worden ist.

Zur Charakterisierung wurde ein ESI-MS aufgenommen:
MS (EI) m/z: 412.9, 342.3 ($M^*$).

Beispiel 44:

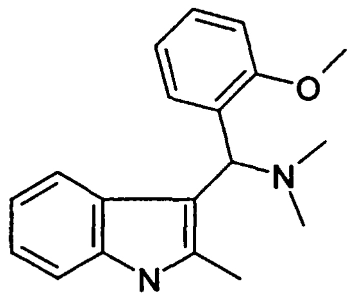

[(2-Methoxyphenyl)-(2-methyl-1H-indol-3-yl)methyl]-dimethylamin

1.Stufe

(2-Methoxy-benzyliden)-dimethyl-ammonium-chlorid

Die Umsetzung von 17,0 ml (0,135 mol) Dimethylaminlösung und 6,8 g (0,050 mol) 2-Methoxybenzaldehyd gemäß der allgemeinen Synthesevorschrift 1 und nachfolgende Reaktion mit 3,0 ml (0,050 mol) Acetylchlorid gemäß der allgemeinen Synthesevorschrift 3 ergaben 4,8 g (entsprechend 48 % der theoretisch berechneten Ausbeute) an 2-Methoxy-benzyliden-dimethylammonium-chlorid.

2. Stufe

[(2-Methoxyphenyl)-(2-methyl-1H-indol-3-yl)-methyl]-dimethylamin

Die Herstellung erfolgte gemäß der allgemeinen Synthesevorschrift 4 aus 2-Methyl-1H-indol und (2-Methoxybenzyliden)-dimethyl-ammonium-chlorid.

Zur Charakterisierung wurde ein ESI-MS aufgenommen:
MS (EI) m/z: 250.3 ($M^*$).

Beispiel 45:

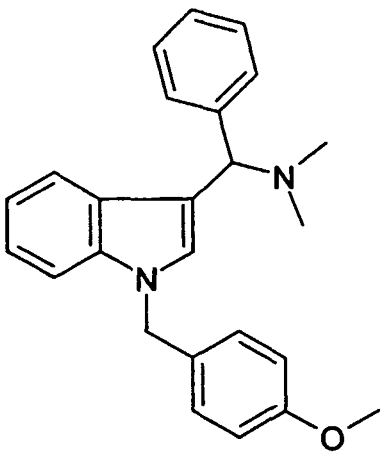

{[1-(4-Methoxybenzyl)-1H-indol-3-yl]-phenylmethyl}-dimethylamin

Die Herstellung erfolgte gemäß der allgemeinen Synthesevorschrift 4 und 5 aus 1H-Indol, Benzyliden-dimethylammoniumchlorid und 4-Methoxybenzylchlorid.

Zur Charakterisierung wurde ein ESI-MS aufgenommen:
MS (EI) m/z: 327.1 ($M^*$).

Beispiel 46:

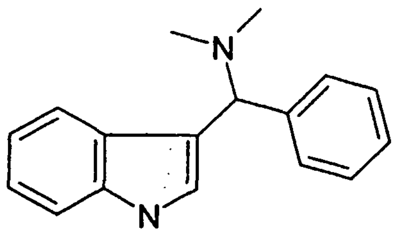

[(1*H*-Indol-3-yl)-phenylmethyl]-dimethylamin

Die Herstellung erfolgte gemäß der allgemeinen Synthesevorschrift 4 aus 1H-indol und Benzyliden-dimethylammoniumchlorid, welches gemäß Beispiel 1 hergestellt worden ist.

Der Nachweis der Struktur erfolgte mittels: $^{13}$C-NMR, δ = 144,35; 136,73; 128,30; 127,89; 126,55; 126,28; 123,32; 121,20; 119,96; 118,75; 111,72; 69,67; 44,49 ppm.

Beispiel 47:

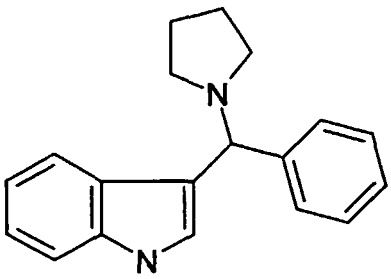

3-(Phenylpyrrolidin-1-yl-methyl)-1*H*-indol

Die Herstellung erfolgte gemäß der allgemeinen Synthesevorschrift 4 aus 1H-indol und 1-Benzyliden-pyrrolidinium-chlorid, welches gemäß Beispiel 27 hergestellt worden ist.

Der Nachweis der Struktur erfolgte mittels ESI-MS: berechnete Masse 276,38 g/mol, gefundene Masse M+H = 276,9 g/mol.

Beispiel 48:

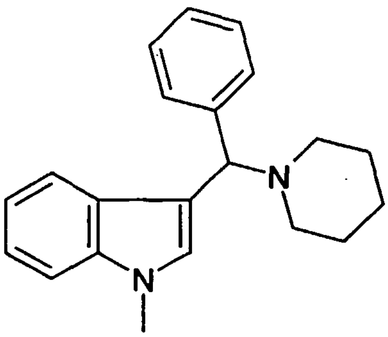

1-Methyl-3-(phenylpiperidin-1-yl-methyl)-1*H*-indol

Die Herstellung erfolgte gemäß der allgemeinen Synthesevorschrift 4 aus 1-Methyl-1H-indol und 1-Benzyliden-piperidinium-chlorid, welches gemäß Beispiel 37 hergestellt worden ist.

Der Nachweis der Struktur erfolgte mittels ESI-MS: berechnete Masse 304,44 g/mol, gefundene Masse M+H = 304,8 g/mol.

Beispiel 49:

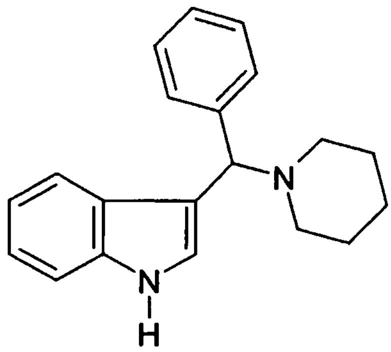

3-(Phenylpiperidin-1-yl-methyl)-1*H*-indol

Die Herstellung erfolgte gemäß der allgemeinen Synthesevorschrift 4 aus 1H-indol und 1-Benzyliden-piperidinium-chlorid, welches gemäß Beispiel 37 hergestellt worden ist.

Der Nachweis der Struktur erfolgte mittels ESI-MS: berechnete Masse 290,41 g/mol, gefundene Masse M+H = 290,9 g/mol.

Beispiel 50:

5-Benzyloxy-3-(phenyl-pyrrolidin-1-yl-methyl)-1H-indol

Die Herstellung erfolgte gemäß der allgemeinen Synthesevorschrift 4 aus 5-Benzyloxyindol und Benzyliden-pyrrolidinium-chlorid, welches gemäß Beispiel 27 hergestellt worden ist.

Beispiel 51:

[(5-Brom-2-fluor-phenyl)-(4-nitro-1H-indol-3-yl)-methyl]-dimethyl-amin

Die Herstellung erfolgte gemäß der allgemeinen Synthesevorschrift 4 aus 4-Nitro-Indol und (5-Brom-2-fluor-benzyliden)-dimethyl-ammoniumchlorid, welches gemäß Beispiel 24 aus 5-Brom-2-fluor-benzaldehyd und Dimethylamin hergestellt worden ist.

Beispiel 52:

[(5-Brom-2-fluor-phenyl)-(1-methyl-1H-indol-3-yl)-methyl]-dimethyl-amin

Die Herstellung erfolgte gemäß der allgemeinen Synthesevorschrift 4 aus 1-Methylindol und (5-Brom-2-fluor-benzyliden)-dimethyl-ammoniumchlorid, welches gemäß Beispiel 24 aus 5-Brom-2-fluor-benzaldehyd und Dimethylamin hergestellt worden ist.

Beispiel 53:

[(2-Chlor-6-fluor-phenyl)-(2-phenyl-1H-indol-3-yl)-methyl]-dimethylamin

Die Herstellung erfolgte gemäß der allgemeinen Synthesevorschrift 4 aus 2-Phenylindol und (2-Chlor-6-fluor-benzyliden)-dimethyl-ammoniumchlorid, welches gemäß Beispiel 24 aus 2-Chlor-6-fluor-benzaldehyd und Dimethylamin hergestellt worden ist.

Beispiel 54:

[(2-Chlor-6-fluor-phenyl)-(1-ethyl-2-phenyl-1H-indol-3-yl)-methyl]-dimethylamin

Die Herstellung erfolgte gemäß der allgemeinen Synthesevorschrift 4 aus 1-Ethylindol und (2-Chlor-6-fluor-benzyliden)-dimethyl-ammoniumchlorid, welches gemäß Beispiel 24 aus 2-Chlor-6-fluor-benzaldehyd und Dimethylamin

hergestellt worden ist.

Beispiel 55:

[(2-Chlor-6-fluor-phenyl)-(4-nitro-1H-indol-3-yl)-methyl]-dimethyl-amin

Die Herstellung erfolgte gemäß der allgemeinen Synthesevorschrift 4 aus 4-Nitroindol und (2-Chlor-6-fluor-benzyliden)-dimethyl-ammoniumchlorid, welches gemäß Beispiel 24 aus 2-Chlor-6-fluor-benzaldehyd und Dimethylamin hergestellt worden ist.

Beispiel 56:

[(2-Chlor-6-fluor-phenyl)-(1-methyl-1H-indol-3-yl)-methyl]-dimethyl-amin

Die Herstellung erfolgte gemäß der allgemeinen Synthesevorschrift 4 aus 1-Methylindol und (2-Chlor-6-fluor-benzyliden)-dimethyl-ammoniumchlorid, welches gemäß Beispiel 24 aus 2-Chlor-6-fluor-benzaldehyd und Dimethylamin hergestellt worden ist.

Beispiel 57:

[(2-Brom-phenyl)-(4-nitro-1H-indol-3-yl)-methyl]-dimethyl-amin

Die Herstellung erfolgte gemäß der allgemeinen Synthesevorschrift 4 aus 4-Nitroindol und (2-Brom-benzyliden)-dimethyl-ammoniumchlorid, welches gemäß Beispiel 24 aus 2-Brom-benzaldehyd und Dimethylamin hergestellt worden ist.

Beispiel 58:

[(2-Brom-phenyl)-(1-methyl-1H-indol-3-yl)-methyl]-dimethyl-amin

Die Herstellung erfolgte gemäß der allgemeinen Synthesevorschrift 4 aus 1-Methylindol und (2-Brom-benzyliden)-dimethyl-ammoniumchlorid, welches gemäß Beispiel 24 aus 2-Brom-benzaldehyd und Dimethylamin hergestellt worden ist.

Beispiel 59:

[(3-Brom-phenyl)-(7-ethyl-1H-indol-3-yl)-methyl]-dimethyl-amin

Die Herstellung erfolgte gemäß der allgemeinen Synthesevorschrift 4 aus 7-Ethylindol und (3-Brom-benzyliden)-dimethyl-ammoniumchlorid, welches gemäß Beispiel 24 aus 3-Brom-benzaldehyd und Dimethylamin hergestellt worden ist.

Beispiel 60:

[(3-Brom-phenyl)-(1-methyl-1H-indol-3-yl)-methyl]-dimethyl-amin

Die Herstellung erfolgte gemäß der allgemeinen Synthesevorschrift 4 aus 1-Methylindol und (3-Brom-benzyliden)-dimethyl-ammoniumchlorid, welches gemäß Beispiel 24 aus 3-Brom-benzaldehyd und Dimethylamin hergestellt worden ist.

Beispiel 61:

[(4-tert-Butyl-phenyl)-(2-methyl-1H-indol-3-y-)-methyl]-dimethyl-amin

Die Herstellung erfolgte gemäß der allgemeinen Synthesevorschrift 4 aus 2-Methylindol und (4-tert-Butyl-benzyliden)-dimethyl-ammoniumchlorid, welches gemäß Beispiel 24 aus 4-tert-Butyl-benzaldehyd und Dimethylamin hergestellt worden ist.

Beispiel 62:

[(4-tert-Butyl-phenyl)-(1-ethyl-2-phenyl-1H-indol-3-yl)-methyl]-dimethyl-amin

Die Herstellung erfolgte gemäß der allgemeinen Synthesevorschrift 4 aus 1-Ethyl-2-phenylindol und (4-tert-Butyl-benzyliden)-dimethyl-ammoniumchlorid, welches gemäß Beispiel 24 aus 4-tert-Butyl-benzaldehyd und Dimethylamin hergestellt worden ist.

Beispiel 63:

[(4-tert-Butyl-phenyl)-(1-methyl-1H-indol-3-yl)-methyl]-dimethyl-amin

Die Herstellung erfolgte gemäß der allgemeinen Synthesevorschrift 4 aus 1-Methylindol und (4-tert-Butyl-benzyliden)-dimethyl-ammoniumchlorid, welches gemäß Beispiel 24 aus 4-tert-Butyl-benzaldehyd und Dimethylamin hergestellt worden ist.

Beispiel 64:

[(2-Chlor-4-fluor-phenyl)-(4-nitro-1H-indol-3-yl)-methyl]-dimethyl-amin

Die Herstellung erfolgte gemäß der allgemeinen Synthesevorschrift 4 aus 4-Nitroindol und (2-Chlor-4-fluor-benzyliden)-dimethyl-ammoniumchlorid, welches gemäß Beispiel 24 aus 2-Chlor-4-fluor-benzaldehyd und Dimethylamin hergestellt worden ist.

Beispiel 65:

[(2-Chlor-4-fluor-phenyl)-(4-methyl-1H-indol-3-yl)-methyl]-dimethyl-amin

Die Herstellung erfolgte gemäß der allgemeinen Synthesevorschrift 4 aus 4-Methylindol und (2-Chlor-4-fluor-benzyliden)-dimethyl-ammoniumchlorid, welches gemäß Beispiel 24 aus 2-Chlor-4-fluor-benzaldehyd und Dimethylamin hergestellt worden ist.

Beispiel 66:

[(2-Chlor-6-fluor-phenyl)-(2-phenyl-1H-indol-3-yl)-methyl]-dimethyl-amin

Die Herstellung erfolgte gemäß der allgemeinen Synthesevorschrift 4 aus 2-Phenylindol und (2-Chlor-6-fluor-benzyliden)-dimethyl-ammoniumchlorid, welches gemäß Beispiel 24 aus 2-Chlor-6-fluor-benzaldehyd und Dimethylamin hergestellt worden ist.

Beispiel 67:

{(2-Chlor-6-fluor-phenyl)-[2-(4-chlor-phenyl)-1H-indol-3-yl]-methyl}-dimethylamin

Die Herstellung erfolgte gemäß der allgemeinen Synthesevorschrift 4 aus 2-(4-Chlorphenyl)-indol und (2-Chlor-6-fluor-benzyliden)-dimethyl-ammoniumchlorid, welches gemäß Beispiel 24 aus 2-Chlor-6-fluor-benzaldehyd und Dimethylamin hergestellt worden ist.

Beispiel 68:

[(2-Chlor-6-fluor-phenyl)-(1-methyl-1H-indol-3-yl)-methyl]-dimethyl-amin

Die Herstellung erfolgte gemäß der allgemeinen Synthesevorschrift 4 aus 1-Methylindol und (2-Chlor-6-fluor-benzyliden)-dimethyl-ammoniumchlorid, welches gemäß Beispiel 24 aus 2-Chlor-6-fluor-benzaldehyd und Dimethylamin hergestellt worden ist.

Beispiel 69:

[(3-Chlor-phenyl)-(1-methyl-1H-indol-3-yl)-methyl]-dimethyl-amin

Die Herstellung erfolgte gemäß der allgemeinen Synthesevorschrift 4 aus 1-Methylindol und (3-Chlor-benzyliden)-dimethyl-ammoniumchlorid, welches gemäß Beispiel 24 aus 3-Chlor-benzaldehyd und Dimethylamin hergestellt worden ist.

Beispiel 70:

[(2,3-Dichlor -phenyl)-(1-methyl-1H-indol-3-yl)-methyl]-dimethyl-amin

Die Herstellung erfolgte gemäß der allgemeinen Synthesevorschrift 4 aus 1-Methylindol und (2,3-Dichlor-benzyliden)-dimethyl-ammoniumchlorid, welches gemäß Beispiel 24 aus 2,3-Dichlor-benzaldehyd und Dimethylamin hergestellt worden ist.

Beispiel 71:

[(2,4-Dichlor-phenyl)-(1-methyl-1H-indol-3-yl)methyl]-dimethyl-amin

Die Herstellung erfolgte gemäß der allgemeinen Synthesevorschrift 4 aus 1-Methylindol und (2,4-Dichlor-benzyliden)-dimethyl-ammoniumchlorid, welches gemäß Beispiel 24 aus 2,4-Dichlor-benzaldehyd und Dimethylamin hergestellt worden ist.

Beispiel 72:

[(4-tert-Butyl-phenyl)-(1-ethyl-2-phenyl-1H-indol-3-yl)-methyl]-dimethyl-amin

Die Herstellung erfolgte gemäß der allgemeinen Synthesevorschrift 4 aus 1-Ethyl-2-phenyl-1H-indol und (4-tert-Butyl-benzyliden)-dimethylammoniumchlorid, das gemäß Beispiel 44 aus 4-tert-Butylbenzaldehyd und Dimethylamin hergestellt worden ist.

Beispiel 73:

[(2-Chlor-4-fluor-phenyl)-(2-methyl-1H-indol-3-yl)-methyl]-dimethyl-amin

Die Herstellung erfolgte gemäß der allgemeinen Synthesevorschrift 4 aus 2-Methyl-1H-indol und (2-Chlor-4-fluor-benzyliden)-dimethylammoniumchlorid, das gemäß Beispiel 44 aus 2-Chlor-4-fluor-benzaldehyd und Dimethylamin hergestellt worden ist.

Beispiel 74:

[(2-Chlor-6-fluor-phenyl)-(2-methyl-1H-indol-3-yl)-methyl]-dimethyl-amin

Die Herstellung erfolgte gemäß der allgemeinen Synthesevorschrift 4 aus 2-Methyl-1H-indol und (2-Chlor-6-fluor-benzyliden)-dimethylammoniumchlorid das gemäß Beispiel 44 aus 2-Chlor-6-fluor-benzaldehyd und Dimethylamin hergestellt worden ist.

Beispiel 75:

[(2,3-Dimethoxy-phenyl)-(4-nitro-1H-indol-3yl)-methyl]-dimethylamin

Die Herstellung erfolgte gemäß der allgemeinen Synthesevorschrift 4 aus 4-Nitro-1H-indol und (2,3-Dimethoxy-benzyliden)-dimethylammoniumchlorid, das gemäß Beispiel 44 aus 2,3-Dimethoxy-benzaldehyd und Dimethylamin hergestellt worden ist.

Beispiel 76:

3-[(2,3-Dimethoxy-phenyl)-dimethylamino-methyl]-4,7-dihydro-1H-indol-6-carbonsäure

Die Herstellung erfolgte gemäß der allgemeinen Synthesevorschrift 4 aus 1H-indol-6-carbonsäure und (2,3-Dimethoxy-benzyliden)-dimethylammoniumchlorid, das gemäß Beispiel 44 aus 2,3-Dimethoxy-benzaldehyd und Dimethylamin hergestellt worden ist.

Beispiel 77:

3-[(2,3-Dimethoxy-phenyl)-dimethylamino-methyl]-5-hydroxy-1H-indol-2-carbonsäure

Die Herstellung erfolgte gemäß der allgemeinen Synthesevorschrift 4 aus 1H-indol-5-hydroxy-2-carbonsäure und (2,3-Dimethoxy-benzyliden)-dimethylammoniumchlorid, das gemäß Beispiel 44 aus 2,3-Dimethoxy-benzaldehyd und Dimethylamin hergestellt worden ist.

Beispiel 78:

[(3,4-Dimethoxy-phenyl)-(2-methyl-1H-indol-3-yl)-methyl]-dimethyl-amin

Die Herstellung erfolgte gemäß der allgemeinen Synthesevorschrift 4 aus 2-Methyl-1H-indol und (3,4-Dimethoxy-benzyliden)-dimethylammoniumchlorid, das gemäß Beispiel 44 aus 3,4-Dimethoxy-benzaldehyd und Dimethylamin hergestellt worden ist.

Beispiel 79:

[[2-(4-Chlor-phenyl)-1H-indol-3-yl]-(3,4-dimethoxy-phenyl)-methyl]-dimethylamin

Die Herstellung erfolgte gemäß der allgemeinen Synthesevorschrift 4 aus 2-(4-Chlor-phenyl)-1H-indol und (3,4-Dimethoxy-benzyliden)-dimethylammoniumchlorid, das gemäß Beispiel 44 aus 3,4-Dimethoxy-benzaldehyd und Dimethylamin hergestellt worden ist.

Beispiel 80:

[(3,4-Dimethoxy-phenyl)-(1-ethyl-2-phenyl-1H-indol-3-yl)-methyl]-dimethyl-amin

Die Herstellung erfolgte gemäß der allgemeinen Synthesevorschrift 4 aus 1-Ethyl-2-Phenyl-1H-indol und (3,4-Dimethoxy-benzyliden)-dimethylammoniumchlorid, das gemäß Beispiel 44 aus 3,4-Dimethoxy-benzaldehyd und Dimethylamin hergestellt worden ist.

Beispiel 81:

[(3,4-Dimethoxy-phenyl)-(7-ethyl-1H-indol-3-yl)-methyl]-dimethyl-amin

Die Herstellung erfolgte gemäß der allgemeinen Synthesevorschrift 4 aus 7-Ethyl-1H-indol und (3,4-Dimethoxy-benzyliden)-dimethylammoniumchlorid, das gemäß Beispiel 44 aus 3,4-Dimethoxy-benzaldehyd und Dimethylamin hergestellt worden ist.

Beispiel 82:

[(3,4-Dimethoxy-phenyl)-(1-methyl-1H-indol-3-yl)-methyl]-dimethyl-amin

Die Herstellung erfolgte gemäß der allgemeinen Synthesevorschrift 4 aus 1-Methyl-1H-indol und (3,4-Dimethoxy-benzyliden)-dimethylammoniumchlorid, das gemäß Beispiel 44 aus 3,4-Dimethoxy-benzaldehyd und Dimethylamin hergestellt worden ist.

Beispiel 83:

[(3,4-Dimethoxy-phenyl)-[2-(4-fluor-phenyl)-1H-indol-3-yl]-methyl}-dimethylamin

Die Herstellung erfolgte gemäß der allgemeinen Synthesevorschrift 4 aus 2-(4-Fluor-phenyl)-1H-indol und (3,4-Dimethoxy-benzyliden)-dimethylammoniumchlorid, das gemäß Beispiel 44 aus 3,4-Dimethoxy-benzaldehyd und Dimethylamin hergestellt worden ist.

Beispiel 84:

[[2-(3-Chlor-4-fluor-phenyl)-1H-indol-3-yl]-(3,4-dimethoxy-phenyl)-methyl]-dimethyl-amin

Die Herstellung erfolgte gemäß der allgemeinen Synthesevorschrift 4 aus 2-(3-Chlor-4-fluor-phenyl)-1H-indol und (3,4-Dimethoxy-benzyliden)-dimethylammoniumchlorid, das gemäß Beispiel 44 aus 3,4-Dimethoxy-benzaldehyd und Dimethylamin hergestellt worden ist.

Beispiel 85:

[(5-Chlor-1H-indol-3-yl)-(2-fluor-phenyl)-methyl]-dimethyl-amin

Die Herstellung erfolgte gemäß der allgemeinen Synthesevorschrift 4 aus 5-Chlor-1H-indol und (2-Fluor-benzyliden)-dimethylammoniumchlorid, das gemäß Beispiel 44 aus 2-Fluor-benzaldehyd und Dimethylamin hergestellt worden ist.

Beispiel 86:

[(4-Fluor-phenyl)-(4-methyl-1H-indol-3-yl)-methyl]-dimethyl-amin

Die Herstellung erfolgte gemäß der allgemeinen Synthesevorschrift 4 aus 4-Methyl-1H-indol und (4-Fluor-benzyliden)-dimethylammoniumchlorid, das gemäß Beispiel 44 aus 4-Fluor-benzaldehyd und Dimethylamin hergestellt worden ist.

Beispiel 87:

[(7-Ethyl-1H-indol-3-yl)-(2-methoxy-phenyl)methyl]-dimethyl-amin

Die Herstellung erfolgte gemäß der allgemeinen Synthesevorschrift 4 aus 7-Ethyl-1H-indol und (2-Methoxy-benzyliden)-dimethylammoniumchlorid, das gemäß Beispiel 44 aus 2-Methoxy-benzaldehyd und Dimethylamin hergestellt worden ist.

Beispiel 88:

Dimethyl-[(2-methyl-1H-indol-3-yl)-o-tolyl-methyl]-amin

Die Herstellung erfolgte gemäß der allgemeinen Synthesevorschrift 4 aus 2-Methyl-1H-indol und (2-Methyl-benzyliden)-dimethylammoniumchlorid, das gemäß Beispiel 44 aus 2-Methyl-benzaldehyd und Dimethylamin hergestellt worden ist.

Beispiel 89:

[(7-Ethyl-1H-indol-3-yl)-o-tolyl-methyl]-dimethyl-amin

Die Herstellung erfolgte gemäß der allgemeinen Synthesevorschrift 4 aus 7-Ethyl-1H-indol und (2-Methyl-benzyliden)-dimethylammoniumchlorid, das gemäß Beispiel 44 aus 2-Methyl-benzaldehyd und Dimethylamin hergestellt worden ist.

Beispiel 90:

Dimethyl-[(1-methyl-1H-indol-3-yl)-o-tolyl-methyl]-amin

Die Herstellung erfolgte gemäß der allgemeinen Synthesevorschrift 4 aus 1-Methyl-1H-indol und (2-Methyl-benzyliden)-dimethylammoniumchlorid, das gemäß Beispiel 44 aus 2-Methyl-benzaldehyd und Dimethylamin hergestellt worden ist.

Beispiel 91:

[(5-Chlor-1H-indol-3-yl)-o-tolyl-methyl]-dimethyl-amin

Die Herstellung erfolgte gemäß der allgemeinen Synthesevorschrift 4 aus 5-Chlor-1H-indol und (2-Methyl-benzyliden)-dimethylammoniumchlorid, das gemäß Beispiel 44 aus 2-Methyl-benzaldehyd und Dimethylamin hergestellt worden ist.

Beispiel 92:

[(5-Chlor-1H-indol-3-yl)-m-tolyl-methyl]-dimethyl-amin

Die Herstellung erfolgte gemäß der allgemeinen Synthesevorschrift 4 aus 5-Chlor-1H-indol und (3-Methyl-benzyliden)-dimethylammoniumchlorid, das gemäß Beispiel 44 aus 3-Methyl-benzaldehyd und Dimethylamin hergestellt worden ist.

Beispiel 93:

Dimethyl-[(2-methyl-1H-indol-3-yl)-p-tolyl-methyl]-amin

Die Herstellung erfolgte gemäß der allgemeinen Synthesevorschrift 4 aus 2-Methyl-1H-indol und (4-Methyl-benzyliden)-dimethylammoniumchlorid, das gemäß Beispiel 44 aus 4-Methyl-benzaldehyd und Dimethylamin hergestellt worden ist.

Beispiel 94:

[(5-Chlor-1H-indol-3-yl)-p-tolyl-methyl]-dimethyl-amin

Die Herstellung erfolgte gemäß der allgemeinen Synthesevorschrift 4 aus 5-Chlor-1H-indol und (4-Methyl-benzyliden)-dimethylammoniumchlorid, das gemäß Beispiel 44 aus 4-Methyl-benzaldehyd und Dimethylamin hergestellt worden ist.

Beispiel 95:

Dimethyl-[(2-methyl-1H-indol-3-yl)-(3-phenoxy-phenyl)-methyl]-amin

Die Herstellung erfolgte gemäß der allgemeinen Synthesevorschrift 4 aus 2-Methyl-1H-indol und (3-Phenoxy-benzyliden)-dimethylammoniumchlorid, das gemäß Beispiel 44 aus 3-Phenoxy-benzaldehyd und Dimethylamin hergestellt worden ist.

Beispiel 96:

[(1-Ethyl-2-phenyl-1H-indol-3-yl)-(3-phenoxy-phenyl)-methyl]-dimethyl-amin

Die Herstellung erfolgte gemäß der allgemeinen Synthesevorschrift 4 aus 1-Ethyl-2-Phenyl-1H-indol und (3-Phenoxy-benzyliden)-dimethylammoniumchlorid, das gemäß Beispiel 44 aus 3-Phenoxy-benzaldehyd und Dimethylamin hergestellt worden ist.

Beispiel 97:

[(7-Ethyl-1H-indol-3-yl)-(3-phenoxy-phenyl)-methyl]-dimethyl-amin

Die Herstellung erfolgte gemäß der allgemeinen Synthesevorschrift 4 aus 7-Ethyl-1H-indol und (3-Phenoxy-benzyliden)-dimethylammoniumchlorid, das gemäß Beispiel 44 aus 3-Phenoxy-benzaldehyd und Dimethylamin hergestellt worden ist.

Beispiel 98:

Dimethyl-[(1-methyl-1H-indol-3-yl)-(3-phenoxy-phenyl)-methyl]-amin

Die Herstellung erfolgte gemäß der allgemeinen Synthesevorschrift 4 aus 1-Methyl-1H-indol und (3-Phenoxy-benzyliden)-dimethylammoniumchlorid, das gemäß Beispiel 44 aus 3-Phenoxy-benzaldehyd und Dimethylamin hergestellt worden ist.

Beispiel 99:

[[2-(4-Fluor-phenyl)-1H-indol-3-yl]-(3-phenoxy-phenyl)-methyl]-dimethyl-amin

Die Herstellung erfolgte gemäß der allgemeinen Synthesevorschrift 4 aus 2-(4-Fluor-phenyl)-1H-indol und (3-Phenoxy-benzyliden)-dimethylammoniumchlorid, das gemäß Beispiel 44 aus 3-Phenoxy-benzaldehyd und Dimethylamin hergestellt worden ist.

Beispiel 100:

[[2-(3-Chlor-4-fluor-phenyl)-1H-indol-3-yl]-(3-phenoxy-phenyl)-methyl]-dimethylamin

Die Herstellung erfolgte gemäß der allgemeinen Synthesevorschrift 4 aus 2-(3-Chlor-4-Fluor-phenyl)-1H-indol und (3-Phenoxy-benzyliden)-dimethylammoniumchlorid, das gemäß Beispiel 44 aus 3-Phenoxy-benzaldehyd und Dimethylamin hergestellt worden ist.

Beispiel 101:

Dimethyl-[(4-methyl-1H-indol-3-yl)-(3-phenoxy-phenyl)-methyl]-amin

Die Herstellung erfolgte gemäß der allgemeinen Synthesevorschrift 4 aus 4-Methyl-1H-indol und (3-Phenoxy-benzyliden)-dimethylammoniumchlorid, das gemäß Beispiel 44 aus 3-Phenoxy-benzaldehyd und Dimethylamin hergestellt worden ist.

Beispiel 102:

[(5-Chlor-1H-indol-3-yl)-(2-trifluormethyl-phenyl)-methyl]-dimethyl-amin

Die Herstellung erfolgte gemäß der allgemeinen Synthesevorschrift 4 aus 5-Chlor-1H-indol und (2-Trifluormethyl-benzyliden)-dimethylammoniumchlorid, das gemäß Beispiel 44 aus 2-Trifluormethyl-benzaldehyd und Dimethylamin hergestellt worden ist.

Beispiel 103:

Dimethyl-[(2-methyl-1H-indol-3-yl)-(4-trifluormethyl-phenyl)-methyl]-amin

Die Herstellung erfolgte gemäß der allgemeinen Synthesevorschrift 4 aus 2-Methyl-1H-indol und (4-Trifluormethyl-benzyliden)-dimethylammoniumchlorid, das gemäß Beispiel 44 aus 4-Trifluormethyl-benzaldehyd und Dimethylamin hergestellt worden ist.

**Pharmakologische Untersuchungen:**

1.) In vitro-Tests

Die Austestung der erfindungsgemäßen Indol-Mannichbasen auf ihre Wirksamkeit erfolgte wie obenstehend angegeben.

Die untersuchten erfindungsgemäßen Verbindungen der allgemeinen Formel 1 eignen sich als Liganden für den α2-Subtypus des menschlichen α-adrenergischen Rezeptors, der für das Schmerzempfinden von Bedeutung ist.

Die Affinität der erfindungsgemäßen Verbindungen zu diesem α2-Subtypus des α-adrenergischen Rezeptors wurde mit Hilfe des SPA-Tests ermittelt, wie er in John P. Devlin, High Throughput Screening, Marcel Dekker Inc. 1997, Seiten 307 bis 316 beschrieben ist. Diese Literatur wird hiermit als Referenz eingeführt und gilt somit als Teil der Offenbarung. Die Affinitäten der erfindungsgemäßen Verbindungen wurden jeweils bei einer Konzentration von 10 μM bestimmt.

Die Ergebnisse ausgewählter Untersuchungen sind in der nachfolgenden Tabelle 1 angegeben:

Tabelle 1:

| Beispiel Nr. | % Hemmung α2 (Mensch), 10 μM |
|---|---|
| 36 | 67 |
| 50 | 51 |
| 34 | 95 |
| 30 | 40 |
| 7 | 68 |
| 15 | 40 |
| 51 | 43 |
| 52 | 37 |
| 53 | 38 |
| 54 | 55 |
| 55 | 49 |
| 56 | 39 |
| 57 | 64 |
| 58 | 82 |
| 59 | 37 |
| 60 | 66 |
| 61 | 63 |
| 62 | 51 |
| 63 | 60 |
| 64 | 48 |
| 65 | 42 |
| 66 | 44 |
| 67 | 36 |
| 68 | 88 |
| 69 | 75 |
| 70 | 63 |
| 71 | 50 |

Die untersuchten erfindungsgemäßen Verbindungen zeigten ferner auch eine Hemmung der Serotonin-Wiederaufnahme.

Die Ergebnisse ausgewählter Untersuchungen zur Hemmung der Serotonin-Wiederaufnahme sind in der nachfolgenden Tabelle 2 wiedergegeben:

Tabelle 2:

| Beispiel Nr. | Hemmung 5HT-Aufnahme in % |
|---|---|
| 44 | 48 |
| 72 | 60 |
| 73 | 41 |
| 74 | 72 |
| 75 | 39 |
| 76 | 42 |
| 77 | 38 |
| 78 | 73 |
| 79 | 50 |
| 80 | 42 |
| 81 | 68 |
| 82 | 42 |
| 83 | 39 |
| 84 | 44 |
| 85 | 44 |
| 86 | 41 |
| 87 | 50 |
| 88 | 62 |
| 89 | 45 |
| 90 | 44 |
| 91 | 49 |
| 92 | 46 |
| 93 | 78 |
| 94 | 64 |
| 95 | 70 |
| 96 | 55 |
| 97 | 79 |
| 98 | 66 |
| 99 | 42 |
| 100 | 48 |
| 101 | 41 |
| 102 | 42 |
| 103 | 74 |

2.) Analgesieprüfung im Writhing-Test an der Maus

Die vertiefte Untersuchung der erfindungsgemäßen Verbindungen auf ihre analgetische Wirksamkeit wurde im Phenylchinon-induzierten Writhing an der Maus durchgeführt, wie obenstehend beschrieben.

Die untersuchten erfindungsgemäßen Verbindungen zeigten eine analgetische Wirkung.

Die Ergebnisse ausgewählter Writhing-Untersuchungen sind in der nachfolgenden Tabelle 3 zusammengefaßt.

Tabelle 3: Analgesieprüfung im Writhing-Test an der Maus

| Beispiel Nr. | Hemmung der Writhingreaktion in % |
|---|---|
| 22 | 39 |
| 23 | 75 |
| 46 | 41 |
| 47 | 57 |
| 48 | 6 |
| 49 | 82 |

## Patentansprüche

**1.** Substituierte Indol-Mannichbasen der allgemeinen Formel I,

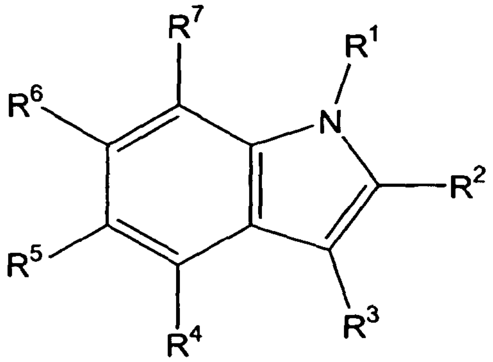

I

worin

$R^1$ = H, einen $C_{1-10}$-Alkyl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet,

$R^2$ = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet,

$R^3$ = $CH(R^{13})N(R^{14})(R^{15})$ bedeutet,

$R^4$, $R^5$, $R^6$, $R^7$, gleich oder verschieden, = H, F, Cl, Br, $CF_3$, CN, $NO_2$, $NHR^8$, $SR^9$, $OR^{10}$, $SO_2NH_2$, $SO_2NHR^{21}$, CO

(OR$^{11}$), $CH_2CO(OR^{12})$, COR$^{19}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeuten,

$R^8$ = H, COR$^{16}$, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,

$R^9$ = H, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,

$R^{10}$ = H, COR$^{17}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet,

$R^{11}$ = H, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,

$R^{12}$ = H, einen $C_{1-10}$-Alkyl-Rest oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,

$R^{13}$ = einen unsubstituierten Phenyl-Rest oder einen wenigstens einfach mit $C_{1-4}$-Alkyl-, Halogen-, $CF_3$-, CN- oder OH substituierten Phenyl-Rest bedeutet,

$R^{14}$, $R^{15}$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest,

oder $R^{14}$ und $R^{15}$ zusammen $(CH_2)_n$ mit n = eine ganze Zahl von 3 bis 6 oder $(CH_2)_2O(CH_2)_2$ bedeuten,

$R^{16}$ = einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen unsubstituierten oder mit einem Halogen-, CN-, $CF_3$- oder OH-Rest substituierten Thiophen-, Pyrrolyl- oder Furfuryl-Rest bedeutet,

$R^{17}$ = einen $C_{1-10}$-Alkyl-Rest bedeutet,

$R^{18}$ = einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen unsubstituierten oder mit einem Halogen-, CN-, $CF_3$- oder OH-Rest substituierten Thiophen-, Pyrrolyl- oder Furfuryl-Rest bedeutet,

$R^{19}$ = H, $NHNH_2$, NHR$^{20}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylengruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylengruppe gebundenen, unsubstituierten oder mit einem Halogen-, CN-, $CF_3$-oder OH-Rest substituierten Thiophen-, Pyrrolyl- oder Furfuryl-Rest bedeutet,

$R^{20}$ = H, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest bedeutet, einen über eine $C_{1-6}$-Alkylengruppe gebundenen, unsubstituierten oder wenigstens einfach mit einem OH-, einem Halogen-, einem $CF_3$-,

einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylengruppe gebundenen, unsubstituierten oder mit einem Halogen-, CN-, $CF_3$-oder OH-Rest substituierten Thiophen-, Pyrrolyl- oder Furfuryl-Rest bedeutet,
$R^{21}$ = H, $COR^{17}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen über eine $C_{1-6}$-Alkylengruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylengruppe gebundenen, unsubstituierten oder mit einem Halogen-, CN-, $CF_3$- oder OH-Rest substituierten Thiophen-, Pyrrolyl- oder Furfuryl-Rest bedeutet,
und/oder deren Racemate, Enantiomere, Diastereomere und/oder entsprechende Basen und/oder entsprechende Salze von physiologisch verträglichen Säuren,
wobei die Racemate der Verbindungen, in denen der Rest $R^3 = CH(R^{13})N(R^{14})(R^{15})$ bedeutet und jeweils
die Reste $R^4$ bis $R^7$und $R^2$ = H, der Rest $R^{13}$ = Phenyl, die Reste $R^{14}$ und $R^{15}$ zusammen = $(CH_2)_5$ und der Rest $R^1$ = H, $CH_3$ oder Benzyl bedeuten
oder
die Reste $R^4$ bis $R^7$ und $R^2$ = H, der Rest $R^{13}$ = Phenyl, die Reste $R^{14}$, $R^{15}$ jeweils = $CH_3$, und der Rest $R^1$ = H, $CH_3$ oder Benzyl bedeuten
oder
die Reste $R^4$ bis $R^7$ und $R^1$ = H, der Rest $R^{13}$ = 2-Chlor-phenyl, die Reste $R^{14}$ und $R^{15}$ jeweils = $CH_3$ oder die Reste $R^{14}$ und $R^{15}$ zusammen = $(CH_2)_2O(CH_2)_2$, der Rest $R^2$ = $COOR^{11}$ und der Rest $R^{11}$ = $C_2H_5$ bedeuten sowie die entsprechenden Hydrochloride
oder
die Reste $R^4$ bis $R^7$ und $R^1$ = H, der Rest $R^{13}$ = Phenyl, die Reste $R^{14}$ und $R^{15}$ zusammen = $(CH_2)_5$, der Rest $R^2$ = $COOR^{11}$ und der Rest $R^{11}$ = $CH_3$ bedeuten und das entsprechende Hydrochlorid
oder
die Reste $R^4$ bis $R^7$ und $R^2$ = H, der Rest $R^{13}$ = Phenyl, die Reste $R^{14}$ und $R^{15}$ zusammen = $(CH_2)_4$, der Rest $R^1$ = H, $CH_3$, $CH_2$-CH=$CH_2$ oder 4-Chlorbenzyl sowie für $R^1$ = $CH_3$ auch das entsprechende Hydrochlorid der Verbindung
oder
die Reste $R^4$ bis $R^7$, $R^1$ und $R^2$ = H, der Rest $R^{13}$ = Phenyl, der Rest $R^{14}$ = Phenyl und der Rest $R^{15}$ = 4-Ethoxyphenyl bedeuten
oder
die Reste $R^4$ bis $R^7$ und $R^1$ = H, der Rest $R^{13}$ = Phenyl, 2-Hydroxyphenyl oder 2-Hydroxy-5-methylphenyl, die Reste $R^{14}$ und $R^{15}$ zusammen = $(CH_2)_5$ und der Rest $R^2$ = $CH_3$ bedeuten, für $R^{13}$ = Phenyl auch das entsprechende Hydrogensulfat
oder
die Reste $R^4$ bis $R^7$ und $R^1$ = H, der Rest $R^{13}$ = Phenyl, die Reste $R^{14}$ und $R^{15}$ zusammen = $(CH_2)_2O(CH_2)_2$ und der Rest $R^2$ = $CH_3$ bedeuten und das entsprechende Hydrogensulfat der Verbindung
ausgenommen sind.

2. Substituierte Indol-Mannichbasen gemäß Anspruch 1, **dadurch gekennzeichnet, daß** der Rest $R^1$ für H steht und die Reste $R^2$ bis $R^{21}$ die Bedeutung gemäß Anspruch 1 haben.

3. Substituierte Indol-Mannichbasen gemäß Anspruch 1, **dadurch gekennzeichnet, daß** der Rest $R^1$ für einen $C_{1-6}$-Alkyl-Rest, vorzugsweise für einen $C_{1-2}$-Alkyl-Rest, steht und die Reste $R^2$ bis $R^{21}$ die Bedeutung gemäß Anspruch 1 haben.

4. Substituierte Indol-Mannichbasen gemäß Anspruch 1, **dadurch gekennzeichnet, daß** der Rest $R^1$ für einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest steht, der gegebenenfalls mit weiteren Ringen kondensiert ist, und die Reste $R^2$ bis $R^{21}$ die Bedeutung gemäß Anspruch 1 haben.

5. Substituierte Indol-Mannichbasen gemäß einem der Ansprüche 1 bis 4, **dadurch gekennzeichnet, daß** der Rest $R^2$ für H steht und die Reste $R^3$ bis $R^{21}$ die Bedeutung gemäß Anspruch 1 haben.

6. Substituierte Indol-Mannichbasen gemäß einem der Ansprüche 1 bis 4, **dadurch gekennzeichnet, daß** der Rest $R^2$ für einen $C_{1-6}$-Alkyl -Rest, vorzugsweise für einen $C_{1-2}$-Alkyl-Rest, steht und die Reste $R^3$ bis $R^{21}$ die Bedeutung gemäß Anspruch 1 haben.

7. Substituierte Indol-Mannichbasen gemäß einem der Ansprüche 1 bis 4, **dadurch gekennzeichnet, daß** der Rest $R^2$ für einen unsubstituierten Phenyl-Rest, steht und die Reste $R^3$ bis $R^{21}$ die Bedeutung gemäß Anspruch 1 haben.

8. Substituierte Indol-Mannichbasen gemäß einem der Ansprüche 1 bis 4 **dadurch gekennzeichnet, daß** der Rest $R^2$ für einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest steht, der gegebenenfalls mit weiteren Ringen kondensiert ist, und die übrigen Reste $R^3$ bis $R^{21}$ die Bedeutung gemäß Anspruch 1 haben.

9. Substituierte Indol-Mannichbasen gemäß einem der Ansprüche 1 bis 4 **dadurch gekennzeichnet, daß** der Rest $R^2$ für Cl oder F steht und die übrigen Reste $R^3$ bis $R^{21}$ die Bedeutung gemäß Anspruch 1 haben.

10. Substituierte Indol-Mannichbasen gemäß einem der Ansprüche 1 bis 4 **dadurch gekennzeichnet, daß** der Rest $R^2$ für $NO_2$ steht und die übrigen Reste $R^3$ bis $R^{21}$ die Bedeutung gemäß Anspruch 1 haben.

11. Substituierte Indol-Mannichbasen gemäß einem der Ansprüche 1 bis 4 **dadurch gekennzeichnet, daß** der Rest $R^2$ für $OR^{10}$ steht und die übrigen Reste $R^3$ bis $R^{21}$ die Bedeutung gemäß Anspruch 1 haben.

12. Substituierte Indol-Mannichbasen gemäß einem der Ansprüche 1 bis 4 **dadurch gekennzeichnet, daß** der Rest $R^2$ für $CO(OR^{11})$ steht und die übrigen Reste $R^3$ bis $R^{21}$ die Bedeutung gemäß Anspruch 1 haben.

13. Substituierte Indol-Mannichbasen gemäß einem der Ansprüche 1 bis 12 **dadurch gekennzeichnet, daß** wenigstens einer der Reste $R^4$, $R^5$, $R^6$ oder $R^7$ für H steht und die jeweils übrigen Reste $R^4$, $R^5$, $R^6$ oder $R^7$ und $R^8$ bis $R^{21}$ die Bedeutung gemäß Anspruch 1 haben.

14. Substituierte Indol-Mannichbasen gemäß einem der Ansprüche 1 bis 12 **dadurch gekennzeichnet, daß** wenigstens einer der Reste $R^4$, $R^5$, $R^6$ oder $R^7$ für Cl oder F steht und die jeweils übrigen Reste $R^4$, $R^5$, $R^6$ oder $R^7$ und $R^8$ bis $R^{21}$ die Bedeutung gemäß Anspruch 1 haben.

15. Substituierte Indol-Mannichbasen gemäß einem der Ansprüche 1 bis 12 **dadurch gekennzeichnet, daß** wenigstens einer der Reste $R^4$, $R^5$, $R^6$ oder $R^7$ für einen $C_{1-6}$-Alkyl-Rest, vorzugsweise für einen $C_{1-2}$-Alkyl-Rest, steht und die jeweils übrigen Reste $R^4$, $R^5$, $R^6$ oder $R^7$ und $R^8$ bis $R^{21}$ die Bedeutung gemäß Anspruch 1 haben.

16. Substituierte Indol-Mannichbasen gemäß einem der Ansprüche 1 bis 12, **dadurch gekennzeichnet, daß** wenigstens einer der Reste $R^4$, $R^5$, $R^6$ oder $R^7$ für $NO_2$ steht und die jeweils übrigen Reste $R^4$, $R^5$, $R^6$ oder $R^7$ und $R^8$ bis $R^{21}$ die Bedeutung gemäß Anspruch 1 haben.

17. Substituierte Indol-Mannichbasen gemäß einem der Ansprüche 1 bis 12, **dadurch gekennzeichnet, daß** wenigstens einer der Reste $R^4$, $R^5$, $R^6$ oder $R^7$ für einen über eine $C_{1-2}$-Alkylen-Gruppen gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest steht, der gegebenenfalls mit weiteren Ringen kondensiert ist, und die jeweils übrigen Reste $R^4$, $R^5$, $R^6$ oder $R^7$ und $R^8$ bis $R^{21}$ die Bedeutung gemäß Anspruch 1 haben.

18. Substituierte Indol-Mannichbasen gemäß einem der Ansprüche 1 bis 12, **dadurch gekennzeichnet, daß** wenigstens einer der Reste $R^4$, $R^5$, $R^6$ oder $R^7$ für $OR^{10}$ steht und die jeweils übrigen Reste $R^4$, $R^5$, $R^6$ oder $R^7$ und $R^8$ bis $R^{21}$ die Bedeutung gemäß Anspruch 1 haben.

19. Substituierte Indol-Mannichbasen gemäß einem der Ansprüche 1 bis 12, **dadurch gekennzeichnet, daß** wenigstens einer der Reste $R^4$, $R^5$, $R^6$ oder $R^7$ für $CO(OR^{11})$ steht und die jeweils übrigen Reste $R^4$, $R^5$, $R^6$ oder $R^7$ und $R^8$ bis $R^{21}$ die Bedeutung gemäß Anspruch 1 haben.

20. Substituierte Indol-Mannichbasen gemäß einem der Ansprüche 1 bis 12, **dadurch gekennzeichnet, daß** wenigstens einer der Reste $R^4$, $R^5$, $R^6$ oder $R^7$ für einen unsubstituierten oder wenigstens einfach mit einem OH-, einem

Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise für einen unsubstituierten Phenyl-Rest steht und die jeweils übrigen Reste $R^4$, $R^5$, $R^6$ oder $R^7$ und $R^8$ bis $R^{21}$ die Bedeutung gemäß Anspruch 1 haben.

**21.** Substituierte Indol-Mannichbasen gemäß einem der Ansprüche 1 bis 20, **dadurch gekennzeichnet, daß** der Rest $R^8$ für einen $C_{1-6}$Alkyl-Rest steht und die Reste $R^9$ bis $R^{21}$ die Bedeutung gemäß Anspruch 1 haben.

**22.** Substituierte Indol-Mannichbasen gemäß einem der Ansprüche 1 bis 21, **dadurch gekennzeichnet, daß** der Rest $R^9$ für einen $C_{1-6}$-Alkyl-Rest steht und die Reste $R^{10}$ bis $R^{21}$ die Bedeutung gemäß Anspruch 1 haben.

**23.** Substituierte Indol-Mannichbasen gemäß einem der Ansprüche 1 bis 21, **dadurch gekennzeichnet, daß** der Rest $R^9$ für einen Phenyl-Rest steht und die Reste $R^{10}$ bis $R^{21}$ die Bedeutung gemäß Anspruch 1 haben.

**24.** Substituierte Indol-Mannichbasen gemäß einem der Ansprüche 1 bis 23, **dadurch gekennzeichnet, daß** der Rest $R^{10}$ für H steht und die Reste $R^{11}$ bis $R^{21}$ die Bedeutung gemäß Anspruch 1 haben.

**25.** Substituierte Indol-Mannichbasen gemäß einem der Ansprüche 1 bis 23, **dadurch gekennzeichnet, daß** der Rest $R^{10}$ für einen $C_{1-6}$-Alkyl-Rest steht und die Reste $R^{11}$ bis $R^{21}$ die Bedeutung gemäß Anspruch 1 haben.

**26.** Substituierte Indol-Mannichbasen gemäß einem der Ansprüche 1 bis 23, **dadurch gekennzeichnet, daß** der Rest $R^{10}$ für einen über eine $C_{1-2}$-AlkylenGruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy-oder einem Phenyl-Rest substituierten Phenyl- oder Naphthyl-Rest steht, der gegebenenfalls mit weiteren Ringen kondensiert ist, und die Reste $R^{11}$ bis $R^{21}$ die Bedeutung gemäß Anspruch 1 haben.

**27.** Substituierte Indol-Mannichbasen gemäß einem der Ansprüche 1 bis 26, **dadurch gekennzeichnet, daß** der Rest $R^{11}$ für H steht und die Reste $R^{12}$ bis $R^{21}$ die Bedeutung gemäß Anspruch 1 haben.

**28.** Substituierte Indol-Mannichbasen gemäß einem der Ansprüche 1 bis 26, **dadurch gekennzeichnet, daß** der Rest $R^{11}$ für einen $C_{1-6}$Alkyl-Rest steht und die Reste $R^{12}$ bis $R^{21}$ die Bedeutung gemäß Anspruch 1 haben.

**29.** Substituierte Indol-Mannichbasen gemäß einem der Ansprüche 1 bis 28, **dadurch gekennzeichnet, daß** der Rest $R^{12}$ für einen $C_{1-6}$Alkyl-Rest steht und die Reste $R^{13}$ bis $R^{21}$ die Bedeutung gemäß Anspruch 1 haben.

**30.** Substituierte Indol-Mannichbasen gemäß einem der Ansprüche 1 bis 29, **dadurch gekennzeichnet, daß** der Rest $R^{13}$ einen unsubstituierten Phenyl-Rest oder einen wenigstens einfach mit Methyl-, tert-Butyl-, F-, Cl-, Br-, oder $CF_3$-substituierten Phenyl-Rest, bevorzugt einen unsubstituierten Phenyl-Rest oder einen 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluor-phenyl-, 2-Chlor-4-Fluor phenyl-, 2-Chlor-5-Fluor-phenyl-, 2-Chlor-6-Fluor-phenyl-, 4-Brom-2-Fluor-phenyl-, 3-Brom-4-Fluor-phenyl-, 3-Brom-2-Fluor-phenyl-, 2,3-Dichlor-phenyl-, 2,4-Dichlor-phenyl-, 2,5-Dichlorphenyl-, 3,4-Dichlor-phenyl-, 2,3-Dimethyl-phenyl-, 2,4-Dimethyl-phenyl-, 2,5-Dimethylphenyl-, 2-Trifluormethyl-phenyl, 3-Trifluormethyl-phenyl oder einen 4-Trifluormethyl-phenyl-Rest, besonders bevorzugt einen unsubstituierten Phenyl-Rest, bedeutet und die Reste $R^{14}$ bis $R^{21}$ die Bedeutung gemäß Anspruch 1 haben.

**31.** Substituierte Indol-Mannichbasen gemäß einem der Ansprüche 1 bis 30, **dadurch gekennzeichnet, daß** wenigstens einer der Reste $R^{14}$ oder $R^{15}$ für einen verzweigten, unverzweigten, gesättigten, ungesättigten, unsubstituierten oder wenigstens einfach substituierten $C_{1-6}$-Alkyl-Rest, bevorzugt für einen $CH_3$-Rest steht, der jeweils andere Rest $R^{14}$ oder $R^{15}$ und die Reste $R^{16}$ bis $R^{21}$ die Bedeutung gemäß Anspruch 1 haben.

**32.** Substituierte Indol-Mannichbasen gemäß einem der Ansprüche 1 bis 30, **dadurch gekennzeichnet, daß** die Reste $R^{14}$ und $R^{15}$ zusammen $(CH_2)_n$ mit n = 4 oder 5 bedeuten und die Reste $R^{16}$ bis $R^{21}$ die Bedeutung gemäß Anspruch 1 haben.

**33.** Substituierte Indol-Mannichbasen gemäß einem der Ansprüche 1 bis 32, **dadurch gekennzeichnet, daß** der Rest $R^{16}$ für einen $C_{1-6}$-Alkyl-Rest steht und die Reste $R^{17}$ bis $R^{21}$ die Bedeutung gemäß Anspruch 1 haben.

**34.** Substituierte Indol-Mannichbasen gemäß einem der Ansprüche 1 bis 33, **dadurch gekennzeichnet, daß** der Rest $R^{17}$ für einen $C_{1-6}$-Alkyl-Rest steht und die Reste $R^{18}$ bis $R^{21}$ die Bedeutung gemäß Anspruch 1 haben.

**35.** Substituierte Indol-Mannichbasen gemäß einem der Ansprüche 1 bis 34, **dadurch gekennzeichnet, daß** der Rest $R^{18}$ für einen $C_{1-6}$-Alkyl-Rest steht und die Reste $R^{19}$ bis $R^{21}$ die Bedeutung gemäß Anspruch 1 haben.

**36.** Substituierte Indol-Mannichbasen gemäß einem der Ansprüche 1 bis 35, **dadurch gekennzeichnet, daß** der Rest $R^{19}$ für einen $C_{1-6}$-Alkyl-Rest steht und die Reste $R^{20}$ und $R^{21}$ die Bedeutung gemäß Anspruch 1 haben.

**37.** Substituierte Indol-Mannichbasen gemäß einem der Ansprüche 1 bis 35, **dadurch gekennzeichnet, daß** der Rest $R^{19}$ für einen über eine $C_{1-2}$-AlkylenGruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy-oder einem Phenyl-Rest substituierten Phenyl- oder Naphthyl-Rest steht, der gegebenenfalls mit weiteren Ringen kondensiert ist, und die Reste $R^{20}$ und $R^{21}$ die Bedeutung gemäß Anspruch 1 haben.

**38.** Substituierte Indol-Mannichbasen gemäß einem der Ansprüche 1 bis 37, **dadurch gekennzeichnet, daß** der Rest $R^{20}$ für einen $C_{1-6}$-Alkyl-Rest steht und der Rest $R^{21}$ die Bedeutung gemäß Anspruch 1 hat.

**39.** Substituierte Indol-Mannichbasen gemäß einem der Ansprüche 1 bis 37, **dadurch gekennzeichnet, daß** der Rest $R^{20}$ für einen über eine $C_{1-2}$-AlkylenGruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy-oder einem Phenyl-Rest substituierten Phenyl- oder Naphthyl-Rest steht, der gegebenenfalls mit weiteren Ringen kondensiert ist, und der Rest $R^{21}$ die Bedeutung gemäß Anspruch 1 hat.

**40.** Substituierte Indol-Mannichbasen gemäß einem der Ansprüche 1 bis 39, **dadurch gekennzeichnet, daß** der Rest $R^{21}$ für H steht.

**41.** Substituierte Indol-Mannichbasen gemäß einem der Ansprüche 1 bis 39, **dadurch gekennzeichnet, daß** der Rest $R^{21}$ für einen $C_{1-6}$-Alkyl-Rest steht.

**42.** Substituierte Indol-Mannichbasen gemäß einem der Ansprüche 1 bis 39, **dadurch gekennzeichnet, daß** der Rest $R^{21}$ für einen über eine $C_{1-2}$-AlkylenGruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy-oder einem Phenyl-Rest substituierten Phenyl- oder Naphthyl-Rest steht, der gegebenenfalls mit weiteren Ringen kondensiert ist.

**43.** Substituierte Indol-Mannichbasen gemäß Anspruch 1:

3-(Dimethylaminophenylmethyl)-1H-indol-2-carbonsäureethylester
3-(Dimethylaminophenylmethyl)-1H-indol-2-carbonsäure
[(5-Fluoro-1H-indol-3-yl)-phenylmethyl]-dimethylamin
[(1-Ethyl-2-phenyl-1H-indol-3-yl)-phenylmethyl]-dimethylamin
[(5-Methoxy-1H-indol-3-yl)-phenylmethyl]-dimethylamin
3-(Dimethylaminophenylmethyl)-5-hydroxy-1H-indol-2-carbonsäure
Dimethyl-[(2-methyl-1H-indol-3-yl)-phenylmethyl]-amin
3-(Dimethylaminophenylmethyl)-1H-indol-4-ol
Dimethyl-[(4-methyl-1H-indol-3-yl)-phenylmethyl]-amin
[(5-Chloro-1H-indol-3-yl)-phenylmethyl]-dimethylamin
[(5-Benzyloxy-1H-indol-3-yl)-phenylmethyl]-dimethylamin
Essigsäure-3-(dimethylaminophenylmethyl)-1H-indol-4-yl-ester
{[2-(4-Chlorophenyl)-1H-indol-3-yl]-phenylmethyl}-dimethylamin
5-Benzyloxy-3-(dimethylaminophenylmethyl)-1H-indol-2-carbonsäure
Dimethyl-[(2-methyl-5-nitro-1H-indol-3-yl)phenylmethyl]-amin
Dimethyl-[(2-methyl-6-nitro-1H-indol-3-yl)-phenylmethyl]-amin
[(6-Fluoro-2-methyl-1H-indol-3-yl)-phenylmethyl]-dimethylamin
{[2-(4-Fluorophenyl)-1H-indol-3-yl]-phenylmethyl}-dimethylamin
{[2-(3-Chloro-4-fluorophenyl)-1H-indol-3-yl]-phenylmethyl}-dimethylamin
[(7-Ethyl-1H-indol-3-yl)-phenylmethyl]-dimethylamin
3-(Dimethylaminophenylmethyl)-1H-indol-6-carbonsäure

Dimethyl-[(1-methyl-1H-indol-3-yl)-phenylmethyl]-amin
Dimethyl-[(2-phenyl-1H-indol-3-yl)-o-tolyl-methyl]-amin
[(5-Benzyloxy-1H-indol-3-yl)-o-tolylmethyl]-dimethylamin
{[1-(4-Methoxybenzyl)-1H-indol-3-yl]-phenyl-methyl}-dimethylamin
[(1H-Indol-3-yl)-phenylmethyl]-dimethylamin
[(5-Brom-2-fluor-phenyl)-(4-nitro-1H-indol-3-yl)-methyl]-dimethyl-amin
[(5-Brom-2-fluor-phenyl)-(1-methyl-1H-indol-3-yl)-methyl]-dimethyl-amin
[(2-Chlor-6-fluor-phenyl)-(2-phenyl-1H-indol-3-yl)-methyl]-dimethylamin
[(2-Chlor-6-fluor-phenyl)-(1-ethyl-2-phenyl-1H-indol-3-yl)-methyl]-dimethylamin
[(2-Chlor-6-fluor-phenyl)-(4-nitro-1H-indol-3-yl)-methyl]-dimethyl-amin
[(2-Chlor-6-fluor-phenyl)-(1-methyl-1H-indol-3-yl)-methyl]-dimethyl-amin
[(2-Brom-phenyl)-(4-nitro-1H-indol-3-yl)-methyl]-dimethyl-amin
[(2-Brom-phenyl)-(1-methyl-1H-indol-3-yl)-methyl]-dimethyl-amin
[(3-Brom-phenyl)-(7-ethyl-1H-indol-3-yl)-methyl]-dimethyl-amin
[(3-Brom-phenyl)-(1-methyl-1H-indol-3-yl)-methyl]-dimethyl-amin
[(4-tert-Butyl-phenyl)-(2-methyl-1H-indol-3-y-)-methyl]-dimethyl-amin
[(4-tert-Butyl-phenyl)-(1-ethyl-2-phenyl-1H-indol-3-yl)-methyl]-dimethyl-amin
[(4-tert-Butyl-phenyl)-(1-methyl-1H-indol-3-yl)-methyl]-dimethyl-amin
[(2-Chlor-4-fluor-phenyl)-(4-nitro-1H-indol-3-yl)-methyl]-dimethyl-amin
[(2-Chlor-4-fluor-phenyl)-(4-methyl-1H-indol-3-yl)-methyl]-dimethyl-amin
[(2-Chlor-6-fluor-phenyl)-(2-phenyl-1H-indol-3-yl)-methyl]-dimethyl-amin
{(2-Chlor-6-fluor-phenyl)-[2-(4-chlor-phenyl)-1H-indol-3-yl]-methyl}-dimethyl-amin
[(2-Chlor-6-fluor-phenyl)-(1-methyl-1H-indol-3-yl)-methyl]-dimethyl-amin
[(3-Chlor-phenyl)-(1-methyl-1H-indol-3-yl)-methyl]-dimethyl-amin
[(2,3-Dichlor-phenyl)-(1-methyl-1H-indol-3-yl)-methyl]-dimethyl-amin
[(2,4-Dichlor-phenyl)-(1-methyl-1H-indol-3-yl)-methyl]-dimethyl-amin
[(4-tert-Butyl-phenyl)-(1-ethyl-2-phenyl-1H-indol-3-yl)-methyl]-dimethyl-amin
[(2-Chlor-4-fluor phenyl)-(2-methyl-1H-indol-3-yl)-methyl]-dimethyl-amin
[(2-Chlor-6-fluor-phenyl)-(2-methyl-1H-indol-3-yl)-methyl]-dimethyl-amin
[(5-Chlor-1H-indol-3-yl)-(2-fluor-phenyl)-methyl]-dimethyl-amin
[(4-Fluor-phenyl)-(4-methyl-1H-indol-3-yl)-methyl]-dimethyl-amin
Dimethyl-[(2-methyl-1H-indol-3-yl)-o-tolyl-methyl]-amin
[(7-Ethyl-1H-indol-3-yl)-o-tolyl-methyl]-dimethyl-amin
Dimethyl-[(1-methyl-1H-indol-3-yl)-o-tolyl-methyl]-amin
[(5-Chlor-1H-indol-3-yl)-o-tolyl-methyl]-dimethyl-amin
[(5-Chlor-1H-indol-3-yl)-m-tolyl-methyl]-dimethyl-amin
Dimethyl-[(2-methyl-1H-indol-3-yl)-p-tolyl-methyl]-amin
[(5-Chlor-1H-indol-3-yl)-p-tolyl-methyl]-dimethyl-amin
[(5-Chlor-1H-indol-3-yl)-(2-trifluormethyl-phenyl)-methyl]-dimethyl-amin
Dimethyl-[(2-methyl-1H-indol-3-yl)-(4-trifluormethyl-phenyl)-methyl]-amin.

**44.** Substituierte Indol-Mannichbasen gemäß Anspruch 1:

1-Methyl-3-(morpholin-4-yl-phenylmethyl)-1H-indol
1-Ethyl-2-phenyl-3-(pyrrolidin-1-yl-*o*-tolylmethyl)-1H-indol
3-[(2-Chlorophenyl)-piperidin-1-yl-methyl]-1-ethyl-2-phenyl-1H-indol
1-Ethyl-2-phenyl-3-(phenylpyrrolidin-1-yl-methyl)-1H-indol
2-Phenyl-3-(pyrrolidin-1-yl-*o*-tolylmethyl)-1H-indol
2-Phenyl-3-(piperidin-1-yl-*o*-tolylmethyl)-1H-indol
3-[(2-Chlorophenyl)-piperidin-1-yl-methyl]-2-phenyl-1H-indol
2-Phenyl-3-(phenylpyrrolidin-1-yl-methyl)-1H-indol
4-Methyl-3-(piperidin-1-yl-o-tolyl-methyl)-1H-indol
5-Benzyloxy-3-(phenyl-piperidin-1-yl-methyl)-1H-indol
3-(Phenylpiperidin-1-y)-methyl)-1H-indol
1-Methyl-3-(phenylpiperidin-1-yl-methyl)-1H-indol
3-(Phenylpyrrolidin-1-yl-methyl)-1H-indol
5-Benzyloxy-3-(phenyl-pyrrolidin-1-yl-methyl)-1H-indol

**45.** Verfahren zur Herstellung von substituierten Indol-Mannichbasen der allgemeinen Formel I nach einem der Ansprüche 1 bis 44, **dadurch gekennzeichnet, daß** man aromatische Aldehydverbindungen der allgemeinen Formel II,

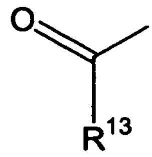

II

in denen $R^{13}$ die Bedeutung gemäß der allgemeinen Formel 1 nach Anspruch 1 hat, in Lösung in Gegenwart einer Base bei einer Temperatur von vorzugsweise - 10 bis + 110°C mit sekundären Aminen der allgemeinen Formel III

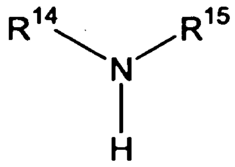

III,

in denen die Reste $R^{14}$ und $R^{15}$ die Bedeutung gemäß der allgemeinen Formel I nach Anspruch 1 haben zu Aminalverbindungen der allgemeinen Formel IV,

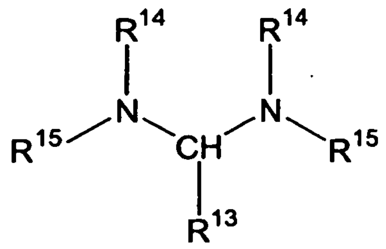

IV

umsetzt, und diese Aminalverbindungen der allgemeinen Formel IV ohne weitere Aufreinigung mit einem Säurechlorid in absolutem Lösungsmittel zu Iminiumsalzen der allgemeinen Formel V

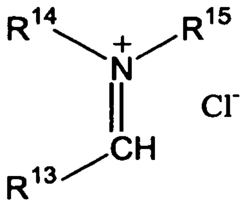

V

umsetzt, und diese Iminiumsalze der allgemeinen Formel V ohne weitere Aufreinigung in Lösung, vorzugsweise in Acetonitril und/oder Toluol mit Indol und/oder substituierten Indolverbindungen der allgemeinen Formel VI,

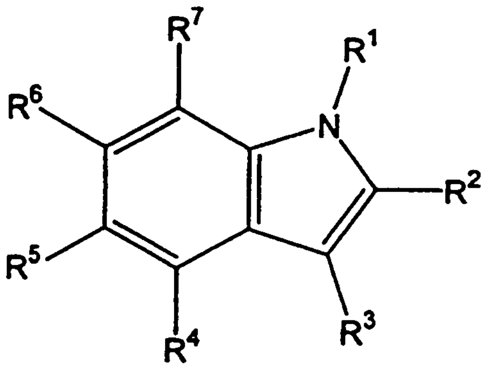

VI,

in denen der Rest $R^3$ = H bedeutet und die Reste $R^1$, $R^2$, $R^4$ bis $R^{12}$ und $R^{16}$ bis $R^{21}$ die Bedeutung gemäß der allgemeinen Formel I haben, umsetzt, und die so erhaltenen substituierten Indol-Mannichbasen der allgemeinen Formel I gemäß einem der Ansprüche 1 bis 44 durch Extraktion und/oder Waschen, vorzugsweise durch Waschen mit Aceton reinigt und nach den üblichen Methoden isoliert.

**46.** Verfahren zur Herstellung von substituierten Indol-Mannichbasen der allgemeinen Formel I gemäß einem der Ansprüche 1 bis 44, in denen der Rest $R^1 \neq$ H ist und die Reste $R^2$ bis $R^{21}$ die Bedeutung gemäß der allgemeinen Formel I haben, **dadurch gekennzeichnet, daß** man aromatische Aldehydverbindungen der allgemeinen Formel II,

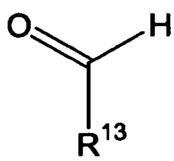

II

worin $R^{13}$ die Bedeutung gemäß der allgemeinen Formel I, in Lösung in Gegenwart einer Base bei einer Temperatur von vorzugsweise -10 bis 110°C mit sekundären Aminen der allgemeinen Formel III,

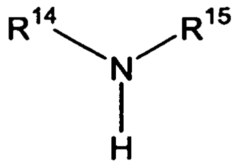

III

in denen die Reste $R^{14}$ und $R^{15}$ die Bedeutung gemäß der allgemeinen Formel I haben, zu Aminalverbindungen der allgemeinen Formel IV

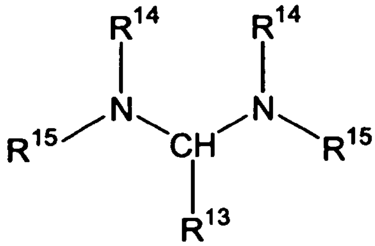

IV,

umsetzt, und diese Aminalverbindungen der allgemeinen Formel IV ohne weitere Aufreinigung mit einem Säurechlorid in absolutem Lösungsmittel zu Iminiumsalzen der allgemeinen Formel V

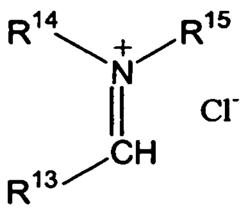

V

umsetzt, und diese Iminiumsalze der allgemeinen Formel V ohne weitere Aufreinigung in Lösung, vorzugsweise in Acetonitril und/oder Toluol mit Indol und/oder substituierten Indolverbindungen der allgemeinen Formel VI,

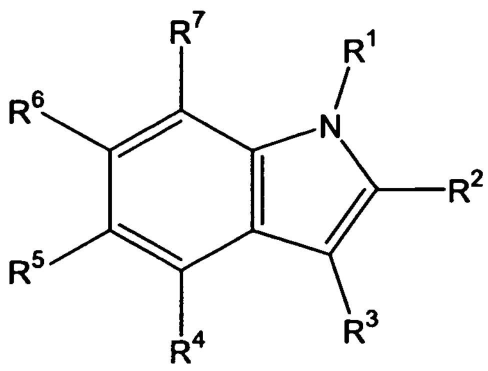

VI

in denen die Reste $R^1$ und $R^3$ jeweils = H bedeuten und die Reste $R^2$, $R^4$ bis $R^{12}$ und $R^{16}$ bis $R^{21}$ die Bedeutung gemäß der allgemeinen Formel I haben, umsetzt, und die so erhaltenen Verbindungen der allgemeinen Formel VI, in denen $R^1$ = H ist und die Reste $R^2$ bis $R^{21}$ die Bedeutung gemäß der allgemeinen Formel I haben, in Lösung mit Verbindungen der allgemeinen Formel $XR^{22}$, in denen $R^{22}$ einen $C_{1-10}$-Alkyl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest steht, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet und X = Cl, Br oder I bedeutet, bei einer Temperatur von vorzugsweise 10 bis 150°C in Gegenwart einer Base umsetzt, und die so erhaltenen Verbindungen der allgemeinen Formel I, in denen $R^1$ für einen $C_{1-10}$-Alkyl-, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest steht, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet und die übrigen Reste $R^2$ bis $R^{21}$ die Bedeutung gemäß der allgemeinen Formel I haben, durch Filtration reinigt und nach den üblichen Methoden isoliert.

**47.** Verfahren gemäß Anspruch 46, **dadurch gekennzeichnet, daß** die Umsetzung mit den Verbindungen der allgemeinen Formel $XR^{22}$ in Dimethylsulfoxid erfolgt.

**48.** Verfahren gemäß Anspruch 46 oder 47, **dadurch gekennzeichnet, daß** X = Cl bedeutet.

**49.** Verfahren gemäß einem der Ansprüche 46 bis 48, **dadurch gekennzeichnet, daß** der Rest $R^{22}$ für einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest steht, der gegebenenfalls mit weiteren Ringen kondensiert ist, steht.

**50.** Verfahren gemäß einem der Ansprüche 46 bis 49, **dadurch gekennzeichnet, daß** die Umsetzung mit den Verbindungen der allgemeinen Formel $XR^{22}$ in Gegenwart von Kaliumhydroxid als Base durchgeführt wird.

**51.** Verfahren gemäß einem der Ansprüche 46 bis 50, **dadurch gekennzeichnet, daß** die Verbindungen der allgemeinen Formel I durch Filtration über ein Scavenger-Harz, vorzugsweise durch Filtration über polymergebundenes Tris (2-aminoethyl)-amin und/oder 3-(3-Mercaptophenyl)propanamidomethylpolystyrol gereinigt werden.

**52.** Verfahren gemäß einem der Ansprüche 45 bis 51, **dadurch gekennzeichnet, daß** die aromatischen Aldehydverbindungen der allgemeinen Formel II in organischen Lösungsmitteln, vorzugsweise Toluol mit sekundären Aminen der allgmeinen Formel III umgesetzt werden.

**53.** Verfahren gemäß einem der Ansprüche 45 bis 52, **dadurch gekennzeichnet, daß** die aromatischen Aldehydverbindungen der allgemeinen Formel II in Gegenwart von Kaliumcarbonat und/oder Borsäureanhydrid als Base umgesetzt werden.

**54.** Verfahren gemäß einem der Ansprüche 45 bis 53, **dadurch gekennzeichnet, daß** die Aminalverbindungen der allgemeinen Formel IV mit Acetylchlorid zu Iminiumsalzen der allgemeinen Formel V umgesetzt werden.

**55.** Verfahren gemäß einem der Ansprüche 45 bis 54, **dadurch gekennzeichnet, daß** die Aminalverbindungen der allgemeinen Formel IV in absolutem Diethylether zu Iminiumsalzen der allgemeinen Formel V umgesetzt werden.

**56.** Arzneimittel enthaltend als Wirkstoff wenigstens eine substituierte Indol-Mannichbase der allgemeinen Formel I,

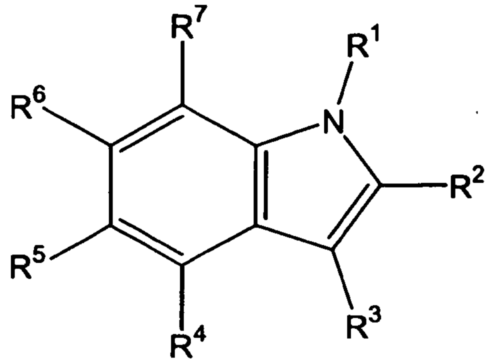

I

worin
$R^1$ = H, einen $C_{1-10}$-Alkyl-, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest, vorzugsweise H, einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, besonders bevorzugt H oder einen $C_{1-2}$-Alkyl-Rest bedeutet,
$R^2$ = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest, vorzugsweise = H, Cl, F, $NO_2$, $OR^{10}$, $CO(OR^{11})$, einen $C_{1-6}$-Alkyl- oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, besonders bevorzugt H, einen $C_{1-2}$-Alkyl- oder einen unsubstituierten Phenyl-Rest bedeutet,
$R^3$ = $CH(R^{13})N(R^{14})(R^{15})$ bedeutet,
$R^4$, $R^5$, $R^6$, $R^7$, gleich oder verschieden, = H, F, Cl, Br, $CF_3$, CN, $NO_2$, $NHR^8$, $SR^9$, $OR^{10}$, $SO_2NH_2$, $SO_2NHR^{21}$, CO $(OR^{11})$, $CH_2CO(OR^{12})$, $COR^{19}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$Alkoxy- oder einem Phenyl-Rest substituierten Phenyl- oder Naphthyl-Rest, der gegebenenfalls mit weiteren Ringen kondensiert ist, einen unsubstituierten

oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyloder Furfuryl-Rest bedeutet, vorzugsweise = H, Cl, F, $NO_2$, $OR^{10}$, $CO(OR^{11})$, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen $C_{1-6}$-Alkyl- oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, besonders bevorzugt H, $NO_2$, einen $C_{1-2}$-Alkyl- oder einen unsubstituierten Phenyl-Rest bedeuten,

$R^8$ = H, $COR^{16}$, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl-Rest bedeutet,

$R^9$ = H, einen $C_{1-10}$-Alkyl- oder einen unsubstituierten oder wenigstens einfach mit einem OH-, einem Halogen-, einem $CF_3$-, einem CN-, einem $C_{1-6}$Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl- oder einen Phenyl-Rest bedeutet,

$R^{10}$ = H, $COR^{17}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet, vorzugsweise = H, einen $C_{1-6}$-Alkyl-Rest oder einen über eine $C_{1-2}$-Alkylen-Gruppe gebundenen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,

$R^{11}$ = H, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise = H oder einen $C_{1-6}$-Alkyl-Rest bedeutet,

$R^{12}$ = H, einen $C_{1-10}$-Alkyl-Rest oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl-Rest bedeutet,

$R^{13}$ = einen unsubstituierten oder wenigstens einfach mit einem OH-, einem Halogen-, einem $CF_3$-, einem CN-, einem $C_{1-6}$-Alkyl-, einem $C_{1-6}$-Alkoxy-, einem - O-Phenyl- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest bedeutet oder einen Alkyl-Rest ohne acides Proton in $\alpha$-Stellung, vorzugsweise einen unsubstituierten Phenyl-Rest oder einen wenigstens einfach mit $C_{1-4}$-Alkyl-, $C_{1-3}$-Alkoxy-, Halogen-, $CF_3$-, CN-, O-Phenyl-oder OH substituierten Phenyl-, Thiophen- oder Furfuryl-Rest, besonders bevorzugt einen unsubstituierten Phenyl-, Thiophen- oder Furfuryl-Rest oder einen 2-Methoxy-phenyl-, 3-Methoxy-phenyl-, 4-Methoxy-phenyl-, 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluor phenyl-, 2-Chlor-4-Fluor-phenyl-, 2-Chlor-5-Fluor-phenyl-, 2-Chlor-6-Fluor-phenyl-, 4-Brom-2-Fluor-phenyl-, 3-Brom-4-Fluor-phenyl-, 3-Brom-2-Fluor-phenyl-, 2,3-Dichlor-phenyl-, 2,4-Dichlor-phenyl-, 2,5-Dichlorphenyl-, 3,4-Dichlor-phenyl-, 2,3-Dimethyl-phenyl-, 2,4-Dimethyl-phenyl-, 2,5-Dimethylphenyl-, 2,3-Dimethoxy-phenyl-, 2,4-Dimethoxy-phenyl-, 2,5-Dimethoxy-phenyl-, 3,4-Dimethoxy-phenyl-, 3,4,5-Trimethoxy-phenyl-, 2-Trifluormethyl-phenyl, 3-Trifluormethyl-phenyl oder einen 4-Trifluormethyl-phenyl-Rest, ganz besonders bevorzugt einen unsubstituierten Phenyl-, Thiophen- oder Furfuryl-Rest bedeutet,

$R^{14}$, $R^{15}$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest, vorzugsweise einen gesättigten, unsubstituierten oder wenigstens

einfach substituierten $C_{1-6}$-Alkyl-Rest, besonders bevorzugt einen $CH_3$-Rest, oder $R^{14}$ und $R^{15}$ zusammen $(CH_2)_n$ mit n = eine ganze Zahl von 3 bis 6 oder $(CH_2)_2O(CH_2)_2$, vorzugsweise $(CH_2)_n$ mit n = 4 oder 5, bedeuten,

$R^{16}$ = einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$Alkoxy- oder einem Phenyl-Rest substituierten Phenyl- oder Naphthyl-Rest, der gegebenenfalls mit weiteren Ringen kondensiert ist oder einen unsubstituierten oder mit einem Halogen-, CN-, $CF_3$- oder OH-Rest substituierten Thiophen-, Pyrrolyl- oder Furfuryl-Rest, vorzugsweise einen $C_{1-6}$-Alkyl-Rest bedeutet,

$R^{17}$ = einen $C_{1-10}$-Alkyl-Rest, vorzugsweise einen $C_{1-6}$-Alkyl-Rest bedeutet,

$R^{18}$ = einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist oder einen unsubstituierten oder mit einem Halogen-, CN-, $CF_3$- oder OH-Rest substituierten Thiophen-, Pyrrolyl- oder Furfuryl-Rest bedeutet, vorzugsweise einen $C_{1-6}$-Alkyl-Rest bedeutet,

$R^{19}$ = H, $NHNH_2$, $NHR^{20}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet, vorzugsweise einen $C_{1-6}$-Alkyl-Rest oder einen über eine $C_{1-2}$-AlkylenGruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy-oder einem Phenyl-Rest substituierten Phenyl- oder Naphthyl-Rest, der gegebenenfalls mit weiteren Ringen kondensiert ist, bedeutet,

$R^{20}$ = H, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet" vorzugsweise einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,

$R^{21}$ = H, $COR^{17}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet, vorzugsweise =H, einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet

und/oder deren Racemate, Enantiomere, Diastereomere und/oder entsprechende Basen und/oder entsprechende Salze von physiologisch verträglichen Säuren und gegebenfalls weitere Wirkstoffe und/oder Hilfsstoffe.

**57.** Arzneimittel gemäß Anspruch 56 enthaltend wenigstens eine substituierte Indol-Mannichbase ausgewählt aus der Gruppe bestehend aus

3-(Dimethylaminophenylmethyl)-1H-indol-2-carbonsäureethylester
3-(Dimethylaminophenylmethyl)-1H-indol-2-carbonsäure
[(5-Fluoro-1H-indol-3-yl)-phenylmethyl]-dimethylamin
[(1-Ethyl-2-phenyl-1H-indol-3-yl)-phenylmethyl]-dimethylamin
[(5-Methoxy-1H-indol-3-yl)-phenylmethyl]-dimethylamin
3-(Dimethylaminophenylmethyl)-5-hydroxy-1H-indol-2-carbonsäure
Dimethyl-[(2-methyl-1H-indol-3-yl)-phenylmethyl]-amin
3-(Dimethylaminophenylmethyl)-1H-indol-4-ol
Dimethyl-[(4-methyl-1H-indol-3-yl)-phenylmethyl]-amin
[(5-Chloro-1H-indol-3-yl)-phenylmethyl]-dimethylamin
[(5-Benzyloxy-1H-indol-3-yl)-phenylmethyl]-dimethylamin
Essigsäure-3-(dimethylaminophenylmethyl)-1H-indol-4-yl-ester
{[2-(4-Chlorophenyl)-1H-indol-3-yl]-phenylmethyl}-dimethylamin
5-Benzyloxy-3-(dimethylaminophenylmethyl)-1H-indol-2-carbonsäure
Dimethyl-[(2-methyl-5-nitro-1H-indol-3-yl)-phenylmethyl]-amin
Dimethyl-[(2-methyl-6-nitro-1H-indol-3-yl)-phenylmethyl]-amin
[(6-Fluoro-2-methyl-1H-indol-3-yl)-phenylmethyl]-dimethylamin
{[2-(4-Fluorophenyl)-1H-indol-3-yl]-phenylmethyl}-dimethylamin
{[2-(3-Chloro-4-fluorophenyl)-1H-indol-3-yl]-phenylmethyl}-dimethylamin
[(7-Ethyl-1H-indol-3-yl)-phenylmethyl]-dimethylamin
3-(Dimethylaminophenylmethyl)-1H-indol-6-carbonsäure
Dimethyl-[(1-methyl-1H-indol-3-yl)-phenylmethyl]-amin
Dimethyl-[(2-phenyl-1H-indol-3-yl)-o-tolyl-methyl]-amin
[(5-Benzyloxy-1H-indol-3-yl)-o-tolylmethyl]-dimethylamin
[(2-Methoxyphenyl)-(2-methyl-1H-indol-3-yl)-methyl]-dimethylamin
{[1-(4-Methoxybenzyl)-1H-indol-3-yl]-phenyl-methyl}-dimethylamin
[(1H-Indol-3-yl)-phenylmethyl]-dimethylamin
[(5-Brom-2-fluor-phenyl)-(4-nitro-1H-indol-3-yl)-methyl]-dimethyl-amin
[(5-Brom-2-fluor-phenyl)-(1-methyl-1H-indol-3-yl)-methyl]-dimethyl-amin
[(2-Chlor-6-fluor-phenyl)-(2-phenyl-1H-indol-3-yl)-methyl]-dimethylamin
[(2-Chlor-6-fluor-phenyl)-(1-ethyl-2-phenyl-1H-indol-3-yl)-methyl]-dimethylamin
[(2-Chlor-6-fluor-phenyl)-(4-nitro-1H-indol-3-yl)-methyl]-dimethyl-amin
[(2-Chlor-6-fluor-phenyl)-(1-methyl-1H-indol-3-yl)-methyl]-dimethyl-amin
[(2-Brom-phenyl)-(4-nitro-1H-indol-3-yl)-methyl]-dimethyl-amin
[(2-Brom-phenyl)-(1-methyl-1H-indol-3-yl)-methyl]-dimethyl-amin
[(3-Brom-phenyl)-(7-ethyl-1H-indol-3-yl)-methyl]-dimethyl-amin
[(3-Brom-phenyl)-(1-methyl-1H-indol-3-yl)-methyl]-dimethyl-amin
[(4-tert-Butyl-phenyl)-(2-methyl-1H-indol-3-y-)-methyl]-dimethyl-amin
[(4-tert-Butyl-phenyl)-(1-ethyl-2-phenyl-1H-indol-3-yl)-methyl]-dimethyl-amin
[(4-tert-Butyl-phenyl)-(1-methyl-1H-indol-3-yl)-methyl]-dimethyl-amin
[(2-Chlor-4-fluor-phenyl)-(4-nitro-1H-indol-3-yl)-methyl]-dimethyl-amin
[(2-Chlor-4-fluor-phenyl)-(4-methyl-1H-indol-3-yl)-methyl]-dimethyl-amin
[(2-Chlor-6-fluor-phenyl)-(2-phenyl-1H-indol-3-yl)-methyl]-dimethyl-amin
{(2-Chlor-6-fluor-phenyl)-[2-(4-chlor-phenyl)-1H-indol-3-yl]-methyl}-dimethyl-amin
[(2-Chlor-6-fluor-phenyl)-(1-methyl-1H-indol-3-yl)-methyl]-dimethyl-amin
[(3-Chlor-phenyl)-(1-methyl-1H-indol-3-yl)-methyl]-dimethyl-amin
[(2,3-Dichlor-phenyl)-(1-methyl-1H-indol-3-yl)-methyl]-dimethyl-amin
[(2,4-Dichlor-phenyl)-(1-methyl-1H-indol-3-yl)-methyl]-dimethyl-amin
[(4-tert-Butyl-phenyl)-(1-ethyl-2-phenyl-1H-indol-3-yl)-methyl]-dimethyl-amin
[(2-Chlor-4-fluor -phenyl)-(2-methyl-1H-indol-3-yl)-methyl]-dimethyl-amin
[(2-Chlor-6-fluor-phenyl)-(2-methyl-1H-indol-3-yl)-methyl]-dimethyl-amin
[(2,3-Dimethoxy-phenyl)-(4-nitro-1H-indol-3yl)-methyl]-dimethylamin
3-[(2,3-Dimethoxy-phenyl)-dimethylamino-methyl]-4,7-dihydro-1H-indol-6-carbonsäure
3-[(2,3-Dimethoxy-phenyl)-dimethylamino-methyl]-5-hydroxy-1H-indol-2-carbonsäure
[(3,4-Dimethoxy-phenyl)-(2-methyl-1H-indol-3-yl)-methyl]-dimethyl-amin
[[2-(4-Chlor-phenyl)-1H-indol-3-yl]-(3,4-dimethoxy-phenyl)-methyl]-dimethyl-amin
[(3,4-Dimethoxy-phenyl)-(1-ethyl-2-phenyl-1H-indol-3-ylrmethyl]-dimethyl-amin
[(3,4-Dimethoxy-phenyl)-(7-ethyl-1H-indol-3-yl)-methyl]-dimethyl-amin

[(3,4-Dimethoxy-phenyl)-(1-methyl-1H-indol-3-yl)-methyl]-dimethyl-amin
[(3,4-Dimethoxy-phenyl)-[2-(4-fluor phenyl)-1H-indol-3-yl]-methyl}-dimethylamin
[[2-(3-Chlor-4-fluor-phenyl)-1H-indol-3-yl]-(3,4-dimethoxy-phenyl)-methyl]-dimethyl-amin
[(5-Chlor-1H-indol-3-yl)-(2-fluor-phenyl)-methyl]-dimethyl-amin
[(4-Fluor-phenyl)-(4-methyl-1H-indol-3-yl)-methyl]-dimethyl-amin
[(7-Ethyl-1H-indol-3-yl)-(2-methoxy-phenyl)-methyl]-dimethyl-amin
Dimethyl-[(2-methyl-1H-indol-3-yl)-o-tolyl-methyl]-amin
[(7-Ethyl-1H-indol-3-yl)-o-tolyl-methyl]-dimethyl-amin
Dimethyl-[(1-methyl-1H-indol-3-yl)-o-tolyl-methyl]-amin
[(5-Chlor-1H-indol-3-yl)-o-tolyl-methyl]-dimethyl-amin
[(5-Chlor-1H-indol-3-yl)-m-tolyl-methyl]-dimethyl-amin
Dimethyl-[(2-methyl-1H-indol-3-yl)-p-tolyl-methyl]-amin
[(5-Chlor-1H-indol-3-yl)-p-tolyl-methyl]-dimethyl-amin
Dimethyl-[(2-methyl-1H-indol-3-yl)-(3-phenoxy-phenyl)-methyl]-amin
[(1-Ethyl-2-phenyl-1H-indol-3-yl)-(3-phenoxy-phenyl)-methyl]-dimethyl-amin
[(7-Ethyl-1H-indol-3-yl)-(3-phenoxy-phenyl)-methyl]-dimethyl-amin
Dimethyl-[(1-methyl-1H-indol-3-yl)-(3-phenoxy-phenyl)-methyl]-amin
[[2-(4-Fluor-phenyl)-1H-indol-3-yl]-(3-phenoxy-phenyl)-methyl]-dimethyl-amin
[[2-(3-Chlor-4-fluor-phenyl)-1H-indol-3-yl]-(3-phenoxy-phenyl)-methyl]-dimethylamin
Dimethyl-[(4-methyl-1H-indol-3-yl)-(3-phenoxy-phenyl)-methyl]-amin
[(5-Chlor-1H-indol-3-yl)-(2-trifluormethyl-phenyl)-methyl]-dimethyl-amin
Dimethyl-[(2-methyl-1H-indol-3-yl)-(4-trifluormethyl-phenyl)-methyl]-amin.

**58.** Arzneimittel gemäß Anspruch 56 enthaltend wenigstens eine substituierte Indol-Mannichbase ausgewählt aus der Gruppe bestehend aus

1-Methyl-3-(morpholin-4-yl-phenylmethyl)-1H-indol
3-[(2-Methoxyphenyl)-pyrrolidin-1-yl-methyl]-1-methyl-1H-indol
1-Ethyl-2-phenyl-3-(pyrrolidin-1-yl-o-tolylmethyl)-1H-indol
3-[(2-Chlorophenyl)-pipeddin-1-yl-methyl]-1-ethyl-2-phenyl-1H-indol
1-Ethyl-2-phenyl-3-(phenylpyrrolidin-1-yl-methyl)-1H-indol
1-Ethyl-3-[(2-methoxyphenyl)-pyrrolidin-1-yl-methyl]-2-phenyl-1H-indol
2-Phenyl-3-(pyrrolidin-1-yl-o-tolylmethyl)-1H-indol
2-Phenyl-3-(piperidin-1-yl-o-tolylmethyl)-1H-indol
3-[(2-Chlorophenyl)-piperidin-1-yl-methyl]-2-phenyl-1H-indol
2-Phenyl-3-(phenylpyrrolidin-1-yl-methyl)-1H-indol
3-[(2-Methoxy-phenyl)-pyrrolidin-1-yl-methyl]-2-phenyl-1H-indol
4-Methyl-3-(pipendin-1-yl-o-tolyl-methyl)-1H-indol
3-[(2-Methoxyphenyl)-pyrrolidin-1-yl-methyl]-4-methyl-1H-indol
5-Benzyloxy-3-(phenyl-piperidin-1-yl-methyl)-1H-indol
7-Ethyl-3-[(2-methoxyphenyl)-morpholin-4-yl-methyl]-1H-indol
3-[(2-Methoxyphenyl)-piperidin-1-yl-methyl]-2-phenyl-1H-indol
5-Chloro-3-[(2-methoxyphenyl)-piperidin-1-yl-methyl]-1H-indol
5-Chloro-3-[(2-methoxyphenyl)-pyrrolidin-1-yl-methyl]-1H-indol
5-Benzyloxy-3-[(2-methoxyphenyl)-pyrrolidin-1-yl-methyl]-1H-indol
3-(Phenylpiperidin-1-yl-methyl)-1H-indol
1-Methyl-3-(phenylpiperidin-1-yl-methyl)-1H-indol
3-(Pheny)pyrrolidin-1-yl-methyl)-1H-indol
5-Benzyloxy-3-(phenyl-pyrrolidin-1-yl-methyl)-1H-indol.

**59.** Arzneimittel gemäß einem der Ansprüche 56 bis 58, **dadurch gekennzeichnet, daß** es als Wirkstoff ein Gemisch aus Enantiomeren wenigstens einer substituierten Indol-Mannichbase der allgemeinen Formel I gemäß einem der Ansprüche 56 bis 58 enthält, wobei das Gemisch die Enantiomere in nicht äquimolaren Mengen aufweist.

**60.** Arzneimittel gemäß Anspruch 59, **dadurch gekennzeichnet, daß** der relative Anteil eines der Enantiomere an dem Gemisch 5 bis 45 Mol-%, vorzugsweise 10 bis 40 Mol-%, bezogen auf das Gemisch der Enantiomere beträgt.

**61.** Arzneimittel gemäß einem der Ansprüche 56 bis 60 zur Behandlung/Bekämpfung von Schmerzen, vorzugsweise

von chronischen Schmerzen, und/oder inflammatorischen Reaktionen und/oder allergischen Reaktionen und/oder Drogenmißbrauch und/oder Alkoholmißbrauch und/oder Diarrhoe und/oder Gastritis und/oder Ulcera und/oder cardiovaskulären Erkrankungen und/oder Harninkontinenz und/oder Depressionen und/oder Schockzuständen und/oder Migräne und/oder Narkolepsie und/oder Übergewicht und/oder Asthma und/oder Glaukom und/oder hyperkinetischem Syndrom und/oder Antriebslosigkeit und/oder Bulimie und/oder Anorexie und/oder Katalepsie und/oder zur Anxiolyse und/oder zur Vigilanzsteigerung und/oder zur Libidosteigerung.

**62.** Verwendung wenigstens einer Verbindung der allgemeinen Formel I,

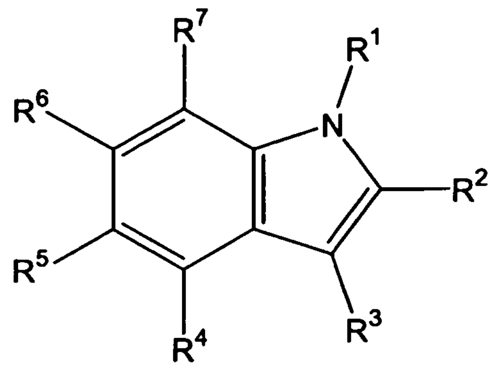

I

worin
$R^1$ = H, einen $C_{1-10}$-Alkyl-, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest, vorzugsweise H, einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, besonders bevorzugt H oder einen $C_{1-2}$-Alkyl-Rest bedeutet,
$R^2$ = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest, vorzugsweise = H, Cl, F, $NO_2$, $OR^{10}$, $CO(OR^{11})$, einen $C_{1-6}$-Alkyl- oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, besonders bevorzugt H, einen $C_{1-2}$-Alkyl- oder einen unsubstituierten Phenyl-Rest bedeutet,
$R^3$ = $CH(R^{13})N(R^{14})(R^{15})$ bedeutet,
$R^4$, $R^5$, $R^6$, $R^7$, gleich oder verschieden, = H, F, Cl, Br, $CF_3$, CN, $NO_2$, $NHR^8$, $SR^9$, $OR^{10}$, $SO_2NH_2$, $SO_2NHR^{21}$, CO $(OR^{11})$, $CH_2CO(OR^{12})$, $COR^{19}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten

oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet, vorzugsweise = H, Cl, F, $NO_2$, $OR^{10}$, $CO(OR^{11})$, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen $C_{1-6}$-Alkyl- oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, besonders bevorzugt H, $NO_2$, einen $C_{1-2}$-Alkyl- oder einen unsubstituierten Phenyl-Rest bedeuten,

$R^8$ = H, $COR^{16}$, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$Alkyl-Rest bedeutet,

$R^9$ = H, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl- oder einen Phenyl-Rest bedeutet,

$R^{10}$ = H, $COR^{17}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet, vorzugsweise = H, einen $C_{1-6}$-Alkyl-Rest oder einen über eine $C_{1-2}$-Alkylen-Gruppe gebundenen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,

$R^{11}$ = H, einen $C_{1-10}$-Alkyl- oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise = H oder einen $C_{1-6}$-Alkyl-Rest bedeutet,

$R^{12}$ = H, einen $C_{1-10}$-Alkyl-Rest oder einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl-Rest bedeutet,

$R^{13}$ = einen unsubstituierten oder wenigstens einfach mit einem OH-, einem Halogen-, einem $CF_3$-, einem CN-, einem $C_{1-6}$-Alkyl-, einem $C_{1-6}$-Alkoxy-, einem - O-Phenyl- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest bedeutet oder einen Alkyl-Rest ohne acides Proton in $\alpha$-Stellung, vorzugsweise einen unsubstituierten Phenyl-Rest oder einen wenigstens einfach mit $C_{1-4}$-Alkyl-, $C_{1-3}$-Alkoxy-, Halogen-, $CF_3$-, CN-, O-Phenyl-oder OH substituierten Phenyl-, Thiophen- oder Furfuryl-Rest, besonders bevorzugt einen unsubstituierten Phenyl-, Thiophen- oder Furfuryl-Rest oder einen 2-Methoxy-phenyl-, 3-Methoxy-phenyl-, 4-Methoxy-phenyl-, 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluor-phenyl-, 2-Chlor-4-Fluor-phenyl-, 2-Chlor-5-Fluor-phenyl-, 2-Chlor-6-Fluor-phenyl-, 4-Brom-2-Fluor-phenyl-, 3-Brom-4-Fluor phenyl-, 3-Brom-2-Fluor-phenyl-, 2,3-Dichlor-phenyl-, 2,4-Dichlor-phenyl-, 2,5-Dichlorphenyl-, 3,4-Dichlor-phenyl-, 2,3-Dimethyl-phenyl-, 2,4-Dimethyl-phenyl-, 2,5-Dimethylphenyl-, 2,3-Dimethoxy-phenyl-, 2,4-Dimethoxy-phenyl-, 2,5-Dimethoxy-phenyl-, 3,4-Dimethoxy-phenyl-, 3,4,5-Trimethoxy-phenyl-, 2-Trifluormethyl-phenyl, 3-Trifluormethyl-phenyl oder einen 4-Trifluormethyl-phenyl-Rest, ganz besonders bevorzugt einen unsubstituierten Phenyl-, Thiophen- oder Furfuryl-Rest bedeutet,

$R^{14}$, $R^{15}$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest, vorzugsweise einen gesättigten, unsubstituierten oder wenigstens

einfach substituierten $C_{1-6}$-Alkyl-Rest, besonders bevorzugt einen $CH_3$-Rest,
oder $R^{14}$ und $R^{15}$ zusammen $(CH_2)_n$ mit n = eine ganze Zahl von 3 bis 6 oder $(CH_2)_2O(CH_2)_2$, vorzugsweise $(CH_2)_n$ mit n = 4 oder 5, bedeuten,
$R^{16}$ = einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist oder einen unsubstituierten oder mit einem Halogen-, CN-, $CF_3$- oder OH-Rest substituierten Thiophen-, Pyrrolyl- oder Furfuryl-Rest, vorzugsweise einen $C_{1-6}$-Alkyl-Rest bedeutet,
$R^{17}$ = einen $C_{1-10}$-Alkyl-Rest, vorzugsweise einen $C_{1-6}$-Alkyl-Rest bedeutet,
$R^{18}$ = einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist oder einen unsubstituierten oder mit einem Halogen-, CN-, $CF_3$- oder OH-Rest substituierten Thiophen-, Pyrrolyl- oder Furfuryl-Rest bedeutet, vorzugsweise einen $C_{1-6}$-Alkyl-Rest bedeutet,
$R^{19}$ = H, $NHNH_2$, $NHR^{20}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet, vorzugsweise einen $C_{1-6}$-Alkyl-Rest oder einen über eine $C_{1-2}$-AlkylenGruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy-oder einem Phenyl-Rest substituierten Phenyl- oder Naphthyl-Rest, der gegebenenfalls mit weiteren Ringen kondensiert ist, bedeutet,
$R^{20}$ = H, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen unsubstituierten oder mit einem Halogen-, CN-, $CF_3$- oder OH-Rest substituierten Thiophen-, Pyrrolyl- oder Furfuryl-Rest, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$-Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet" vorzugsweise einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, vorzugsweise einen $C_{1-6}$-Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,
$R^{21}$ = H, $COR^{17}$, einen $C_{1-10}$-Alkyl-, einen unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, einen über eine $C_{1-6}$-Alkylen-Gruppe gebundenen, unsubstituierten oder wenigstens einfach mit einem OH-, einem Halogen-, einem $CF_3$-, einem CN-, einem $C_{1-6}$-Alkyl-, einem $C_{1-6}$-Alkoxy- oder einem Phenyl-Rest substituierten Phenyl- oder Naphthyl-Rest, der gegebenenfalls mit weiteren Ringen kondensiert ist, oder einen über eine $C_{1-6}$Alkylen-Gruppe gebundenen, unsubstituierten oder mit einem Halogen-, CN-, $CF_3$- oder OH-Rest substituierten Thiophen-, Pyrrolyl- oder Furfuryl-Rest bedeutet, vorzugsweise =H, einen $C_{1-6}$Alkyl-Rest oder einen über eine $C_{1-2}$-Alkylen-Gruppe gebundenen, unsubstituierten oder wenigstens einfach mit einem OH-, einem Halogen-, einem $CF_3$-, einem CN-, einem $C_{1-6}$Alkyl-, einem $C_{1-6}$-Alkoxy- oder einem Phenyl-Rest substituierten Phenyl- oder Naphthyl-Rest, der gegebenenfalls mit weiteren Ringen kondensiert ist, bedeutet,
und/oder deren Racemate, Enantiomere, Diastereomere und/oder entsprechende Basen und/oder entsprechende Salze von physiologisch verträglichen Säuren,
zur Herstellung eines Arzneimittels zur Bekämpfung von Schmerzen, vorzugsweise zur Bekämpfung von chronischen Schmerzen, zur Behandlung von inflammatorischen Reaktionen, zur Behandlung von allergischen Reaktionen, zur Behandlung von Drogen- und/oder Alkoholmißbrauch, zur Behandlung von Diarrhoe, zur Behandlung von Gastritis, zur Behandlung von Ulcera, zur Behandlung von cardiovaskulären Erkrankungen, zur Behandlung von

Harninkontinenz, zur Behandlung von Depressionen, zur Behandlung von Schockszuständen, zur Behandlung von Migräne, zur Behandlung von Narkolepsie, zur Behandlung von Übergewicht, zur Behandlung von Asthma, zur Behandlung von Glaukomen, zur Behandlung bei hyperkinetischem Syndrom, zur Behandlung bei Antriebslosigkeit, zur Behandlung bei Bulimie, zur Behandlung bei Anorexie, zur Behandlung bei Katalepsie, zur Anxiolyse, zur Vigilanzsteigerung und zur Libidosteigerung.

**63.** Verwendung gemäß Anspruch 62, **dadurch gekennzeichnet, dass** die substituierte Indol-Mannichbase ausgewählt wird aus der Gruppe bestehend aus

3-(Dimethylaminophenylmethyl)-1H-indol-2-carbonsäureethylester
3-(Dimethylaminophenylmethyl)-1H-indol-2-carbonsäure
[(5-Fluoro-1H-indol-3-yl)-phenylmethyl]-dimethylamin
[(1-Ethyl-2-phenyl-1H-indol-3-yl)-phenylmethyl]-dimethylamin
[(5-Methoxy-1H-indol-3-yl)-phenylmethyl]-dimethylamin
3-(Dimethylaminophenylmethyl)-5-hydroxy-1H-indol-2-carbonsäure
Dimethyl-[(2-methyl-1H-indol-3-yl)-phenylmethyl]-amin
3-(Dimethylaminophenylmethyl)-1H-indol-4-ol
Dimethyl-[(4-methyl-1H-indol-3-yl)-phenylmethyl]-amin
[(5-Chloro-1H-indol-3-yl)-phenylmethyl]-dimethylamin
[(5-Benzyloxy-1H-indol-3-yl)-phenylmethyl]-dimethylamin
Essigsäure-3-(dimethylaminophenylmethyl)-1H-indol-4-yl-ester
{[2-(4-Chlorophenyl)-1H-indol-3-yl]-phenylmethyl}-dimethylamin
5-Benzyloxy-3-(dimethylaminophenylmethyl)-1H-indol-2-carbonsäure
Dimethyl-[(2-methyl-5-nitro-1H-indol-3-yl)-phenylmethyl]-amin
Dimethyl-[(2-methyl-6-nitro-1H-indol-3-yl)-phenylmethyl]-amin
[(6-Fluoro-2-methyl-1H-indol-3-yl)-phenylmethyl]-dimethylamin
{(2-(4-Fluorophenyl)-1H-indol-3-yl]-phenylmethyl}-dimethylamin
{[2-(3-Chloro-4-fluorophenyl)-1H-indol-3-yl]-phenylmethyl}-dimethylamin
[(7-Ethyl-1H-indol-3-yl)-phenylmethyl]-dimethylamin
3-(Dimethylaminophenylmethyl)-1H-indol-6-carbonsäure
Dimethyl-[(1-methy)-1H-indol-3-yl)-phenylmethyl]-amin
Dimethyl-[(2-phenyl-1H-indol-3-yl)-o-tolyl-methyl]-amin
[(5-Benzyloxy-1H-indol-3-yl)-o-tolylmethyl]-dimethylamin
[(2-Methoxyphenyl)-(2-methyl-1H-indol-3-yl)-methyl]-dimethylamin
{[1-(4-Methoxybenzyl)-1H-indol-3-yl]-phenyl-methyl}-dimethylamin
[(1H-Indol-3-yl)-phenylmethyl]-dimethylamin
[(5-Brom-2-fluor-phenyl)-(4-nitro-1H-indol-3-yl)-methyl]-dimethyl-amin
[(5-Brom-2-fluor-phenyl)-(1-methyl-1H-indol-3-yl)-methyl]-dimethyl-amin
[(2-Chlor-6-fluor-phenyl)-(2-phenyl-1H-indol-3-yl)-methyl]-dimethylamin
[(2-Chlor-6-fluor-phenyl)-(1-ethyl-2-phenyl-1H-indol-3-yl)-methyl]-dimethylamin
[(2-Chlor-6-fluor-phenyl)-(4-nitro-1H-indol-3-yl)-methyl]-dimethyl-amin
[(2-Chlor-6-fluor-phenyl)-(1-methyl-1H-indol-3-yl)-methyl]-dimethyl-amin
[(2-Brom-phenyl)-(4-nitro-1H-indol-3-yl)-methyl]-dimethyl-amin
[(2-Brom-phenyl)-(1-methyl-1H-indol-3-yl)-methyl]-dimethyl-amin
[(3-Brom-phenyl)-(7-ethyl-1H-indol-3-yl)-methyl]-dimethyl-amin
[(3-Brom-phenyl)-(1-methyl-1H-indol-3-yl)-methyl]-dimethyl-amin
[(4-tert-Butyl-phenyl)-(2-methyl-1H-indol-3-y-)-methyl]-dimethyl-amin
[(4-tert-Butyl-phenyl)-(1-ethyl-2-phenyl-1H-indol-3-yl)-methyl]-dimethyl-amin
[(4-tert-Butyl-phenyl)-(1-methyl-1H-indol-3-yl)-methyl]-dimethyl-amin
[(2-Chlor-4-fluor-phenyl)-(4-nitro-1H-indol-3-yl)-methyl]-dimethyl-amin
[(2-Chlor-4-fluor-phenyl)-(4-methyl-1H-indol-3-ylrmethyl]-dimethyl-amin
[(2-Chlor-6-fluor-phenyl)-(2-phenyl-1H-indol-3-yl)-methyl]-dimethyl-amin
{(2-Chlor-6-fluor-phenyl)-[2-(4-chlor-phenyl)-1H-indol-3-yl]-methyl}-dimethyl-amin
[(2-Chlor-6-fluor phenyl)-(1-methyl-1H-indol-3-yl)-methyl]-dimethyl-amin
[(3-Chlor-pheny)-(1-methyl-1H-indo)-3-yl)-methyl]-dimethyl-amin
[(2,3-Dichlor-phenyl-(1-methyl-1H-indol-3-yl)-methyl]-dimethyl-amin
[(2,4-Dichlor-phenyl)-(1-methyl-1H-indol-3-yl)-methyl]-dimethyl-amin
[(4-tert-Butyl-phenyl)-(1-ethyl-2-phenyl-1H-indol-3-yl)-methyl]-dimethyl-amin

[(2-Chlor 4-fluor-phenyl)-(2-methyl-1H-indol-3-yl)-methyl]-dimethyl-amin
[(2-Chlor-6-fluor-phenyl)-(2-methyl-1H-indol-3-yl)-methyl]-dimethyl-amin
[(2,3-Dimethoxy-phenyl)-(4-nitro-1H-indol-3yl)-methyl]-dimethylamin
3-[(2,3-Dimethoxy-phenyl)-dimethylamino-methyl]-4,7-dihydro-1H-indol-6-carbonsäure
3-[(2,3-Dimethoxy-phenyl)-dimethylamino-methyl]-5-hydroxy-1H-indol-2-carbonsäure
[(3,4-Dimethoxy-phenyl)-(2-methyl-1H-indol-3-yl)-methyl]-dimethyl-amin
[[2-(4-Chlor-phenyl)-1H-indol-3-yl]-(3,4-dimethoxy-phenyl)-methyl]-dimethyl-amin
[(3,4-Dimethoxy-phenyl)-(1-ethyl-2-phenyl-1H-indol-3-yl)-methyl]-dimethyl-amin
[(3,4-Dimethoxy-phenyl)-(7-ethyl-1H-indol-3-yl)methyl]-dimethyl-amin
[(3,4-Dimethoxy-phenyl)-(1-methyl-1H-indol-3-yl)methyl]-dimethyl-amin
[(3,4-Dimethoxy-phenyl)-[2--(4-fluor-phenyl)-1H-indol-3-yl]-methyl}-dimethylamin
[[2-(3-Chlor-4-fluor-phenyl)-1H-indol-3-yl]-(3,4-dimethoxy-phenyl)-methyl]-dimethyl-amin
[(5-Chlor-1H-indol-3-yl)-(2-fluor-phenyl)methyl]-dimethyl-amin
[(4-Fluor-phenyl)-(4-methyl-1H-indol-3-yl)-methyl]-dimethyl-amin
[(7-Ethyl-1H-indol-3-yl)-(2-methoxy-phenyl)-methyl]-dimethyl-amin
Dimethyl-[(2-methyl-1H-indol-3-yl)-o-tolyl-methyl]-amin
[(7-Ethyl-1H-indol-3-yl)-o-tolyl-methyl]-dimethyl-amin
Dimethyl-[(1-methyl-1H-indol-3-yl)-o-tolyl-methyl]-amin
[(5-Chlor-1H-indol-3-yl)-o-tolyl-methyl]-dimethyl-amin
[(5-Chlor-1H-indol-3-yl)-m-tolyl-methyl]-dimethyl-amin
Dimethyl-[(2-methyl-1H-indol-3-yl)-p-tolyl-methyl]-amin
[(5-Chlor-1H-indol-3-yl)-p-tolyl-methyl]-dimethyl-amin
Dimethyl-[(2-methyl-1H-indol-3-yl)-(3-phenoxy-phenyl)-methyl]-amin
[(1-Ethyl-2-phenyl-1H-indol-3-yl)-(3-phenoxy-phenyl)-methyl]-dimethyl-amin
[(7-Ethyl-1H-indol-3-yl)-(3-phenoxy-phenyl)-methyl]-dimethyl-amin
Dimethyl-[(1-methyl-1H-indol-3-yl)-(3-phenoxy-phenyl)-methyl]-amin
[[2-(4-Fluor-phenyl)-1H-indol-3-yl]-(3-phenoxy-phenyl)-methyl]-dimethyl-amin
[[2-(3-Chlor-4-fluor-phenyl)-1H-indol-3-yl]-(3-phenoxy-phenyl)-methyl]-dimethylamin
Dimethyl-[(4-methyl-1H-indol-3-yl)-(3-phenoxy-phenyl)-methyl]-amin
[(5-Chlor-1H-indol-3-yl)-(2-trifluormethyl-phenyl)-methyl]-dimethyl-amin
Dimethyl-[(2-methyl-1H-indol-3-yl)-(4-trifluormethyl-phenyl)-methyl]-amin.

**64.** Verwendung gemäß Anspruch 62, **dadurch gekennzeichnet, dass** die substituierte Indol-Mannichbase ausgewählt wird aus der Gruppe bestehend aus

1-Methyl-3-(morpholin-4-yl-phenylmethyl)-1H-indol
3-[(2-Methoxyphenyl)-pyrrolidin-1-yl-methyl]-1-methyl-1H-indol
1-Ethyl-2-phenyl-3-(pyrrolidin-1-yl-*o*-tolylmethyl)-1H-indol
3-[(2-Chlorophenyl)-piperidin-1-yl-methyl]-1-ethyl-2-phenyl-1H-indol
1-Ethyl-2-phenyl-3-(phenylpyrrolidin-1-yl-methyl)-1H-indol
1-Ethyl-3-[(2-methoxyphenyl)-pyrrolidin-1-yl-methyl]-2-phenyl-1H-indol
2-Phenyl-3-(pyrrolidin-1-yl-*o*-tolylmethyl)-1H-indol
2-Phenyl-3-(piperidin-1-yl-*o*-tolylmethyl)-1H-indol
3-[(2-Chlorophenyl)-piperidin-1-yl-methyl]-2-phenyl-1H-indol
2-Phenyl-3-(phenylpyrrolidin-1-yl-methyl)-1H-indol
3-[(2-Methoxy-phenyl)-pyrrolidin-1-yl-methyl]-2-phenyl-1H-indol
4-Methyl-3-(piperidin-1-yl-o-tolyl-methyl)-1H-indol
3-[(2-Methoxyphenyl)-pyrrolidin-1-yl-methyl]-4-methyl-1H-indol
5-Benzyloxy-3-(phenyl-piperidin-1-yl-methyl)-1H-indol
7-Ethyl-3-[(2-methoxyphenyl)-morpholin-4-yl-methyl]-1H-indol
3-[(2-Methoxyphenyl)-piperidin-1-yl-methyl]-2-phenyl-1H-indol
5-Chloro-3-[(2-methoxyphenyl)-piperidin-1-yl-methyl]-1H-indol
5-Chloro-3-[(2-methoxyphenyl)-pyrrolidin-1-yl-methyl]-1H-indol
5-Benzyloxy-3-[(2-methoxyphenyl)-pyrrolidin-1-yl-methyl]-1H-indol
3-(Phenylpiperidin-1-yl-methyl)-1H-indol
1-Methyl-3-(phenylpiperidin-1-yl-methyl)-1H-indol
3-(Phenylpyrrolidin-1-yl-methyl)-1H-indol
5-Benzyloxy-3-(phenyl-pyrrolidin-1-yl-methyl)-1H-indol.

**Claims**

1. Substituted indole Mannich bases of the general formula I

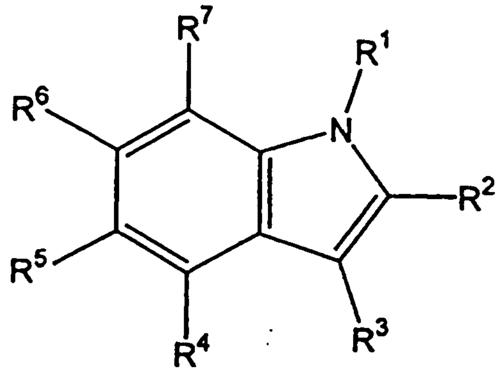

I

wherein

$R^1$ = H, a $C_{1-10}$-alkyl radical, a phenyl or naphthyl radical bonded via a $C_{1-6}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, or a thiophene, pyrrolyl or furfuryl radical bonded via a $C_{1-6}$-alkylene group, and which is unsubstituted or is substituted with a halogen, CN, $CF_3$, or OH radical,

$R^2$ = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, a $C_{1-10}$-alkyl, a phenyl or naphthyl radical which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted with a halogen, CN, $CF_3$, or OH radical, a phenyl or naphthyl radical bonded via a $C_{1-6}$-alkylene group, which is unsubstituted or is at least monosubstituted with an OH, halogen, a $CF_3$, a CN, a $C_{1-6}$-alkyl, a $C_{1-6}$-alkoxy or a phenyl radical, which is optionally condensed with further rings, or denotes a thiophene, pyrollyl or furfuryl radical bonded via a $C_{1-6}$-alkylene group, which is unsubstituted or is substituted with a halogen, CN, $CF_3$, or OH radical,

$R^3$ = $CH(R^{13})N(R^{14})(R^{15})$,

$R^4$, $R^5$, $R^6$, $R^7$ are identical or different and = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, a $C_{1-10}$-alkyl radical, a phenyl or naphthyl radical which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted with a halogen, CN, $CF_3$, or OH radical, a phenyl or naphthyl radical bonded via a $C_{1-6}$-alkylene group, which is unsubstituted or is at least monosubstituted with an OH, halogen, a $CF_3$, a CN, a $C_{1-6}$-alkyl, a $C_{1-6}$-alkoxy or a phenyl radical, which is optionally condensed with further rings, or denotes a thiophene, pyrollyl or furfuryl radical bonded via a $C_{1-6}$-alkylene group, which is unsubstituted or is substituted with a halogen, CN, $CF_3$, or OH radical,

$R^8$ = H, $COR^{16}$, a $C_{1-10}$-alkyl radical or a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings,

$R^9$ = H, a $C_{1-10}$-alkyl radical or a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings,

$R^{10}$ = H, $COR^{17}$, a $C_{1-10}$-alkyl radical or a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, a phenyl or naphthyl radical bonded via a $C_{1-6}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, or a thiophene, pyrrolyl or furfuryl radical bonded via a $C_{1-6}$-alkylene group, and which is unsubstituted or is substituted with a halogen, CN, $CF_3$, or OH radical,

$R^{11}$ = H or a $C_{1-10}$-alkyl radical or a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings,

$R^{12}$ = H, a $C_{1-10}$-alkyl radical or a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings,
$R^{13}$ = an unsubstituted phenyl radical or a phenyl radical which is at least monosubstituted by $C_{1-4}$-alkyl, halogen, $CF_3$, CN or OH,
$R^{14}$, $R^{15}$ are identical or different and denote a branched or unbranched, saturated or unsaturated, unsubstituted or at least monosubstituted $C_{1-6}$-alkyl radical or an unsubstituted or at least monosubstituted phenyl, benzyl or phenethyl radical,
or $R^{14}$ and $R^{15}$ together denote $(CH_2)_n$, where n = an integer from 3 to 6, or $(CH_2)_2O(CH_2)_2$,
$R^{16}$ = a $C_{1-10}$-alkyl radical, a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, or a thiophene, pyrrolyl or furfuryl radical which is unsubstituted or is substituted with a halogen, CN, $CF_3$ or OH radical,
$R^{17}$ = a $C_{1-10}$-alkyl radical
$R^{18}$ = a $C_{1-10}$-alkyl radical, a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, or a thiophene, pyrrolyl or furfuryl radical which is unsubstituted or is substituted with a halogen, CN, $CF_3$ or OH radical,
$R^{19}$ = H, $NHNH_2$, $NHR^{20}$, a $C_{1-10}$-alkyl, a phenyl or naphthyl radical which is unsubstituted or at least monosubstituted with an OH, halogen a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted with a halogen, CN, $CF_3$, or OH radical, a phenyl or naphthyl radical bonded via a $C_{1-6}$-alkylene group, which is unsubstituted or is at least monosubstituted with an OH, halogen, a $CF_3$, a CN, a $C_{1-6}$-alkyl, a $C_{1-6}$-alkoxy or a phenyl radical, which is optionally condensed with further rings, or denotes a thiophene, pyrollyl or furfuryl radical bonded via a $C_{1-6}$-alkylene group, which is unsubstituted or is substituted with a halogen, CN, $CF_3$, or OH radical,
$R^{20}$ = H, a $C_{1-10}$-alkyl radical, a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, or a thiophene, pyrrolyl or furfuryl radical which is unsubstituted or is substituted with a halogen, CN, $CF_3$ or OH radical, a phenyl or naphthyl radical bonded via a $C_{1-6}$-alkylene group, which is unsubstituted or is at least monosubstituted with an OH, halogen, a $CF_3$, a CN, a $C_{1-6}$-alkyl, a $C_{1-6}$-alkoxy or a phenyl radical, which is optionally condensed with further rings, or denotes a thiophene, pyrollyl or furfuryl radical bonded via a $C_{1-6}$-alkylene group, which is unsubstituted or is substituted with a halogen, CN, $CF_3$, or OH radical,
$R^{21}$ = H, $COR^{17}$, a $C_{1-10}$-alkyl radical, a phenyl or naphthyl radical that is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, a phenyl or naphthyl radical bonded via a $C_{1-6}$-alkylene group, and which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, or denotes a thiophene, pyrollyl or furfuryl radical bonded via a $C_{1-6}$-alkylene group, and which is unsubstituted or is substituted with a halogen, CN, $CF_3$ or OH radical,
and/or their racemates, enantiomers, diastereomers and/or corresponding bases and/or corresponding salts of physiologically tolerated acids,
excluding the racemates of the compounds in which the radical $R^3$ = CH ($R^{13}$) N ($R^{14}$) ($R^{15}$) and in each case
the radicals $R^4$ to $R^7$ and $R^2$ = H, the radical $R^{13}$ = phenyl, the radicals $R^{14}$ and $R^{15}$ together = $(CH_2)_5$ and the radical $R^1$ = H, $CH_3$ or benzyl
or
the radicals $R^4$ to $R^7$ and $R^2$ = H, the radical $R^{13}$ = phenyl, the radicals $R^{14}$, $R^{15}$ each = $CH_3$ and the radical $R^1$ = H, $CH_3$ or benzyl
or
the radicals $R^9$ to $R^7$ and $R^1$ = H, the radical $R^{13}$ = 2-chlorophenyl, the radicals $R^{14}$ and $R^{15}$ each = $CH_3$ or the radicals $R^{14}$ and $R^{15}$ together = $(CH_2)_2O(CH_2)_2$, the radical $R^2$ = $COOR^{11}$ and the radical $R^{11}$ = $C_2H_5$ and the corresponding hydrochlorides
or
the radicals $R^4$ to $R^7$ and $R^1$ = H, the radical $R^{13}$ = phenyl, the radicals $R^{14}$ and $R^{15}$ together = $(CH_2)_5$, the radical $R^2$ = $COOR^{11}$ and the radical $R^{11}$ = $CH_3$ and the corresponding hydrochloride
or
the radicals $R^4$ to $R^7$ and $R^2$ = H, the radical $R^{13}$ = phenyl, the radicals $R^{14}$ and $R^{15}$ together = $(CH_2)_4$, the radical $R^1$ = H, $CH_3$, $CH_2$-$CH$=$CH_2$ or 4-chlorobenzyl and for $R^1$ = $CH_3$ also the corresponding hydrochloride of the compound
or
the radicals $R^4$ to $R^7$, $R^1$ and $R^2$ = H, the radical $R^{13}$ = phenyl, the radical $R^{14}$ = phenyl and the radical $R^{15}$ = 4-

ethoxyphenyl
or
the radicals $R^4$ to $R^7$ and $R^1$= H, the radical $R^{13}$ = phenyl, 2-hydroxyphenyl or 2-hydroxy-5-methylphenyl, the radicals $R^{14}$ and $R^{15}$ together = $(CH_2)_5$ and the radical $R^2$ = $CH_3$, and for $R^{13}$ = phenyl, also the corresponding hydrogen sulphate,
or
the radicals $R^4$ to $R^7$ and $R^1$ = H, the radical $R^{13}$ = phenyl, the radicals $R^{14}$ and $R^{15}$ together = $(CH_2)_2O(CH_2)_2$ and the radical $R^2$ = $CH_3$ and the corresponding hydrogen sulfate of the compound.

**2.** Substituted indole Mannich bases according to claim 1, **characterized in that** the radical $R^1$ represents H and the radicals $R^2$ to $R^{21}$ have the meaning according to claim 1.

**3.** Substituted indole Mannich bases according to claim 1, **characterized in that** the radical $R^1$ represents a $C_{1-6}$-alkyl radical, preferably a $C_{1-2}$-alkyl radical, and the radicals $R^2$ to $R^{21}$ have the meaning according to claim 1.

**4.** Substituted indole Mannich bases according to claim 1, **characterized in that** the radical $R^1$ denotes a phenyl or naphthyl radical bonded via a $C_{1-2}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, and the radicals $R^2$ to $R^{21}$ have the meaning according to claim 1.

**5.** Substituted indole Mannich bases according to one of claims 1 to 4, **characterized in that** the radical $R^2$ represents H and the radicals $R^3$ to $R^{21}$ have the meaning according to claim 1.

**6.** Substituted indole Mannich bases according to one of claims 1 to 4, **characterized in that** the radical $R^2$ represents a $C_{1-6}$-alkyl radical, preferably a $C_{1-2}$-alkyl radical, and the radicals $R^3$ to $R^{21}$ have the meaning according to claim 1.

**7.** Substituted indole Mannich bases according to one of claims 1 to 4, **characterized in that** the radical $R^2$ represents an unsubstituted phenyl radical and the radicals $R^3$ to $R^{21}$ have the meaning according to claim 1.

**8.** Substituted indole Mannich bases according to one of claims 1 to 4, **characterized in that** the radical $R^2$ represents a phenyl or naphthyl radical bonded via a $C_{1-2}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, and the other radicals $R^3$ to $R^{21}$ have the meaning according to claim 1.

**9.** Substituted indole Mannich bases according to one of claims 1 to 4, **characterized in that** the radical $R^2$ represents Cl or F and the other radicals $R^3$ to $R^{21}$ have the meaning according to claim 1.

**10.** Substituted indole Mannich bases according to one of claims 1 to 4, **characterized in that** the radical $R^2$ represents $NO_2$ and the other radicals $R^3$ to $R^{21}$ have the meaning according to claim 1.

**11.** Substituted indole Mannich bases according to one of claims 1 to 4, **characterized in that** the radical $R^2$ represents $OR^{10}$ and the other radicals $R^3$ to $R^{21}$ have the meaning according to claim 1.

**12.** Substituted indole Mannich bases according to one of claims 1 to 4, **characterized in that** the radical $R^2$ represents $CO(OR^{11})$ and the other radicals $R^3$ to $R^{21}$ have the meaning according to claim 1.

**13.** Substituted indole Mannich bases according to one of claims 1 to 12, **characterized in that** at least one of the radicals $R^4$, $R^5$, $R^6$ or $R^7$ represents H and the in each case remaining radicals $R^4$, $R^5$, $R^6$ or $R^7$ and $R^8$ to $R^{21}$ have the meaning according to claim 1.

**14.** Substituted indole Mannich bases according to one of claims 1 to 12, **characterized in that** at least one of the radicals $R^4$, $R^5$, $R^6$ or $R^7$ represents Cl or F and in each case remaining particular radicals $R^4$, $R^5$, $R^6$ or $R^7$ and $R^8$ to $R^{21}$ have the meaning according to claim 1.

**15.** Substituted indole Mannich bases according to one of claims 1 to 12, **characterized in that** at least one of the radicals $R^4$, $R^5$, $R^6$ or $R^7$ represents a $C_{1-6}$-alkyl radical, preferably a $C_{1-2}$-alkyl radical, and the in each case remaining radicals $R^4$, $R^5$, $R^6$ or $R^7$ and $R^8$ to $R^{21}$ have the meaning according to claim 1.

**16.** Substituted indole Mannich bases according to one of claims 1 to 12, **characterized in that** at least one of the radicals $R^4$, $R^5$, $R^6$ or $R^7$ represents $NO_2$ and the in each case remaining radicals $R^4$, $R^5$, $R^6$ or $R^7$ and $R^8$ to $R^{21}$ have the meaning according to claim 1.

**17.** Substituted indole Mannich bases according to one of claims 1 to 12, **characterized in that** at least one of the radicals $R^4$, $R^5$, $R^6$ or $R^7$ denotes a phenyl or naphthyl radical bonded via a $C_{1-6}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, and the in each case remaining radicals $R^4$, $R^5$, $R^6$ or $R^7$ and $R^8$ to $R^{21}$ have the meaning according to claim 1.

**18.** Substituted indole Mannich bases according to one of claims 1 to 12, **characterized in that** at least one of the radicals $R^4$, $R^5$, $R^6$ or $R^7$ represents $OR^{10}$ and the in each case remaining radicals $R^4$, $R^5$, $R^6$ or $R^7$ and $R^8$ to $R^{21}$ have the meaning according to claim 1.

**19.** Substituted indole Mannich bases according to one of claims 1 to 12, **characterized in that** at least one of the radicals $R^4$, $R^5$, $R^6$ or $R^7$ represents CO ($OR^{11}$) and the in each case remaining radicals $R^4$, $R^5$, $R^6$ or $R^7$ and $R^8$ to $R^{21}$ have the meaning according to claim 1.

**20.** Substituted indole Mannich bases according to one of claims 1 to 12, **characterized in that** at least one of the radicals $R^4$, $R^5$, $R^6$ or $R^7$ denotes a phenyl or naphthyl radical, which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, preferably represents an unsubstituted phenyl radical, and the in each case remaining radicals $R^4$, $R^5$, $R^6$ or $R^7$ and $R^8$ to $R^{21}$ have the meaning according to claim 1.

**21.** Substituted indole Mannich bases according to one of claims 1 to 20, **characterized in that** the radical $R^8$ represents a $C_{1-6}$-alkyl radical and the radicals $R^9$ to $R^{21}$ have the meaning according to claim 1.

**22.** Substituted indole Mannich bases according to one of claims 1 to 21, **characterized in that** the radical $R^9$ represents a $C_{1-6}$-alkyl radical and the radicals $R^{10}$ to $R^{21}$ have the meaning according to claim 1.

**23.** Substituted indole Mannich bases according to one of claims 1 to 21, **characterized in that** the radical $R^9$ represents a phenyl radical and the radicals $R^{10}$ to $R^{21}$ have the meaning according to claim 1.

**24.** Substituted indole Mannich bases according to one of claims 1 to 23, **characterized in that** the radical $R^{10}$ represents H and the radicals $R^{11}$ to $R^{21}$ have the meaning according to claim 1.

**25.** Substituted indole Mannich bases according to one of claims 1 to 23, **characterized in that** the radical $R^{10}$ represents a $C_{1-6}$-alkyl radical and the radicals $R^{11}$ to $R^{21}$ have the meaning according to claim 1.

**26.** Substituted indole Mannich bases according to one of claims 1 to 23, **characterized in that** the radical $R^{10}$ represents a phenyl or naphthyl radical bonded via a $C_{1-2}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, and the radicals $R^{11}$ to $R^{21}$ have the meaning according to claim 1.

**27.** Substituted indole Mannich bases according to one of claims 1 to 26, **characterized in that** the radical $R^{11}$ represents H and the radicals $R^{12}$ to $R^{21}$ have the meaning according to claim 1.

**28.** Substituted indole Mannich bases according to one of claims 1 to 26, **characterized in that** the radical $R^{11}$ represents a $C_{1-6}$-alkyl radical and the radicals $R^{12}$ to $R^{21}$ have the meaning according to claim 1.

**29.** Substituted indole Mannich bases according to one of claims 1 to 28, **characterized in that** the radical $R^{12}$ represents a $C_{1-6}$-alkyl radical and the radicals $R^{13}$ to $R^{21}$ have the meaning according to claim 1.

**30.** Substituted indole Mannich bases according to one of claims 1 to 29, **characterized in that** the radical $R^{13}$ denotes an unsubstituted phenyl radical or a phenyl radical which is at least monosubstituted by methyl, tert-butyl, F, Cl, Br or $CF_3$, preferably an unsubstituted phenyl radical or a 2-methyl-phenyl, 3-methyl-phenyl, 4-methyl-phenyl, 2-tert-butyl-phenyl, 3-tert-butyl-phenyl, 4-tert-butyl-phenyl, 2-fluoro-phenyl, 3-fluoro-phenyl, 4-fluoro-phenyl, 2-chloro-phenyl, 3-chloro-phenyl, 4-chloro-phenyl, 2-bromo-phenyl, 3-bromo-phenyl, 4-bromo-phenyl, 5-bromo-2-fluoro-phenyl,

2-chloro-4-fluoro-phenyl, 2-chloro-5-fluoro-phenyl, 2-chloro-6-fluoro-phenyl, 4-bromo-2-fluoro-phenyl, 3-bromo-4-fluoro-phenyl, 3-bromo-2-fluoro-phenyl, 2,3-dichloro-phenyl, 2,4-dichlorophenyl, 2,5-dichlorophenyl, 3,4-dichloro-phenyl, dimethyl-phenyl, 2,4-dimethyl-phenyl, 2,5-dimethylphenyl, 2-trifluoromethyl-phenyl, 3-trifluoromethyl-phenyl or 4-trifluoromethyl-phenyl radical, particularly preferably an unsubstituted phenyl radical, and the radicals $R^{14}$ to $R^{21}$ have the meaning according to claim 1.

**31.** Substituted indole Mannich bases according to one of claims 1 to 30, **characterized in that** at least one of the radicals $R^{14}$ or $R^{15}$ represents a branched or unbranched, saturated or unsaturated, unsubstituted or at least monosubstituted $C_{1-6}$-alkyl radical, preferably a $CH_3$ radical, and the in each case other radical $R^{14}$ or $R^{15}$ and the radicals $R^{16}$ to $R^{21}$ have the meaning according to claim 1.

**32.** Substituted indole Mannich bases according to one of claims 1 to 30, **characterized in that** the radicals $R^{14}$ and $R^{15}$ together denote $(CH_2)_n$, where n = 4 or 5, and the radicals $R^{16}$ to $R^{21}$ have the meaning according to claim 1.

**33.** Substituted indole Mannich bases according to one of claims 1 to 32, **characterized in that** the radical $R^{16}$ represents a $C_{1-6}$-alkyl radical and the radicals $R^{17}$ to $R^{21}$ have the meaning according to claim 1.

**34.** Substituted indole Mannich bases according to one of claims 1 to 33, **characterized in that** the radical $R^{17}$ represents a $C_{1-6}$-alkyl radical and the radicals $R^{18}$ to $R^{21}$ have the meaning according to claim 1.

**35.** Substituted indole Mannich bases according to one of claims 1 to 34, **characterized in that** the radical $R^{18}$ represents a $C_{1-6}$-alkyl radical and the radicals $R^{19}$ to $R^{21}$ have the meaning according to claim 1.

**36.** Substituted indole Mannich bases according to one of claims 1 to 35, **characterized in that** the radical $R^{19}$ represents a $C_{1-6}$-alkyl radical and the radicals $R^{20}$ and $R^{21}$ have the meaning according to claim 1.

**37.** Substituted indole Mannich bases according to one of claims 1 to 35, **characterized in that** the radical $R^{19}$ represents a phenyl or naphthyl radical bonded via a $C_{1-2}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, and the radicals $R^{20}$ and $R^{21}$ have the meaning according to claim 1.

**38.** Substituted indole Mannich bases according to one of claims 1 to 37, **characterized in that** the radical $R^{20}$ represents a $C_{1-6}$-alkyl radical and the radical $R^{21}$ has the meaning according to claim 1.

**39.** Substituted indole Mannich bases according to one of claims 1 to 37, **characterized in that** the radical $R^{20}$ represents a phenyl or naphthyl radical bonded via a $C_{1-2}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, and the radical $R^{21}$ has the meaning according to claim 1.

**40.** Substituted indole Mannich bases according to one of claims 1 to 39, **characterized in that** the radical $R^{21}$ represents H.

**41.** Substituted indole Mannich bases according to one of claims 1 to 39, **characterized in that** the radical $R^{21}$ represents a $C_{1-6}$-alkyl radical.

**42.** Substituted indole Mannich bases according to one of claims 1 to 39, **characterized in that** the radical $R^{21}$ represents a phenyl or naphthyl radical bonded via a $C_{1-2}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings.

**43.** Substituted indole Mannich bases according to claim 1:

3-(dimethylaminophenylmethyl)-1H-indole-2-carboxylic acid ethyl ester
3-(dimethylaminophenylmethyl)-1H-indole-2-carboxylic acid
[(5-fluoro-1H-indol-3-yl)-phenylmethyl]-dimethylamine
[(1-ethyl-2-phenyl-1H-indol-3-yl)-phenylmethyl]-dimethylamine
[(5-methoxy-1H-indol-3-yl)-phenylmethyl]-dimethylamine
3-(dimethylaminophenylmethyl)-5-hydroxy-1H-indole-2-carboxylic acid

dimethyl-[(2-methyl-1H-indol-3-yl)-phenylmethyl]-amine
3-(dimethylaminophenylmethyl)-1H-indol-4-Ol
dimethyl-[(4-methyl-1H-indol-3-yl)-phenylmethyl]-amine
[(5-chloro-1H-indol-3-yl)-phenylmethyl]-dimethylamine
[(5-benzyloxy-1H-indol-3-yl)-phenylmethyl]-dimethylamine
acetic acid 3-(dimethylaminophenylmethyl)-1H-indol-4-yl ester
{[2-(4-chlorophenyl)-1H-indol-3-yl]-phenylmethyl}-dimethylamine
5-benzyloxy-3-(dimethylaminophenylmethyl)-1H-indole-2-carboxylic acid
dimethyl-[(2-methyl-5-nitro-1H-indol-3-yl)-phenylmethyl]-amine
dimethyl-[(2-methyl-6-nitro-1H-indol-3-yl)-phenylmethyl]-amine
[(6-fluoro-2-methyl-1H-indol-3-yl)-phenylmethyl]-dimethylamine
{[2-(4-fluorophenyl)-1H-indol-3-yl]-phenylmethyl}-dimethylamine
{[2-(3-chloro-4-fluorophenyl)-1H-indol-3-yl]-phenylmethyl}-dimethylamine
[(7-ethyl-1H-indol-3-yl)-phenylmethyl]-dimethylamine
3-(dimethylaminophenylmethyl)-1H-indole-6-carboxylic acid
dimethyl-[(1-methyl-1H-indol-3-yl)-phenylmethyl]-amine
dimethyl-[(2-phenyl-1H-indol-3-yl)-o-tolyl-methyl]-amine
[5-benzyloxy-1H-indol-3-yl)-o-tolylmethyl]-dimethylamine
{[1-(4-methoxybenzyl)-1H-indol-3-yl]-phenyl-methyl}-dimethylamine
[(1H-indol-3-yl)-phenylmethyl]-dimethylamine
[(5-bromo-2-fluoro-phenyl)-(4-nitro-1H-indol-3-yl)-methyl]-dimethyl-amine
[(5-bromo-2-fluoro-phenyl)-(1-methyl-1H-indol-3-yl)-methyl]-dimethyl-amine
[(2-chloro-6-fluoro-phenyl)-(2-phenyl-1H-indol-3-yl)-methyl]-dimethylamine
[(2-chloro-6-fluorophenyl)-(1-ethyl-2-phenyl-1H-indol-3-yl)-methyl]-dimethylamine
[(2-chloro-6-fluorophenyl)-(4-nitro-1H-indol-3-yl)-methyl]-dimethylamine
[(2-chloro-6-fluorophenyl)-(1-methyl-1H-indol-3-yl)-methyl]-dimethylamine
[(2-bromophenyl)-(4-nitro-1H-indol-3-yl)-methyl]-dimethylamine
[(2-bromophenyl)-(1-methyl-1H-indol-3-yl)-methyl]-dimethylamine
[(3-bromophenyl)-(7-ethyl-1H-indol-3-yl)-methyl]-dimethylamine
[(3-bromophenyl)-(1-methyl-1H-indol-3-yl)-methyl]-dimethylamine
[(4-tert-butylphenyl)-(2-methyl-1H-indol-3-yl)-methyl]-dimethylamine
[(4-tert-butylphenyl)-(1-ethyl-2-phenyl-1H-indol-3-yl)-methyl]-dimethylamine
[(4-tert-butylphenyl)-(1-methyl-1H-indol-3-yl)-methyl]-dimethylamine
[(2-chloro-4-fluorophenyl)-(4-nitro-1H-indol-3-yl)-methyl]-dimethylamine
[(2-chloro-4-fluorophenyl)-(4-methyl-1H-indol-3-yl)-methyl]-dimethylamine
[(2-chloro-6-fluorophenyl)-(2-phenyl-1H-indol-3-yl)-methyl]-dimethylamine
{(2-chloro-6-fluoro-phenyl)-[2-(4-chlorophenyl)-1H-indol-3-yl]-methyl}-dimethylamine
[(2-chloro-6-fluorophenyl)-(1-methyl-1H-indol-3-yl)-methyl]-dimethylamine
[(3-chlorophenyl)-(1-methyl-1H-indol-3-yl)-methyl]-dimethylamine
[(2,3-dichloro-phenyl)-(1-methyl-1H-indol-3-yl)-methyl]-dimethyl-amine
[(2,4-dichlorophenyl)-(1-methyl-1H-indol-3-yl)-methyl]-dimethylamine.
[(4-tert-butylphenyl)-(1-ethyl-2-phenyl-1H-indol-3-yl)-methyl]-dimethylamine
[(2-chloro-4-fluorophenyl)-(2-methyl-1H-indol-3-yl)-methyl] -dimethylamine
[(2-chloro-6-fluorophenyl)-(2-methyl-1H-indol-3-yl)-methyl]-dimethylamine
[(5-chloro-1H-indol-3-yl)-(2-fluorophenyl)-methyl]-dimethylamine
[(4-fluorophenyl)-(4-methyl-1H-indol-3-yl)-methyl]-dimethylamine
dimethyl-[(2-methyl-1H-indol-3-yl)-o-tolylmethyl]-amine
[(7-ethyl-1H-indol-3-yl)-o-tolylmethyl]-dimethyl-amine
dimethyl-[(1-methyl-1H-indol-3-yl)-o-tolylmethyl]-amine
[(5-chloro-1H-indol-3-yl)-o-tolylmethyl]-dimethylamine
[(5-chloro-1H-indol-3-yl)-m-tolylmethyl]-dimethylamine
dimethyl-[(2-methyl-1H-indol-3-yl)-p-tolylmethyl]-amine
[(5-chloro-1H-indol-3-yl)-p-tolylmethyl]-dimethylamine
[(5-chloro-1H-indol-3-yl)-(2-trifluoromethylphenyl)-methyl]-dimethylamine
dimethyl-[(2-methyl-1H-indol-3-yl)-(4-trifluoromethyl-phenyl)-methyl]-amine.

**44.** Substituted indol-Mannic base according to claim 1:

1-methyl-3-(morpholin-4-yl-phenylmethyl)-1H-indole
1-ethyl-2-phenyl-3-(pyrrolidin-1-yl-*o*-tolylmethyl)-1H-indole
3-[(2-chlorophenyl)-piperidin-1-yl-methyl]-1-ethyl-2-phenyl-1H-indole
1-ethyl-2-phenyl-3-(phenylpyrrolidin-1-yl-methyl)-1H-indole
2-phenyl-3-(pyrrolidin-1-yl-*o*-tolylmethyl)-1H-indole
2-phenyl-3-(piperidin-1-yl-*o*-tolylmethyl)-1H-indole
3-[(2-chlorophenyl)-piperidin-1-yl-methyl]-2-phenyl-1H-indole
2-phenyl-3-(phenylpyrrolidin-1-yl-methyl)-1H-indole
4-methyl-3-(piperidin-1-yl-o-tolyl-methyl)-1H-indole
5-benzyloxy-3-(phenylpiperidin-1-yl-methyl)-1H-indole
3-(phenylpiperidin-1-yl-methyl)-1H-indole
1-methyl-3-(phenylpiperidin-1-yl-methyl)-1H-indole
3-(phenylpyrrolidin-1-yl-methyl)-1H-indole
5-benzyloxy-3-(phenylpyrrolidin-1-yl-methyl)-1H-indole

**45.** Process for the preparation of substituted indole Mannich bases of the general formula I according to one of claims 1 to 44, **characterized in that** aromatic aldehyde compounds of the general formula II

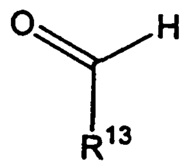

II

wherein $R^{13}$ has the meaning according to the general formula I according to claim 1, are reacted in solution in the presence of a base at a temperature of preferably -10 to 110°C with secondary amines of the general formula III

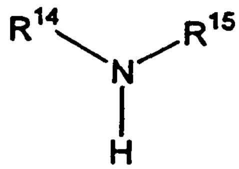

III,

in which the radicals $R^{14}$ and $R^{15}$ have the meaning according to the general formula I according to claim 1, to give aminal compounds of the general formula IV

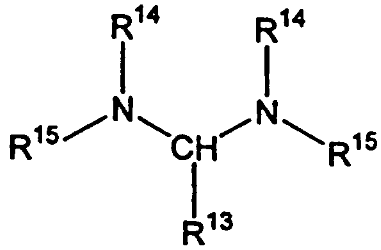

IV

and these aminal compounds of the general formula IV are reacted, without further purification, with an acid chloride in an absolute solvent to give iminium salts of the general formula V

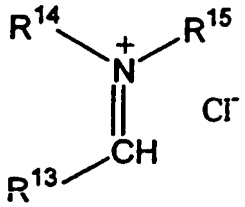

V

and these iminium salts of the general formula V are reacted, without further purification and in solution, preferably in acetonitrile and/or toluene, with indole and/or substituted indole compounds of the general formula VI

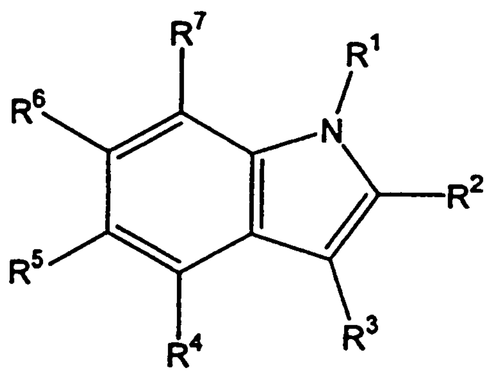

VI,

in which the radical $R^3$ = H and the radicals $R^1$, $R^2$, $R^4$ to $R^{12}$ and $R^{16}$ to $R^{21}$ have the meaning according to the general formula I, and the substituted indole Mannich bases of the general formula I according to one of claims 1 to 44 obtained in this way are purified by extraction and/or washing, preferably by washing with acetone, and are

isolated by conventional methods.

**46.** Process for the preparation of substituted indole Mannich bases of the general formula I according to one of claims 1 to 44, in which the radical $R^1 \neq H$ and the radicals $R^2$ to $R^{21}$ have the meaning according to the general formula I, **characterized in that** aromatic aldehyde compounds of the general formula II

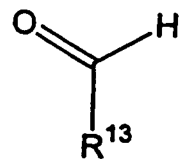

II

wherein $R^{13}$ has the meaning according to the general formula I, are reacted in solution in the presence of a base at a temperature of preferably -10 to +110°C with secondary amines of the general formula III

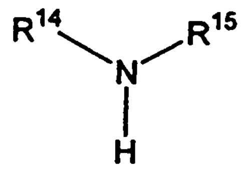

III

in which the radicals $R^{14}$ and $R^{15}$ have the meaning according to the general formula I,
to give aminal compounds of the general formula IV

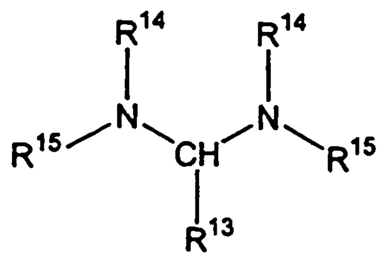

IV,

and these aminal compounds of the general formula IV are reacted, without further purification, with an acid chloride in an absolute solvent to give iminium salts of the general formula V

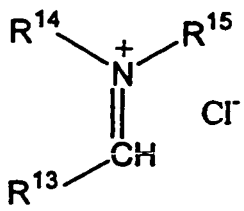

V

and these iminium salts of the general formula V are reacted, without further purification and in solution, preferably in acetonitrile and/or toluene, with indole and/or substituted indole compounds of the general formula VI

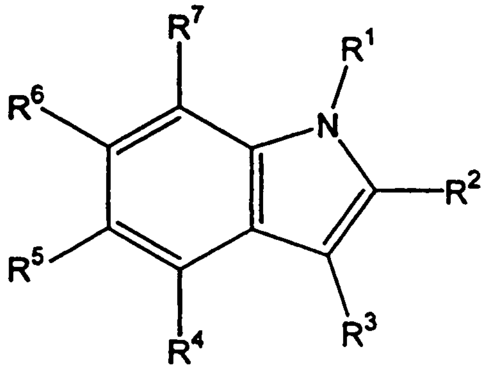

VI

in which the radical $R^1$ and $R^3$ each = H and the radicals $R^2$, $R^4$ to $R^{12}$ and $R^{16}$ to $R^{21}$ have the meaning according to the general formula I, and the compounds of the general formula VI obtained in this way, in which $R^1$ = H and the radicals $R^2$ to $R^{21}$ have the meaning according to the general formula I, are reacted in solution with compounds of the general formula $XR^{22}$, in which $R^{22}$ denotes a $C_{1-10}$-alkyl radical, a phenyl or naphthyl radical bonded via a $C_{1-6}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, or a thiophene, pyrrolyl or furfuryl radical bonded via a $C_{1-6}$-alkylene group, and which is unsubstituted or is substituted with a halogen, CN, $CF_3$, or OH radical, and X = Cl, Br or I, at a temperature of preferably 10 to 150°C in the presence of a base, and the compounds of the general formula I obtained in this way, in which $R^1$ denotes a $C_{1-10}$-alkyl radical, a phenyl or naphthyl radical bonded via a $C_{1-6}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, or a thiophene, pyrrolyl or furfuryl radical bonded via a $C_{1-6}$-alkylene group, and which is unsubstituted or is substituted with a halogen, CN, $CF_3$, or OH radical, and the remaining radicals $R^2$ to $R^{21}$ have the meaning according to the general formula I, are purified by filtration and are isolated by conventional methods.

47. Process according to claim 46, **characterized in that** the reaction with the compounds of the general formula $XR^{22}$ is carried out in dimethyl sulfoxide.

48. Process according to claim 46 or 47, **characterized in that** X = Cl.

49. Process according to one of claims 46 to 48, **characterized in that** the radical $R^{22}$ represents a $C_{1-6}$-alkyl radical, a phenyl or naphthyl radical bonded via a $C_{1-2}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings.

**50.** Process according to one of claims 46 to 49, **characterized in that** the reaction with the compounds of the general formula $XR^{22}$ is carried out in the presence of potassium hydroxide as the base.

**51.** Process according to one of claims 46 to 50, **characterized in that** the compounds of the general formula I are purified by filtration over a scavenger resin, preferably by filtration over polymer-bonded tris(2-aminoethyl)-amine and/or 3-(3-mercaptophenyl)propane-amidomethylpolystyrene.

**52.** Process according to one of claims 45 to 51, **characterized in that** the aromatic aldehyde compounds of the general formula II are reacted in organic solvents, preferably toluene, with secondary amines of the general formula III.

**53.** Process according to one of claims 45 to 52, **characterized in that** the aromatic aldehyde compounds of the general formula II are reacted in the presence of potassium carbonate and/or boric acid anhydride as the base.

**54.** Process according to one of claims 45 to 53, **characterized in that** the aminal compounds of the general formula IV are reacted with acetyl chloride to give iminium salts of the general formula V.

**55.** Process according to one of claims 45 to 54, **characterized in that** the aminal compounds of the general formula IV are reacted in absolute diethyl ether to give iminium salts of the general formula V.

**56.** Medicament containing as active compound at least one substituted indole Mannich base of the general formula I

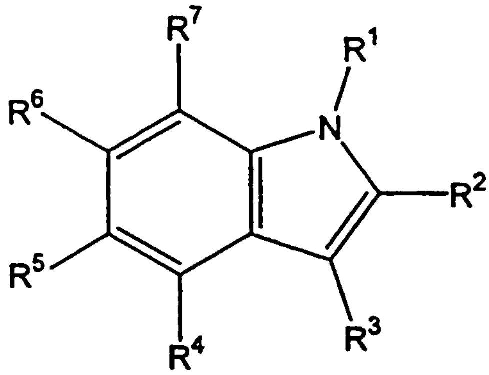

I

wherein

$R^1$ = H, a $C_{1-10}$-alkyl radical, a phenyl or naphthyl radical bonded via a $C_{1-6}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, or a thiophene, pyrrolyl or furfuryl radical bonded via a $C_{1-6}$-alkylene group, and which is unsubstituted or is substituted with a halogen, CN, $CF_3$ or OH radical, and preferably represents H, a $C_{1-6}$-alkyl radical or a phenyl or naphthyl radical bonded via a $C_{1-2}$-alkylene group, and which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, and particularly preferably represents H or a $C_{1-2}$-alkyl radical,

$R^2$ = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, a $C_{1-10}$-alkyl,

a phenyl or naphthyl radical which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted with a halogen, CN, $CF_3$ or OH radical, a phenyl or naphthyl radical bonded via a $C_{1-6}$-alkylene group, which is unsubstituted or is at least monosubstituted with an OH, a halogen, a $CF_3$, a CN, a $C_{1-6}$-alkyl, a $C_{1-6}$-alkoxy or a phenyl radical, which is optionally condensed with further rings, or denotes a thiophene, pyrollyl or furfuryl radical bonded via a $C_{1-6}$-alkylene group, which is unsubstituted or is substituted with a halogen, CN, $CF_3$ or OH radical, and preferably represents H, Cl, F, $NO_2$, $OR^{10}$, $CO(OR^{11})$, a $C_{1-6}$-alkyl radical or a phenyl or naphthyl radical bonded via a $C_{1-2}$-alkylene group, which is unsubstituted or at least

monosubstituted with an OH, a halogen, a $CF_3$, a CN, a $C_{1-6}$-alkyl, $C_{1-6}$-alkoxy or a phenyl radical, which is optionally condensed with further rings, and particularly preferably represents H, a $C_{1-2}$-alkyl radical or an unsubstituted phenyl radical,

$R^3 = CH(R^{13})N(R^{14})(R^{15})$,

$R^4$, $R^5$, $R^6$, $R^7$ are identical or different and = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, a $C_{1-10}$-alkyl, a phenyl or naphthyl radical which is unsubstituted or at least monosubstituted by an OH, a halogen, a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted by a halogen, CN, $CF_3$, or OH radical, a phenyl or naphthyl radical bonded via a $C_{1-6}$-alkylene group, which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings, a thiophene, purrolyl or furforyl radical bonded via a $C_{1-6}$-alkylene group, which is unsubstituted or substituted with a halogen, CN, $CF_3$, or an OH radical, preferably represents H, Cl, F, $NO_2$, $OR^{10}$, $CO(OR^{11})$, a phenyl or naphthyl radical, which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, a $C_{1-6}$-alkyl radical or a phenyl or naphthyl radical bonded via a $C_{1-2}$-alkylene group, which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, and particularly preferably represents H, $NO_2$, a $C_{1-2}$-alkyl or an unsubstituted phenyl radical,

$R^8$ = H, $COR^{16}$ or a $C_{1-10}$-alkyl radical, a phenyl or naphthyl radical which is unsubstituted or at least monosubstituted with an OH, halogen a $CF_3$, a CN, a $C_{1-6}$-alkyl, a $C_{1-6}$-alkoxy or a phenyl radical, which is optionally condensed with further rings, preferably represents a $C_{1-6}$-alkyl radical,

$R^9$ = H or a $C_{1-10}$-alkyl radical, a phenyl or naphthyl radical which is unsubstituted or at least monosubstituted with an OH, halogen a $CF_3$, a CN, a $C_{1-6}$-alkyl, a $C_{1-6}$-alkoxy or a phenyl radical, which is optionally condensed with further rings, preferably represents a $C_{1-6}$-alkyl radical or a phenyl radical, $R^{10}$= H, $COR^{17}$, a $C_{1-10}$-alkyl radical, a phenyl or naphthyl radical which is unsubstituted or at least monosubstituted with an OH, a halogen a $CF_3$, a CN, a $C_{1-6}$-alkyl, a $C_{1-6}$-alkoxy or a phenyl radical, which is optionally condensed with further rings, a phenyl or naphthyl radical bonded via a $C_{1-6}$-alkylene group, which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, or a thiophene, pyrroryl or furfuryl radical bonded via a $C_{1-6}$-alkylene group, which is unsubstituted or substituted by a halogen, CN, $CF_3$, or OH radical and preferably represents H, a $C_{1-6}$-alkyl radical or a phenyl or naphthyl radical bonded via a $C_{1-2}$-alkylene group, which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings,

$R^{11}$ =H or a $C_{1-10}$-alkyl or $C_{1-10}$-alkyl radical or a phenyl or naphthyl radical which is unsubstituted or is at least monosubstituted with an OH, halogen, a $CF_3$, a CN, a $C_{1-6}$-alkyl, a $C_{1-6}$-alkoxy or a phenyl radical, and which is optionally condensed with further rings, and preferably represents H or a $C_{1-6}$-alkyl radical,

$R^{12}$ = H, a $C_{1-10}$-alkyl radical or $C_{1-10}$-alkyl radical or a phenyl or naphthyl radical which is unsubstituted or is at least monosubstituted with an OH, halogen, a $CF_3$, a CN, a $C_{1-6}$-alkyl, a $C_{1-6}$-alkoxy or a phenyl radical, and which is optionally condensed with further rings, preferably represents a $C_{1-6}$-alkyl radical,

$R^{13}$ = a phenyl or naphthyl radical which is unsubstituted or is at least monosubstituted with an OH, halogen, a $CF_3$, CN, a $C_{1-6}$-alkyl, a $C_{1-6}$-alkoxy, an O-phenyl or a phenyl radical, which is optionally condensed with further rings, a thiophene, pyrrolyl or furfuryl radical which is unsubstituted or is substituted with a halogen, CN, $CF_3$ or OH radical, or represents an alkyl radical without acidic proton in the $\alpha$-position, preferably an unsubstituted phenyl radical or a phenyl, thiophene or furfuryl radical that is at least monosubstituted with $C_{1-4}$-alkyl, $C_{1-3}$-alkoxy, halogen, $CF_3$, CN, O-phenyl or OH, and particularly preferably represents an unsubstituted phenyl, thiophene or furfuryl radical or a 2-methoxy-phenyl, 3-methoxy-phenyl, 4-methoxy-phenyl, 2-methyl-phenyl, 3-methyl-phenyl, 4-methyl-phenyl, 2-tert-butyl-phenyl, 3-tert-butyl-phenyl, 4-tert-butyl-phenyl, 2-fluoro-phenyl, 3-fluoro-phenyl, 4-fluoro-phenyl, 2-chloro-phenyl, 3-chloro-phenyl, 4-chloro-phenyl, 2-bromo-phenyl, 3-bromo-phenyl, 4-bromo-phenyl, 5-bromo-2-fluoro-phenyl, 2-chloro-4-fluoro-phenyl, 2-chloro-5-fluoro-phenyl, 2-chloro-6-fluoro-phenyl, 4-bromo-2-fluoro-phenyl, 3-bromo-4-fluoro-phenyl, 3-bromo-2-fluoro-phenyl, 2,3-dichloro-phenyl, 2,4-dichloro-phenyl, 2,5-dichlorophenyl, 3,4-dichloro-phenyl, 2,3-dimethyl-phenyl, 2,4-dimethyl-phenyl, 2,5-dimethylphenyl, 2,3-dimethoxy-phenyl, 2,4-dimethoxy-phenyl, 2,5-dimethoxy-phenyl, 3,4-dimethoxy-phenyl, 3,4,5-trimethoxy-phenyl, 2-trifluoromethyl-phenyl, 3-trifluoromethyl-phenyl or a 4-trifluoromethyl-phenyl radical, particularly preferably an unsubstituted phenyl, thiophene or furfuryl radical,

$R^{14}$, $R^{15}$ are identical or different and denote a branched or unbranched, saturated or unsaturated, unsubstituted or at least monosubstituted $C_{1-6}$-alkyl radical or an unsubstituted or at least monosubstituted phenyl, benzyl or phenethyl radical, and preferably denote a saturated, unsubstituted or at least monosubstituted $C_{1-6}$-alkyl radical, particularly preferably a $CH_3$ radical,

or $R^{14}$ and $R^{15}$ together denote $(CH_2)_n$, where n = an integer from 3 to 6, or $(CH_2)_2O(CH_2)_2$, preferably $(CH_2)_n$,

where n = 4 or 5,
$R^{16}$ = a $C_{1-10}$-alkyl radical, a phenyl or naphthyl radical which is unsubstituted or is at least monosubstituted with an OH, halogen, a $CF_3$, a CN, a $C_{1-6}$-alkyl, a $C_{1-6}$-alkoxy or a phenyl radical, which is optionally condensed with further rings, or denotes a thiophene, pyrrolyl or furfuryl radical that is unsubstituted or is substituted with a halogen, CN, $CF_3$ or OH radical, and preferably denotes a $C_{1-6}$-alkyl radical,
$R^{17}$ = a $C_{1-10}$-alkyl radical, preferably a $C_{1-6}$-alkyl radical,
$R^{18}$ = a $C_{1-10}$-alkyl radical, a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, or a thiophene, pyrrolyl or furfuryl radical which is unsubstituted or is substituted with a halogen, CN, $CF_3$ or OH radical, preferably a $C_{1-6}$-alkyl radical,
$R^{19}$ = H, $NHNH_2$, $NHR^{20}$, a $C_{1-10}$-alkyl, a phenyl or naphthyl radical which is unsubstituted or at least monosubstituted with an OH, halogen a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted with a halogen, CN, $CF_3$, or OH radical, a phenyl or naphthyl radical bonded via a $C_{1-6}$-alkylene group, which is unsubstituted or is at least monosubstituted with an OH, halogen, a $CF_3$, a CN, a $C_{1-6}$-alkyl, a $C_{1-6}$-alkoxy or a phenyl radical, which is optionally condensed with further rings, or denotes a thiophene, pyrollyl or furfuryl radical bonded via a $C_{1-6}$-alkylene group, which is unsubstituted or is substituted with a halogen, CN, $CF_3$, or OH radical, preferably a $C_{1-6}$-alkyl radical or a phenyl or naphthyl radical bonded via a $C_{1-2}$-alkylene group, and which is unsubstituted or is at least monosubstituted with an OH, a halogen, a $CF_3$, a CN, a $C_{1-6}$-alkyl, a $C_{1-6}$-alkoxy or a phenyl radical, which is optionally condensed with further rings,
$R^{20}$ = H, a $C_{1-10}$-alkyl radical, a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted with a halogen, CN, $CF_3$ or OH radical, a phenyl or naphthyl radical bonded via a $C_{1-6}$-alkylene group and which is unsubstituted or at least monosubstituted with an OH, a halogen a $CF_3$, a CN, a $C_{1-6}$-alkyl, a $C_{1-6}$-alkoxy or a phenyl radical, which is optionally condensed with further rings, or a thiophene, pyrrolyl or furfuryl radical bonded via a $C_{1-6}$-alkylene group and which is unsubstituted or is substituted by a halogen, CN, $CF_3$, or OH radical, preferably denotes a $C_{1-6}$-alkyl radical or a phenyl or naphthyl radical bonded via a $C_{1-2}$-alkylene group and which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings,
$R^{21}$ = H, $COR^{17}$, a $C_{1-10}$-alkyl radical, a phenyl or naphthyl radical that is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, a phenyl or naphthyl radical bonded via a $C_{1-6}$-alkylene group, and which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, or denotes a thiophene, pyrollyl or furfuryl radical bonded via a $C_{1-6}$-alkylene group, and which is unsubstituted or is substituted with a halogen, CN, $CF_3$ or OH radical, preferably denotes H, a $C_{1-6}$-alkyl radical or a phenyl or naphthyl radical bonded via a $C_{1-2}$-alkylene group and which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings,
and/or their racemates, enantiomers, diastereomers and/or corresponding bases and/or corresponding salts of physiologically tolerated acids, and optionally further active substances and/or auxiliary substances.

**57.** Medicament according to claim 56 containing at least one substituted indole Mannich base selected from the group consisting of

3-(dimethylaminophenylmethyl)-1H-indole-2-carboxylic acid ethyl ester
3-(dimethylaminophenylmethyl)-1H-indole-2-carboxylic acid
[(5-fluoro-1H-indol-3-yl)-phenylmethyl]-dimethylamine
[(1-ethyl-2-phenyl-1H-indol-3-yl)-phenylmethyl]-dimethylamine
[(5-methoxy-1H-indol-3-yl)-phenylmethyl]-dimethylamine
3-(dimethylaminophenylmethyl)-5-hydroxy-1H-indole-2-carboxylic acid
dimethyl-[(2-methyl-1H-indol-3-yl)-phenylmethyl]-amine
3-(dimethylaminophenylmethyl)-1H-indol-4-0l
dimethyl-[(4-methyl-1H-indol-3-yl)-phenylmethyl]-amine
[(5-chloro-1H-indol-3-yl)-phenylmethyl]-dimethylamine
[(5-benzyloxy-1H-indol-3-yl)-phenylmethyl]-dimethylamine
acetic acid 3-(dimethylaminophenylmethyl)-1H-indol-4-yl ester
{[2-(4-chlorophenyl)-1H-indol-3-yl]-phenylmethyl}-dimethylamine

5-benzyloxy-3-(dimethylaminophenylmethyl)-1H-indole-2-carboxylic acid
dimethyl-[(2-methyl-5-nitro-1H-indol-3-yl)-phenylmethyl]-amine
dimethyl-[(2-methyl-6-nitro-1H-indol-3-yl)-phenylmethyl]-amine
[(6-fluoro-2-methyl-1H-indol-3-yl)-phenylmethyl]-dimethylamine
{[2-(4-fluorophenyl)-1H-indol-3-yl]-phenylmethyl}-dimethylamine
{[2-(3-chloro-4-fluorophenyl)-1H-indol-3-yl]-phenylmethyl}-dimethylamine
[(7-ethyl-1H-indol-3-yl)-phenylmethyl]-dimethylamine
3-(dimethylaminophenylmethyl)-1H-indole-6-carboxylic acid
dimethyl-[(1-methyl-1H-indol-3-yl)-phenylmethyl]-amine
dimethyl-[(2-phenyl-1H-indol-3-yl)-o-tolyl-methyl]-amine
[5-benzyloxy-1H-indol-3-yl)-o-tolylmethyl]-dimethylamine
[(2-methoxyphenyl)-(2-methyl-1H-indol-3-yl)-methyl]-dimethylamine
{[1-(4-methoxybenzyl)-1H-indol-3-yl]-phenyl-methyl}-dimethylamine
[(1H-indol-3-yl)-phenylmethyl]-dimethylamine
[(5-bromo-2-fluoro-phenyl)-(4-nitro-1H-indol-3-yl)-methyl]-dimethyl-amine
[(5-bromo-2-fluoro-phenyl)-(1-methyl-1H-indol-3-yl)-methyl]-dimethyl-amine
[(2-chloro-6-fluoro-phenyl)-(2-phenyl-1H-indol-3-yl)-methyl]-dimethylamine
[(2-chloro-6-fluorophenyl)-(1-ethyl-2-phenyl-1H-indol-3-yl)-methyl]-dimethylamine
[(2-chloro-6-fluorophenyl)-(4-nitro-1H-indol-3-yl)-methyl]-dimethylamine
[(2-chloro-6-fluorophenyl)-(1-methyl-1H-indol-3-yl)-methyl]-dimethylamine
[(2-bromophenyl)-(4-nitro-1H-indol-3-yl)-methyl]-dimethylamine
[(2-bromophenyl)-(1-methyl-1H-indol-3-yl)-methyl]-dimethylamine
[(3-bromophenyl)-(7-ethyl-1H-indol-3-yl)-methyl]-dimethylamine
[(3-bromophenyl)-(1-methyl-1H-indol-3-yl)-methyl]-dimethylamine
[(4-tert-butylphenyl)-(2-methyl-1H-indol-3-yl)-methyl]-dimethylamine
[(4-tert-butylphenyl)-(1-ethyl-2-phenyl-1H-indol-3-yl)-methyl]-dimethylamine
[(4-tert-butylphenyl)-(1-methyl-1H-indol-3-yl)-methyl]-dimethylamine
[(2-chloro-4-fluorophenyl)-(4-nitro-1H-indol-3-yl)-methyl]-dimethylamine
[(2-chloro-4-fluorophenyl)-(4-methyl-1H-indol-3-yl)-methyl]-dimethylamine
[(2-chloro-6-fluorophenyl)-(2-phenyl-1H-indol-3-yl)-methyl]-dimethylamine
{(2-chloro-6-fluoro-phenyl)-[2-(4-chlorophenyl)-1H-indol-3-yl]-methyl}-dimethylamine
[(2-chloro-6-fluorophenyl)-(1-methyl-1H-indol-3-yl)-methyl]-dimethylamine
[(3-chlorophenyl)-(1-methyl-1H-indol-3-yl)-methyl]-dimethylamine
[(2,3-dichloro-phenyl)-(1-methyl-1H-indol-3-yl)-methyl]-dimethyl-amine
[(2,4-dichlorophenyl)-(1-methyl-1H-indol-3-yl)-methyl]-dimethylamine.
[(4-tert-butylphenyl)-(1-ethyl-2-phenyl-1H-indol-3-yl)-methyl]-dimethylamine
[(2-chloro-4-fluorophenyl)-(2-methyl-1H-indol-3-yl)-methyl]-dimethylamine
[(2-chloro-6-fluorophenyl)-(2-methyl-1H-indol-3-yl)-methyl]-dimethylamine
[(2,3-dimethoxyphenyl)-(4-nitro-1H-indol-3yl)-methyl]-dimethylamine
3-[(2,3-dimethoxyphenyl)-dimethylaminomethyl]-4,7-dihydro-1H-indol-6-carboxylic acid
3-[2,3-dimethoxyphenyl)-dimethylaminomethyl]-5-hydroxy-1H-indol-2-carboxylic acid
[(3,4-dimethoxyphenyl)-(2-methyl-1H-indol-3-yl)-methyl]-dimethylamine
[[2-(4-chlorophenyl)-1H-indol-3-yl]-(3,4-dimethoxyphenyl)methyl]-dimethylamine
[(3,4-dimethoxyphenyl)-(1-ethyl-2-phenyl-1H-indol-3-yl)-methyl]-dimethylamine
[(3,4-dimethoxyphenyl)-(7-ethyl-1H-indol-3-yl)-methyl]-dimethylamine
[(3,4-dimethoxyphenyl)-(1-methyl-1H-indol-3-yl)-methyl]-dimethylamine
[(3,4-dimethoxyphenyl)-[2-(4-fluorophenyl)-1H-indol-3-yl]-methyl}-dimethylamine
[[2-(3-chloro-4-fluorophenyl)-1H-indol-3-yl]-(3,4-dimethoxyphenyl)-methyl]-dimethylamine
[(5-chloro-1H-indol-3-yl)-(2-fluorophenyl)-methyl]-dimethylamine
[(4-fluorophenyl)-(4-methyl-1H-indol-3-yl)-methyl]-dimethylamine
[(7-ethyl-1H-indol-3-yl)-(2-methoxyphenyl)-methyl]-dimethylamine
dimethyl-[(2-methyl-1H-indol-3-yl)-o-tolylmethyl]-amine
[(7-ethyl-1H-indol-3-yl)-o-tolylmethyl]-dimethyl-amine
dimethyl-[(1-methyl-1H-indol-3-yl)-o-tolylmethyl]-amine
[(5-chloro-1H-indol-3-yl)-o-tolylmethyl]-dimethylamine
[(5-chloro-1H-indol-3-yl)-m-tolylmethyl]-dimethylamine
dimethyl-[(2-methyl-1H-indol-3-yl)-p-tolylmethyl]-amine
[(5-chloro-1H-indol-3-yl)-p-tolylmethyl]-dimethylamine

dimethyl-[(2-methyl-1H-indol-3-yl)-(3-phenoxyphenyl)-methyl]-amine
[(1-ethyl-2-phenyl-1H-indol-3-yl)-(3-phenoxyphenyl)-methyl]-dimethylamine
[(7-ethyl-1H-indol-3-yl)-(3-phenoxyphenyl)-methyl]-dimethylamine
dimethyl-[(1-methyl-1H-indol-3-yl)-(3-phenoxyphenyl)-methyl]-amine
[[2-(4-fluorophenyl)-1H-indol-3-yl]-(3-phenoxyphenyl)-methyl]-dimethylamine
[[2-(3-chloro-4-fluorophenyl)-1H-indol-3-yl]-(3-phenoxyphenyl)-methyl]-dimethylamine
dimethyl-[(4-methyl-1H-indol-3-yl)-(3-phenoxyphenyl)-methyl]-amine
[(5-chloro-1H-indol-3-yl)-(2-trifluomethyl-phenyl)-methyl]-dimethylamine
dimethyl-[(2-methyl-1H-indol-3-yl)-(4-trifluoromethyl-phenyl)-methyl]-amine.

**58.** Medicament according to claim 56, containing at least one substituted indole Mannic base selected from the group consisting of

1-methyl-3-(morpholin-4-yl-phenylmethyl)-1H-indole
3-[(2-methoxyphenyl)-pyrrolidin-1-yl-methyl]-1-methyl-1H-indole
1-ethyl-2-phenyl-3-(pyrrolidin-1-yl-*o*-tolylmethyl)-1H-indole
3-[(2-chlorophenyl)-piperidin-1-yl-methyl]-1-ethyl-2-phenyl-1H-indol
1-ethyl-2-phenyl-3-(phenylpyrrolidin-1-yl-methyl)-1H-indole
1-ethyl-3-[(2-methoxyphenyl)-pyrrolidin-1-yl-methyl]-2-phenyl-1H-indole
2-phenyl-3-(pyrrolidin-1-yl-o-tolylmethyl)-1H-indole
2-phenyl-3-(piperidin-1-yl-o-tolylmethyl)-1H-indole
3-[(2-chlorophenyl)-piperidin-1-yl-methyl]-2-phenyl-1H-indole
2-phenyl-3-(phenylpyrrolidin-1-yl-methyl)-1H-indole
3-[(2-methoxy-phenyl)-pyrrolidin-1-yl-methyl] -2-phenyl-1H-indol
4-methyl-3-(piperidin-1-yl-o-tolyl-methyl)-1H-indole
3-[(2-methoxyphenyl) -pyrrolidin-1-yl-methyl]-4-methyl-1H-indole
5-benzyloxy-3-(phenylpiperidin-1-yl-methyl)-1H-indole
7-ethyl-3-[(2-methoxyphenyl)-morpholin-4-yl-methyl]-1H-indole
3-[(2-methoxyphenyl)-piperidin-1-yl-methyl]-2-phenyl-1H-indole
5-chloro-3-[(2-methoxyphenyl)-piperidin-1-yl-methyl]-1H-indole
5-chloro-3-[(2-methoxyphenyl)-pyrrolidin-1-yl-methyl]-1H-indole
5-benzyloxy-3-[(2 methoxyphenyl)-pyrrolidin-1-ylmethyl]-1H-indole
3-(phenylpiperidin-1-yl-methyl)-1H-indole
1-methyl-3-(phenylpiperidin-1-yl-methyl)-1H-indole
3-(phenylpyrrolidin-1-yl-methyl)-1H-indole
5-benzyloxy-3-(phenylpyrrolidin-1-yl-methyl)-1H-indole

**59.** Medicament according to one of claims 56 to 58, **characterised in that** it contains as active constituent a mixture of enantiomers of at least one substituted indole Mannich base of the general formula I of claims 56 to 58, in which the mixture comprises the enantiomers in non-equimolar amounts.

**60.** Medicament according to claim 59, **characterized in that** the relative proportion of one of the enantiomers of the mixture is 5 to 45 mol%, preferably 10 to 40 mol%, based on the mixture of enantiomers.

**61.** Medicament according to one of claims 56 to 60 for treatment of/combating pain, preferably chronic pain, and/or inflammatory reactions and/or allergic reactions and/or drug abuse and/or alcohol abuse and/or diarrhoea and/or gastritis and/or ulcers and/or cardiovascular diseases and/or urinary incontinence and/or depression and/or states of shock and/or migraines and/or narcolepsy and/or excess weight and/or asthma and/or glaucoma and/or hyperkinetic syndrome and/or lack of drive and/or bulimia and/or anorexia and/or catalepsy and/or for anxiolysis and/or for increasing vigilance and/or for increasing libido.

**62.** Use of at least one compound of the general formula I

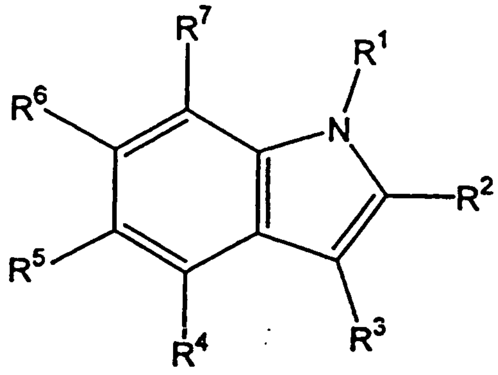

I

wherein

$R^1$ = H, a $C_{1-10}$-alkyl radical, a phenyl or naphthyl radical bonded via a $C_{1-6}$ alkylene group, and which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, or a thiophene, pyrrolyl or furfuryl radical bonded via a $C_{1-6}$-alkylene group, and which is unsubstituted or is substituted with a halogen, CN, $CF_3$ or OH radical, and preferably represents H, a $C_{1-6}$-alkyl radical or a phenyl or naphthyl radical bonded via a $C_{1-2}$-alkylene group, and which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, and particularly preferably represents H or a $C_{1-2}$-alkyl radical,

$R^2$ = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{19}$, a $C_{1-10}$-alkyl, a phenyl or naphthyl radical which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted with a halogen, CN, $CF_3$ or OH radical, a phenyl or naphthyl radical bonded via a $C_{1-6}$-alkylene group, which is unsubstituted or is at least monosubstituted with an OH, a halogen, a $CF_3$, a CN, a $C_{1-6}$-alkyl, a $C_{1-6}$-alkoxy or a phenyl radical, which is optionally condensed with further rings, or denotes a thiophene, pyrollyl or furfuryl radical bonded via a $C_{1-6}$-alkylene group, which is unsubstituted or is substituted with a halogen, CN, $CF_3$ or OH radical, and preferably represents H, Cl, F, $NO_2$, $OR^{10}$, CO $(OR^{11})$, a $C_{1-6}$-alkyl radical or a phenyl or naphthyl radical bonded via a $C_{1-2}$-alkylene group, which is unsubstituted or at least monosubstituted with an OH, a halogen, a $CF_3$, a CN, a $C_{1-6}$-alkyl, $C_{1-6}$-alkoxy or a phenyl radical, which is optionally condensed with further rings, and particularly preferably represents H, a $C_{1-2}$-alkyl radical or an unsubstituted phenyl radical,

$R^3$ = $CH(R^{13})N(R^{14})$ $(R^{15})$,

$R^4$, $R^5$, $R^6$, $R^7$ are identical or different and = H, F, Cl, Br, $CF_3$, CN, $NO_2$, $NHR^8$ , $SR^9$, $OR^{10}$, $SO_2NH_2$, $SO_2NHR^{21}$, $CO(OR^{11})$, $CH_2CO(OR^{12})$, $COR^{19}$, a $C_{1-10}$-alkyl, a phenyl or naphthyl radical which is unsubstituted or at least monosubstituted by an OH, a halogen, a $CF_3$, a CN, a $C_1$-6-alkyl, a $C_{1-6}$-alkoxy or a phenyl radical, which is optionally condensed with further rings, a thiophene, pyrrolyl or furfuryl radical which is unsubstituted or is substituted by a halogen, CN, $CF_3$, or OH radical, a phenyl or naphthyl radical bonded via a $C_{1-6}$-alkylene group, which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings, a thiophene, purrolyl or furforyl radical bonded via a $C_{1-6}$-alkylene group, which is unsubstituted or substituted with a halogen, CN, $CF_3$, or an OH radical, preferably represents H, Cl, F, $NO_2$, $OR^{10}$, $CO(OR^{11})$, a phenyl or naphthyl radical, which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, a $C_{1-6}$-alkyl radical or a phenyl or naphthyl radical bonded via a $C_{1-2}$-alkylene group, which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, and particularly preferably represents H, $NO_2$, a $C_{1-2}$-alkyl or an unsubstituted phenyl radical,

$R^8$ = H, $COR^{16}$ or a $C_{1-10}$-alkyl radical, a phenyl or naphthyl radical which is unsubstituted or at least monosubstituted with an OH, halogen a $CF_3$, a CN, a $C_{1-6}$-alkyl, a $C_{1-6}$-alkoxy or a phenyl radical, which is optionally condensed with further rings, preferably represents a $C_{1-6}$-alkyl radical,

$R^9$ = H or a $C_{1-10}$-alkyl radical, a phenyl or naphthyl radical which is unsubstituted or at least monosubstituted with an OH, halogen a $CF_3$, a CN, a $C_{1-6}$-alkyl, a $C_{1-6}$-alkoxy or a phenyl radical, which is optionally condensed with further rings, preferably represents a $C_{1-6}$-alkyl radical or a phenyl radical,

$R^{10}$ = H, $COR^{17}$, a $C_{1-10}$-alkyl radical, a phenyl or naphthyl radical which is unsubstituted or at least monosubstituted

with an OH, a halogen a $CF_3$, a CN, a $C_{1-6}$-alkyl, a $C_{1-6}$-alkoxy or a phenyl radical, which is optionally condensed with further rings, a phenyl or naphthyl radical bonded via a $C_{1-6}$-alkylene group, which is unsubstituted or is at least monosubstituted by an OH, a halogen, a $CF_3$, a CN, a $C_{1-6}$-alkyl, a $C_{1-6}$-alkoxy or a phenyl radical, which is optionally condensed with further rings, or a thiophene, pyrroryl or furfuryl radical bonded via a $C_{1-6}$-alkylene group, which is unsubstituted or substituted by a halogen, CN, $CF_3$, or OH radical and preferably represents H, a $C_{1-6}$-alkyl radical or a phenyl or naphthyl radical bonded via a $C_{1-2}$-alkylene group, which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings,

$R^{11}$ = H or a $C_{1-10}$-alkyl or $C_{1-10}$-alkyl radical or a phenyl or naphthyl radical which is unsubstituted or is at least monosubstituted with an OH, halogen, a $CF_3$, a CN, a $C_{1-6}$-alkyl, a $C_{1-6}$-alkoxy or a phenyl radical, and which is optionally condensed with further rings, and preferably represents H or a $C_{1-6}$-alkyl radical,

$R^{12}$ = H, a $C_{1-10}$-alkyl radical or $C_{1-10}$-alkyl radical or a phenyl or naphthyl radical which is unsubstituted or is at least monosubstituted with an OH, halogen, a $CF_3$, a CN, a $C_{1-6}$-alkyl, a $C_{1-6}$-alkoxy or a phenyl radical, and which is optionally condensed with further rings, preferably represents a $C_{1-6}$-alkyl radical,

$R^{13}$ = a phenyl or naphthyl radical which is unsubstituted or is at least monosubstituted with an OH, halogen, a $CF_3$, CN, a $C_{1-6}$-alkyl, a $C_{1-6}$-alkoxy, an O-phenyl or a phenyl radical, which is optionally condensed with further rings, a thiophene, pyrrolyl or furfuryl radical which is unsubstituted or is substituted with a halogen, CN, $CF_3$ or OH radical, or represents an alkyl radical without acidic proton in the $\alpha$-position, preferably an unsubstituted phenyl radical or a phenyl, thiophene or furfuryl radical that is at least monosubstituted with $C_{1-4}$-alkyl, $C_{1-3}$-alkoxy, halogen, $CF_3$, CN, O-phenyl or OH, and particularly preferably represents an unsubstituted phenyl, thiophene or furfuryl radical or a 2-methoxy-phenyl, 3-methoxy-phenyl, 4-methoxy-phenyl, 2-methyl-phenyl, 3-methyl-phenyl, 4-methyl-phenyl, 2-tert-butyl-phenyl, 3-tert-butyl-phenyl, 4-tert-butyl-phenyl, 2-fluoro-phenyl, 3-fluoro-phenyl, 4-fluoro-phenyl, 2-chloro-phenyl, 3-chloro-phenyl, 4-chloro-phenyl, 2-bromo-phenyl, 3-bromo-phenyl, 4-bromo-phenyl, 5-bromo-2-fluoro-phenyl, 2-chloro-4-fluoro-phenyl, 2-chloro-5-fluoro-phenyl, 2-chloro-6-fluoro-phenyl, 4-bromo-2-fluoro-phenyl, 3-bromo-4-fluoro-phenyl, 3-bromo-2-fluoro-phenyl, 2,3-dichloro-phenyl, 2,4-dichloro-phenyl, 2,5-dichlorophenyl, 3,4-dichloro-phenyl, 2,3-dimethyl-phenyl, 2,4-dimethyl-phenyl, 2,5-dimethylphenyl, 2,3-dimethoxy-phenyl, 2,4-dimethoxy-phenyl, 2,5-dimethoxy-phenyl, 3,4-dimethoxy-phenyl, 3,4,5-trimethoxy-phenyl, 2-trifluoromethyl-phenyl, 3-trifluoromethyl-phenyl or a 4-trifluoromethyl-phenyl radical, particularly preferably an unsubstituted phenyl, thiophene or furfuryl radical,

$R^{14}$, $R^{15}$ are identical or different and denote a branched or unbranched, saturated or unsaturated, unsubstituted or at least monosubstituted $C_{1-6}$-alkyl radical or an unsubstituted or at least monosubstituted phenyl, benzyl or phenethyl radical, and preferably denote a saturated, unsubstituted or at least monosubstituted $C_{1-6}$-alkyl radical, particularly preferably a $CH_3$ radical,

or $R^{14}$ and $R^{15}$ together denote $(CH_2)_n$, where n = an integer from 3 to 6, or $(CH_2)_2O(CH_2)_2$, preferably $(CH_2)_n$, where n = 4 or 5,

$R^{16}$ = a $C_{1-10}$-alkyl radical, a phenyl or naphthyl radical which is unsubstituted or is at least monosubstituted with an OH, halogen, a $CF_3$, a CN, a $C_{1-6}$-alkyl, a $C_{1-6}$-alkoxy or a phenyl radical, which is optionally condensed with further rings, or denotes a thiophene, pyrrolyl or furfuryl radical that is unsubstituted or is substituted with a halogen, CN, $CF_3$ or OH radical, and preferably denotes a $C_{1-6}$-alkyl radical,

$R^{17}$ = a $C_{1-10}$-alkyl radical, preferably a $C_{1-6}$-alkyl radical,

$R^{18}$ = a $C_{1-10}$-alkyl radical, a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, or a thiophene, pyrrolyl or furfuryl radical which is unsubstituted or is substituted with a halogen, CN, $CF_3$ or OH radical, preferably a $C_{1-6}$-alkyl radical,

$R^{19}$ = H, $NHNH_2$, $NHR^{20}$, a $C_{1-10}$-alkyl, a phenyl or naphthyl radical which is unsubstituted or at least monosubstituted with an OH, halogen a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted with a halogen, CN, $CF_3$, or OH radical, a phenyl or naphthyl radical bonded via a $C_{1-6}$-alkylene group, which is unsubstituted or is at least monosubstituted with an OH, halogen, a $CF_3$, a CN, a $C_{1-6}$-alkyl, a $C_{1-6}$-alkoxy or a phenyl radical, which is optionally condensed with further rings, or denotes a thiophene, pyrollyl or furfuryl radical bonded via a $C_{1-6}$-alkylene group, which is unsubstituted or is substituted with a halogen, CN, $CF_3$, or OH radical, preferably a $C_{1-6}$-alkyl radical or a phenyl or naphthyl radical bonded via a $C_{1-2}$-alkylene group, and which is unsubstituted or is at least monosubstituted with an OH, a halogen, a $CF_3$, a CN, a $C_{1-6}$-alkyl, a $C_{1-6}$-alkoxy or a phenyl radical, which is optionally condensed with further rings,

$R^{20}$ = H, a $C_{1-10}$-alkyl radical, a phenyl or naphthyl radical which is unsubstituted or is at least monosubstituted with an OH, a halogen, a $CF_3$, a CN, a $C_{1-6}$-alkyl, a $C_{1-6}$-alkoxy or a phenyl radical, which is optionally condensed with further rings, a thiophene, pyrrolyl or furfuryl radical which is unsubstituted or is substituted with a halogen, CN, $CF_3$ or OH radical, a phenyl or naphthyl radical bonded via a $C_{1-6}$-alkylene group and which is unsubstituted or at least

monosubstituted with an OH, a halogen a $CF_3$, a CN, a $C_{1-6}$-alkyl, a $C_{1-6}$-alkoxy or a phenyl radical, which is optionally condensed with further rings, or a thiophene, pyrrolyl or furfuryl radical bonded via a $C_{1-6}$-alkylene group and which is unsubstituted or is substituted by a halogen, CN, $CF_3$, or OH radical, preferably denotes a $C_{1-6}$-alkyl radical or a phenyl or naphthyl radical bonded via a $C_{1-2}$-alkylene group and which is unsubstituted or at least monosubstituted with an OH, a halogen, a $CF_3$, a CN, a $C_{1-6}$-alkyl, a $C_{1-6}$-alkoxy or a phenyl radical, which is optionally condensed with further rings,

$R^{21}$ = H, $COR^{17}$, a $C_{1-10}$-alkyl radical, a phenyl or naphthyl radical that is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, a phenyl or naphthyl radical bonded via a $C_{1-6}$-alkylene group, and which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings, or denotes a thiophene, pyrollyl or furfuryl radical bonded via a $C_{1-6}$-alkylene group, and which is unsubstituted or is substituted with a halogen, CN, $CF_3$ or OH radical, preferably denotes H, a $C_{1-6}$-alkyl radical or a phenyl or naphthyl radical bonded via a $C_{1-2}$-alkylene group and which is unsubstituted or is at least monosubstituted with an OH, a halogen, a $CF_3$, a CN, a $C_{1-6}$-alkyl, a $C_{1-6}$-alkoxy or a phenyl radical, and which is optionally condensed with further rings,

and/or their racemates, enantiomers, diastereomers and/or corresponding bases and/or corresponding salts of physiologically compatible acids,

for the production of a medicament for treating pain, chronic pain, and/or inflammatory reactions and/or allergic reactions and/or drug abuse and/or alcohol abuse and/or diarrhoea and/or gastritis and/or ulcers and/or cardiovascular diseases and/or urinary incontinence and/or depression and/or states of shock and/or migraines and/or narcolepsy and/or excess weight and/or asthma and/or glaucoma and/or hyperkinetic syndrome and/or lack of drive and/or bulimia and/or anorexia and/or catalepsy and/or for anxiolysis and/or for increasing vigilance and/or for increasing libido.

**63.** Use according to claim 62, **characterised in that** the substituted indole Mannich base selected from the group consisting of

3-(dimethylaminophenylmethyl)-1H-indole-2-carboxylic acid ethyl ester
3-(dimethylaminophenylmethyl)-1H-indole-2-carboxylic acid
[(5-fluoro-1H-indol-3-yl)-phenylmethyl]-dimethylamine
[(1-ethyl-2-phenyl-1H-indol-3-yl)-phenylmethyl]-dimethylamine
[(5-methoxy-1H-indol-3-yl)-phenylmethyl]-dimethylamine
3-(dimethylaminophenylmethyl)-5-hydroxy-1H-indole-2-carboxylic acid
dimethyl-[(2-methyl-1H-indol-3-yl)-phenylmethyl]-amine
3-(dimethylaminophenylmethyl)-1H-indol-4-ol
dimethyl-[(4-methyl-1H-indol-3-yl)-phenylmethyl]-amine
[(5-chloro-1H-indol-3-yl)-phenylmethyl]-dimethylamine
[(5-benzyloxy-1H-indol-3-yl)-phenylmethyl]-dimethylamine
acetic acid 3-(dimethylaminophenylmethyl)-1H-indol-4-yl ester
{[2-(4-chlorophenyl)-1H-indol-3-yl]-phenylmethyl}-dimethylamine
5-benzyloxy-3-(dimethylaminophenylmethyl)-1H-indole-2-carboxylic acid
dimethyl-[(2-methyl-5-nitro-1H-indol-3-yl)-phenylmethyl]-amine
dimethyl-[(2-methyl-6-nitro-1H-indol-3-yl)-phenylmethyl]-amine
[(6-fluoro-2-methyl-1H-indol-3-yl)-phenylmethyl]-dimethylamine
{[2-(4-fluorophenyl)-1H-indol-3-yl]-phenylmethyl}-dimethylamine
{[2-(3-chloro-4-fluorophenyl)-1H-indol-3-yl]-phenylmethyl}-dimethylamine
[(7-ethyl-1H-indol-3-yl)-phenylmethyl]-dimethylamine
3-(dimethylaminophenylmethyl)-1H-indol-6-carboxylic acid
dimethyl-[(1-methyl-1H-indol-3-yl)-phenylmethyl]-amine
dimethyl-[(2-phenyl-1H-indol-3-yl)-o-tolyl-methyl]-amine
[5-benzyloxy-1H-indol-3-yl)-o-tolylmethyl]-dimethylamine
[(2-methoxyphenyl)-(2-methyl-1H-indol-3-yl)-methyl]-dimethylamine
{[1-(4-methoxybenzyl)-1H-indol-3-yl]-phenyl-methyl}-dimethylamine
[(1H-indol-3-yl)-phenylmethyl]-dimethylamine
[(5-bromo-2-fluoro-phenyl)-(4-nitro-1H-indol-3-yl)-methyl]-dimethyl-amine
[(5-bromo-2-fluoro-phenyl)-(1-methyl-1H-indol-3-yl)-methyl]-dimethyl-amine
[(2-chloro-6-fluoro-phenyl)-(2-phenyl-1H-indol-3-yl)-methyl]-dimethylamine
[(2-chloro-6-fluorophenyl)-(1-ethyl-2-phenyl-1H-indol-3-yl)-methyl]-dimethylamine

[(2-chloro-6-fluorophenyl)-(4-nitro-1H-indol-3-yl)-methyl]-dimethylamine
[(2-chloro-6-fluorophenyl)-(1-methyl-1H-indol-3-yl)-methyl]-dimethylamine
[(2-bromophenyl)-(4-nitro-1H-indol-3-yl)-methyl]-dimethylamine
[(2-bromophenyl)-(1-methyl-1H-indol-3-yl)-methyl]-dimethylamine
[(3-bromophenyl)-(7-ethyl-1H-indol-3-yl)-methyl]-dimethylamine
[(3-bromophenyl)-(1-methyl-1H-indol-3-yl)-methyl]-dimethylamine
[(4-tert-butylphenyl)-(2-methyl-1H-indol-3-yl)-methyl]-dimethylamine
[(4-tert-butylphenyl)-(1-ethyl-2-phenyl-1H-indol-3-yl)-methyl]-dimethylamine
[(4-tert-butylphenyl)-(1-methyl-1H-indol-3-yl)-methyl]-dimethylamine
[(2-chloro-4-fluorophenyl)-(4-nitro-1H-indol-3-yl)-methyl]-dimethylamine
[(2-chloro-4-fluorophenyl)-(4-methyl-1H-indol-3-yl)-methyl]-dimethylamine
[(2-chloro-6-fluorophenyl)-(2-phenyl-1H-indol-3-yl)-methyl]-dimethylamine
{(2-chloro-6-fluoro-phenyl)-[2-(4-chlorophenyl)-1H-indol-3-yl]-methyl}-dimethylamine
[(2-chloro-6-fluorophenyl)-(1-methyl-1H-indol-3-yl)-methyl]-dimethylamine
[(3-chlorophenyl)-(1-methyl-1H-indol-3-yl)-methyl]-dimethylamine
[(2,3-dichloro-phenyl)-(1-methyl-1H-indol-3-yl)-methyl]-dimethyl-amine
[(2,4-dichlorophenyl)-(1-methyl-1H-indol-3-yl)-methyl]-dimethylamine.
[(4-tert-butylphenyl)-(1-ethyl-2-phenyl-1H-indol-3-yl)-methyl]-dimethylamine
[(2-chloro-4-fluorophenyl)-(2-methyl-1H-indol-3-yl)-methyl]-dimethylamine
[(2-chloro-6-fluorophenyl)-(2-methyl-1H-indol-3-yl)-methyl]-dimethylamine
[(2,3-dimethoxyphenyl)-(4-nitro-1H-indol-3-yl)-methyl]-dimethylamine
3-[(2,3-dimethoxyphenyl)-dimethylaminomethyl]-4,7-dihydro-1H-indol-6-carboxylic acid 3-[(2,3-dimethoxyphenyl)-dimethylaminomethyl]-5-hydroxy-1H-indol-2-carboxylic acid
[(3,4-dimethoxyphenyl)-(2-methyl-1H-indol-3-yl)-methyl]-dimethylamine
[[2-(4-chlorophenyl)-1H-indol-3-yl]-(3,4-dimethoxyphenyl)-methyl]-dimethylamine
[(3,4-dimethoxyphenyl)-(1-ethyl-2-phenyl-1H-indol-3-yl)-methyl]-dimethylamine
[(3,4-dimethoxyphenyl)-(7-ethyl-1H-indol-3-yl)-methyl]-dimethylamine
[(3,4-dimethoxyphenyl)-(1-methyl-1H-indol-3-yl)-methyl]-dimethylamine
[(3,4-dimethoxyphenyl)-[2-(4-fluorophenyl)-1H-indol-3-yl)-methyl]-dimethylamine
[[2-(3-chloro-4-fluorophenyl)-1H-indol-3-yl]-(3,4-dimethoxyphenyl)-methyl]-dimethylamine
[(5-chloro-1H-indol-3-yl)-(2-fluorophenyl)-methyl]-dimethylamine
[(4-fluorophenyl)-(4-methyl-1H-indol-3-yl)-methyl]-dimethylamine
[(7-ethyl-1H-indol-3-yl)-(2-methoxyphenyl)-methyl]-dimethylamine
dimethyl-[(2-methyl-1H-indol-3-yl)-o-tolylmethyl]-amine
[(7-ethyl-1H-indol-3-yl)-o-tolylmethyl]-dimethyl-amine
dimethyl-[(1-methyl-1H-indol-3-yl)-o-tolylmethyl]-amine
[(5-chloro-1H-indol-3-yl)-o-tolylmethyl]-dimethylamine
[(5-chloro-1H-indol-3-yl)-m-tolylmethyl]-dimethylamine
dimethyl-[(2-methyl-1H-indol-3-yl)-p-tolylmethyl]-amine
[(5-chloro-1H-indol-3-yl)-p-tolylmethyl]-dimethylamine
dimethyl-[(2-methyl-1H-indol-3-yl)-(-3-phenoxyhenyl)-methyl]-amine
[(1-ethyl-2-phenyl-1H-indol-3-yl)-(-3-phenoxyphenyl)-methyl]-dimethylamine
[(7-ethyl-1H-indol-3-yl)-(3-phenoxyphenyl)-methyl]-dimethylamine
dimethyl-[(1-methyl-1H-indol-3-yl)-(3-phenoxyphenyl)-methyl]-amine
[[2-(4-fluorophenyl)-1H-indol-3-yl]-(3-phenoxyphenyl)-methyl]-dimethylamine
[[2-(3-chloro-4-fluorophenyl)-1H-indol-3-yl]-(3-phenoxyphenyl)-methyl]-dimethylamine
dimethyl-[(4-methyl-1H-indol-3-yl)-(3-phenoxyphenyl)-methyl]-amine
[(5-chloro-1H-indol-3-yl)-(2-trifluoromethylphenyl)-methyl]-dimethylamine
dimethyl-[(2-methyl-1H-indol-3-yl)-(4-trifluoromethyl-phenyl)-methyl]-amine.

**64.** Use according to claim 62, **characterised in that** the substituted indole Mannich base is selected from the group consisting of

1-methyl-3-(morpholin-4-yl-phenylmethyl)-1H-indole
3-[(2-methoxyphenyl)-pyrrolidin-1-yl-methyl]-1-methyl-1H-indole
1-ethyl-2-phenyl-3-(pyrrolidin-1-yl-o-tolylmethyl)-1H-indole
3-[(2-chlorophenyl)-piperidin-1-yl-methyl]-1-ethyl-2-phenyl-1H-indole
1-ethyl-2-phenyl-3-(phenylpyrrolidin-1-yl-methyl)-1H-indole

1-ethyl-3-[(2-methoxyphenyl)-pyrrolidin-1-yl-methyl]-2-phenyl-1H-indole
2-phenyl-3-(pyrrolidin-1-yl-*o*-tolylmethyl)-1H-indole 2-phenyl-3-(piperidin-1-yl-o-tolylmethyl)-1H-indole
3-[(2-chlorophenyl)-piperidin-1-yl-methyl]-2-phenyl-1H-indole
2-phenyl-3-(phenylpyrrolidin-1-yl-methyl)-1H-indole
3-[(2-methoxy-phenyl)-pyrrolidin-1-yl-methyl]-2-phenyl-1H-indole
4-methyl-3-(piperidin-1-yl-o-tolyl-methyl)-1H-indole
3-[(2-methoxyphenyl)-pyrrolidin-1-yl-methyl]-4-methyl-1H-indole
5-benzyloxy-3-(phenylpiperidin-1-yl-methyl)-1H-indole
7-ethyl-3-[(2-methoxyphenyl)-morpholin-4-yl-methyl]-1H-indole
3-[(2-methoxyphenyl)-piperidin-1-yl-methyl]-2-phenyl-1H-indole
5-chloro-3-[(2-methoxyphenyl)-piperidin-1-yl-methyl]-1H-indole
5-chloro-3-[(2-methoxyphenyl)-pyrrolidin-1-yl-methyl]-1H-indole
5-benzyloxy-3-[(2 methoxyphenyl)-pyrrolidin-1-yl-methyl]-1H-indole
3-(phenylpiperidin-1-yl-methyl)-1H-indole
1-methyl-3-(phenylpiperidin-1-yl-methyl)-1H-indole 3-(phenylpyrrolidin-1-yl-methyl)-1H-indole
5-benzyloxy-3-(phenylpyrrolidin-1-yl-methyl)-1H-indole

## Revendications

1. Bases substituées de Mannich de type indole de formule générale (I)

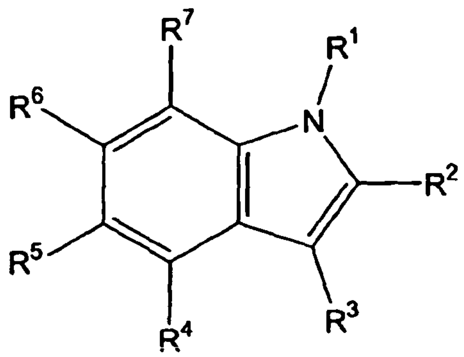

I

où

$R^1$ = H, un radical $C_{1-10}$-alkyle, un radical phényle ou naphtyle, lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH,

$R^2$ = H, F, Cl, Br, $CF_3$, CN, $NO_2$, $NHR^8$, $SR^9$, $OR^{10}$, $SO_2NH_2$, $SO_2NHR^{21}$, CO ($OR^{11}$), $CH_2CO$ ($OR^{12}$), $COR^{19}$, un radical $C_{1-10}$alkyle, un radical phényle ou naphtyle, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles ou un radical thiophène, pyrrolyle ou furfuryle lié via un groupe $C_{1-6}$-alkylène, non substitué ou substitué par un radical halogène, CN, $CF_3$ ou OH,

$R^3$ = $CH(R^{13})N(R^{14})$ ($R^{15}$),

$R^4$, $R^5$, $R^6$, $R^7$, sont identiques ou différents et = H, F, Cl, Br, $CF_3$, CN, $NO_2$, $NHR^8$ , $SR^9$, $OR^{10}$, $SO_2NH_2$, $SO_2NHR^{21}$, CO ($OR^{11}$), $CH_2CO(OR^{12})$, $COR^{19}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou un radical phényle, ledit radical phényle ou naphtyle étant le cas échéant condensé avec d'autres cycles, un radical thiophène, pyrrolyle ou furfuryle, non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN,

$C_{1-6}$-alkyle, $C_{1-6}$-alcoxy ou phényle, ledit radical phényle ou naphtyle étant le cas échéant condensé avec d'autres cycles ou un radical thiophène, pyrrolyle, furfuryle, lié via un groupe $C_{1-6}$-alkylène, non substitué ou substitué par un radical halogène, CN, $CF_3$ ou OH,

$R^8$ = H, $COR^{16}$, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^9$ = H, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^{10}$ = H, $COR^{17}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH,

$R^{11}$ = H, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^{12}$ = H, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^{13}$ = un radical phényle non substitué ou un radical phényle au moins monosubstitué par un radical $C_{1-4}$-alkyle, halogène, $CF_3$, CN ou OH,

$R^{14}$, $R^{15}$ sont identiques ou différents et signifient un radical $C_{1-6}$-alkyle ramifié, non ramifié, saturé, insaturé, non substitué ou au moins monosubstitué ou un radical phényle, benzyle ou phénéthyle non substitué ou au moins monosubstitué,

ou $R^{14}$ et $R^{15}$ signifient ensemble $(CH_2)_n$, avec n = un nombre entier de 3 à 6 ou $(CH_2)_2O(CH_2)_2$,

$R^{16}$ = un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle non substitué ou substitué par un radical halogène, CN, $CF_3$ ou OH,

$R^{17}$ = un radical $C_{1-10}$-alkyle,

$R^{18}$ = un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle non substitué ou substitué par un radical halogène, CN, $CF_3$ ou OH,

$R^{19}$ = H, $NHNH_2$, $NHR^{20}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH,

$R^{20}$ = H, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy
ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH,

$R^{21}$ = H, $COR^{17}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène,

pyrrolyle ou furfuryle lié via un groupe $C_{1-6}$-alkylène, non substitué ou substitué par un radical halogène, CN, $CF_3$ ou OH,

et/ou leurs racémates, énantiomères, diastéréo-isomères et/ou bases correspondantes et/ou sels correspondants d'acides physiologiquement acceptables,

à l'exception des racémates des composés dans lesquels le radical $R^3$ = CH ($R^{13}$) N ($R^{14}$) ($R^{15}$) et à chaque fois les radicaux $R^4$ à $R^7$ et $R^2$ = H, le radical $R^{13}$ = phényle, les radicaux $R^{14}$ et $R^{15}$ ensemble = $(CH_2)_5$ et le radical $R^1$ = H, $CH_3$ ou benzyle

ou

les radicaux $R^4$ à $R^7$ et $R^2$ = H, le radical $R^{13}$ = phényle, les radicaux $R^{14}$, $R^{15}$ à chaque fois = $CH_3$, et le radical $R^1$ = H, $CH_3$ ou benzyle

ou

les radicaux $R^4$ à $R^7$ et $R^1$ = H, le radical $R^{13}$ = 2-chlorophényle, les radicaux $R^{14}$ et $R^{15}$ à chaque fois = $CH_3$ ou les radicaux $R^{14}$ et $R^{15}$ ensemble = $(CH_2)_2O(CH_2)_2$, le radical $R^2$ = $COOR^{11}$ et les radicaux $R^{11}$ = $C_2H_5$ ainsi que les chlorhydrates correspondants

ou

les radicaux $R^4$ à $R^7$ et $R^1$ = H, le radical $R^{13}$ = phényle, les radicaux $R^{14}$ et $R^{15}$ ensemble = $(CH_2)_5$, le radical $R^2$ = $COOR^{11}$ et le radical $R^{11}$ = $CH_3$ et le chlorhydrate correspondant

ou

les radicaux $R^4$ à $R^7$ et $R^2$ = H, le radical $R^{13}$ = phényle, les radicaux $R^{14}$ et $R^{15}$ ensemble = $(CH_2)_4$, le radical $R^1$ = H, $CH_3$, $CH_2$-CH=$CH_2$ ou 4-chlorobenzyle ainsi que pour $R^1$ = $CH_3$ également le chlorhydrate correspondant du composé

ou

les radicaux $R^4$ à $R^7$, $R^1$ et $R^2$ = H, le radical $R^{13}$ = phényle, le radical $R^{14}$= phényle et le radical $R^{15}$ = 4-éthoxyphényle

ou

les radicaux $R^4$ à $R^7$ et $R^1$ = H, le radical $R^{13}$ = phényle, 2-hydroxyphényle ou 2-hydroxy-5-méthylphényle, les radicaux $R^{14}$ et $R^{15}$ ensemble = $(CH_2)_5$ et le radical $R^2$ = $CH_3$, pour $R^{13}$ = phényle également l'hydrogénosulfate correspondant

ou

les radicaux $R^4$ à $R^7$ et $R^1$ = H, le radical $R^{13}$ = phényle, les radicaux $R^{14}$ et $R^{15}$ ensemble = $(CH_2)_2O(CH_2)_2$ et le radical $R^2$ = $CH_3$ et l'hydrogénosulfate correspondant du composé.

2. Bases substituées de Mannich de type indole selon la revendication 1, **caractérisées en ce que** le radical $R^1$ représente H et les radicaux $R^2$ à $R^{21}$ présentent la signification selon la revendication 1.

3. Bases substituées de Mannich de type indole selon la revendication 1, **caractérisées en ce que** le radical $R^1$ représente un radical $C_{1-6}$-alkyle, de préférence un radical $C_{1-2}$-alkyle et les radicaux $R^2$ à $R^{21}$ ont la signification selon la revendication 1.

4. Bases substituées de Mannich de type indole selon la revendication 1 **caractérisées en ce que** le radical $R^1$ représente un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, et les radicaux $R^2$ à $R^{21}$ ont la signification selon la revendication 1.

5. Bases substituées de Mannich de type indole selon l'une quelconque des revendications 1 à 4, **caractérisées en ce que** le radical $R^2$ représente H et les radicaux $R^3$ à $R^{21}$ présentent la signification selon la revendication 1.

6. Bases substituées de Mannich de type indole selon l'une quelconque des revendications 1 à 4, **caractérisées en ce que** le radical $R^2$ représente un radical $C_{1-6}$-alkyle, de préférence un radical $C_{1-2}$-alkyle et les radicaux $R^3$ à $R^{21}$ présentent la signification selon la revendication 1.

7. Bases substituées de Mannich de type indole selon l'une quelconque des revendications 1 à 4, **caractérisées en ce que** le radical $R^2$ représente un radical phényle non substitué et les radicaux $R^3$ à $R^{21}$ présentent la signification selon la revendication 1.

8. Bases substituées de Mannich de type indole selon l'une quelconque des revendications 1 à 4, **caractérisées en ce que** le radical $R^2$ représente un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, et les autres radicaux $R^3$ à $R^{21}$ ont la

signification selon la revendication 1.

**9.** Bases substituées de Mannich de type indole selon l'une quelconque des revendications 1 à 4, **caractérisées en ce que** le radical $R^2$ représente Cl ou F et les autres radicaux $R^3$ à $R^{21}$ présentent la signification selon la revendication 1.

**10.** Bases substituées de Mannich de type indole selon l'une quelconque des revendications 1 à 4, **caractérisées en ce que** le radical $R^2$ représente $NO_2$ et les autres radicaux $R^3$ à $R^{21}$ présentent la signification selon la revendication 1.

**11.** Bases substituées de Mannich de type indole selon l'une quelconque des revendications 1 à 4, **caractérisées en ce que** le radical $R^2$ représente $OR^{10}$ et les autres radicaux $R^3$ à $R^{21}$ présentent la signification selon la revendication 1.

**12.** Bases substituées de Mannich de type indole selon l'une quelconque des revendications 1 à 4, **caractérisées en ce que** le radical $R^2$ représente $CO(OR^{11})$ et les autres radicaux $R^3$ à $R^{21}$ présentent la signification selon la revendication 1.

**13.** Bases substituées de Mannich de type indole selon l'une quelconque des revendications 1 à 12, **caractérisées en ce qu'**au moins un des radicaux $R^4$, $R^5$, $R^6$ ou $R^7$ représente H et à chaque fois les autres radicaux $R^4$, $R^5$, $R^6$ ou $R^7$ et $R^8$ à $R^{21}$ ont la signification selon la revendication 1.

**14.** Bases substituées de Mannich de type indole selon l'une quelconque des revendications 1 à 12, **caractérisées en ce qu'**au moins un des radicaux $R^4$, $R^5$, $R^6$ ou $R^7$ représente Cl ou F et à chaque fois les autres radicaux $R^4$, $R^5$, $R^6$ ou $R^7$ et $R^8$ à $R^{21}$ ont la signification selon la revendication 1.

**15.** Bases substituées de Mannich de type indole selon l'une quelconque des revendications 1 à 12, **caractérisées en ce qu'**au moins un des radicaux $R^4$, $R^5$, $R^6$ ou $R^7$ représente un radical $C_{1-6}$-alkyle, de préférence un radical $C_{1-2}$-alkyle et à chaque fois les autres radicaux $R^4$, $R^5$, $R^6$ ou $R^7$ et $R^8$ à $R^{21}$ ont la signification selon la revendication 1.

**16.** Bases substituées de Mannich de type indole selon l'une quelconque des revendications 1 à 12, **caractérisées en ce qu'**au moins un des radicaux $R^4$, $R^5$, $R^6$ ou $R^7$ représente $NO_2$ et à chaque fois les autres radicaux $R^4$, $R^5$, $R^6$ ou $R^7$ et $R^8$ à $R^{21}$ ont la signification selon la revendication 1.

**17.** Bases substituées de Mannich de type indole selon l'une quelconque des revendications 1 à 12, **caractérisées en ce qu'**au moins un des radicaux $R^4$, $R^5$, $R^6$ ou $R^7$ représente un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, et à chaque fois les autres radicaux $R^4$, $R^5$, $R^6$ ou $R^7$ et $R^8$ à $R^{21}$ ont la signification selon la revendication 1.

**18.** Bases substituées de Mannich de type indole selon l'une quelconque des revendications 1 à 12, **caractérisées en ce qu'**au moins un des radicaux $R^4$, $R^5$, $R^6$ ou $R^7$ représente $OR^{10}$ et à chaque fois les autres radicaux $R^4$, $R^5$, $R^6$ ou $R^7$ et $R^8$ à $R^{21}$ ont la signification selon la revendication 1.

**19.** Bases substituées de Mannich de type indole selon l'une quelconque des revendications 1 à 12, **caractérisées en ce qu'**au moins un des radicaux $R^4$, $R^5$, $R^6$ ou $R^7$ représente $CO(OR^{11})$ et à chaque fois les autres radicaux $R^4$, $R^5$, $R^6$ ou $R^7$ et $R^8$ à $R^{21}$ ont la signification selon la revendication 1.

**20.** Bases substituées de Mannich de type indole selon l'une quelconque des revendications 1 à 12, **caractérisées en ce qu'**au moins un des radicaux $R^4$, $R^5$, $R^6$ ou $R^7$ représente un radical phényle ou naphtyle, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence un radical phényle non substitué et à chaque fois les autres radicaux $R^4$, $R^5$, $R^6$ ou $R^7$ et $R^8$ à $R^{21}$ ont la signification selon la revendication 1.

**21.** Bases substituées de Mannich de type indole selon l'une quelconque des revendications 1 à 20, **caractérisées en ce que** le radical $R^8$ représente un radical $C_{1-6}$-alkyle et les autres radicaux $R^9$ à $R^{21}$ présentent la signification selon la revendication 1.

**22.** Bases substituées de Mannich de type indole selon l'une quelconque des revendications 1 à 21, **caractérisées en**

**ce que** le radical $R^9$ représente un radical $C_{1-6}$-alkyle et les autres radicaux $R^{10}$ à $R^{21}$ présentent la signification selon la revendication 1.

**23.** Bases substituées de Mannich de type indole selon l'une quelconque des revendications 1 à 21, **caractérisées en ce que** le radical $R^9$ représente un radical phényle et les radicaux $R^{10}$ à $R^{21}$ présentent la signification selon la revendication 1.

**24.** Bases substituées de Mannich de type indole selon l'une quelconque des revendications 1 à 23, **caractérisées en ce que** le radical $R^{10}$ représente H et les radicaux $R^{11}$ à $R^{21}$ présentent la signification selon la revendication 1.

**25.** Bases substituées de Mannich de type indole selon l'une quelconque des revendications 1 à 23, **caractérisées en ce que** le radical $R^{10}$ représente un radical $C_{1-6}$-alkyle et les radicaux $R^{11}$ à $R^{21}$ présentent la signification selon la revendication 1.

**26.** Bases substituées de Mannich de type indole selon l'une quelconque des revendications 1 à 23, **caractérisées en ce que** le radical $R^{10}$ représente un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, et les radicaux $R^{11}$ à $R^{21}$ ont la signification selon la revendication 1.

**27.** Bases substituées de Mannich de type indole selon l'une quelconque des revendications 1 à 26, **caractérisées en ce que** le radical $R^{11}$ représente H et les radicaux $R^{12}$ à $R^{21}$ présentent la signification selon la revendication 1.

**28.** Bases substituées de Mannich de type indole selon l'une quelconque des revendications 1 à 26, **caractérisées en ce que** le radical $R^{11}$ représente un radical $C_{1-6}$-alkyle et les radicaux $R^{12}$ à $R^{21}$ présentent la signification selon la revendication 1.

**29.** Bases substituées de Mannich de type indole selon l'une quelconque des revendications 1 à 28, **caractérisées en ce que** le radical $R^{12}$ représente un radical $C_{1-6}$-alkyle et les radicaux $R^{13}$ à $R^{21}$ présentent la signification selon la revendication 1.

**30.** Bases substituées de Mannich de type indole selon l'une quelconque des revendications 1 à 29, **caractérisées en ce que** le radical $R^{13}$ signifie un radical phényle non substitué ou un radical phényle au moins monosubstitué par méthyle, tert-butyle, F, Cl, Br, ou $CF_3$, de préférence un radical phényle non substitué ou un radical 2-méthylphényle, 3-méthylphényle, 4-méthylphényle, 2-tert-butylphényle, 3-tert-butylphényle, 4-tert-butylphényle, 2-fluorophényle, 3-fluorophényle, 4-fluorophényle, 2-chlorophényle, 3-chlorophényle, 4-chlorophényle, 2-bromophényle, 3-bromophényle, 4-bromophényle, 5-bromo-2-fluorophényle, 2-chloro-4-fluorophényle, 2-chloro-5-fluorophényle, 2-chloro-6-fluorophényle, 4-bromo-2-fluorophényle, 3-bromo-4-fluorophényle, 3-bromo-2-fluorophényle, 2,3-dichlorophényle, 2,4-dichlorophényle, 2,5-dichlorophényle, 3,4-dichlorophényle, 2,3-diméthylphényle, 2,4-diméthylphényle, 2,5-diméthylphényle, 2-trifluorométhylphényle, 3-trifluorométhylphényle ou 4-trifluorométhylphényle, de manière particulièrement préférée un radical phényle non substitué et les radicaux $R^{14}$ à $R^{21}$ ont la signification selon la revendication 1.

**31.** Bases substituées de Mannich de type indole selon l'une quelconque des revendications 1 à 30, **caractérisées en ce qu'**au moins un des radicaux $R^{14}$ ou $R^{15}$ représente un radical $C_{1-6}$-alkyle ramifié, non ramifié, saturé, insaturé, non substitué ou au moins monosubstitué, de préférence un radical $CH_3$, et à chaque fois l'autre radical $R^{14}$ ou $R^{15}$ et les radicaux $R^{16}$ à $R^{21}$ ont la signification selon la revendication 1.

**32.** Bases substituées de Mannich de type indole selon l'une quelconque des revendications 1 à 30, **caractérisées en ce que** les radicaux $R^{14}$, $R^{15}$ signifient ensemble $(CH_2)_n$ avec n = 4 ou 5 et les radicaux $R^{16}$ à $R^{21}$ ont la signification selon la revendication 1.

**33.** Bases substituées de Mannich de type indole selon l'une quelconque des revendications 1 à 32, **caractérisées en ce que** le radical $R^{16}$ représente un radical $C_{1-6}$-alkyle et les radicaux $R^{17}$ à $R^{21}$ présentent la signification selon la revendication 1.

**34.** Bases substituées de Mannich de type indole selon l'une quelconque des revendications 1 à 33, **caractérisées en ce que** le radical $R^{17}$ représente un radical $C_{1-6}$-alkyle et les radicaux $R^{18}$ à $R^{21}$ présentent la signification selon la

revendication 1.

**35.** Bases substituées de Mannich de type indole selon l'une quelconque des revendications 1 à 34, **caractérisées en ce que** le radical $R^{18}$ représente un radical $C_{1-6}$-alkyle et les radicaux $R^{19}$ à $R^{21}$ présentent la signification selon la revendication 1.

**36.** Bases substituées de Mannich de type indole selon l'une quelconque des revendications 1 à 35, **caractérisées en ce que** le radical $R^{19}$ représente un radical $C_{1-6}$-alkyle et les radicaux $R^{20}$ et $R^{21}$ présentent la signification selon la revendication 1.

**37.** Bases substituées de Mannich de type indole selon l'une quelconque des revendications 1 à 35, **caractérisées en ce que** le radical $R^{19}$ représente un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, et les radicaux $R^{20}$ et $R^{21}$ ont la signification selon la revendication 1.

**38.** Bases substituées de Mannich de type indole selon l'une quelconque des revendications 1 à 37, **caractérisées en ce que** le radical $R^{20}$ représente un radical $C_{1-6}$-alkyle et le radical $R^{21}$ présente la signification selon la revendication 1.

**39.** Bases substituées de Mannich de type indole selon l'une quelconque des revendications 1 à 37, **caractérisées en ce que** le radical $R^{20}$ représente un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, et le radical $R^{21}$ a la signification selon la revendication 1.

**40.** Bases substituées de Mannich de type indole selon l'une quelconque des revendications 1 à 39, **caractérisées en ce que** le radical $R^{21}$ représente H.

**41.** Bases substituées de Mannich de type indole selon l'une quelconque des revendications 1 à 39, **caractérisées en ce que** le radical $R^{21}$ représente un radical $C_{1-6}$-alkyle.

**42.** Bases substituées de Mannich de type indole selon l'une quelconque des revendications 1 à 39, **caractérisées en ce que** le radical $R^{21}$ représente un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles.

**43.** Bases substituées de Mannich de type indole selon la revendication 1 :

ester éthylique de l'acide 3-(diméthylaminophénylméthyl)-1H-indole-2-carboxylique acide 3-(diméthylaminophénylméthyl)-1H-indole-2-carboxylique
[(5-fluoro-1H-indol-3-yl)-phénylméthyl]-diméthylamine [(1-éthyl-2-phényl-1H-indol-3-yl)-phénylméthyl]-diméthylamine
[(5-méthoxy-1H-indol-3-yl)-phénylméthyl]-diméthylamine acide 3-(diméthylaminophénylméthyl)-5-hydroxy-1H-indole-2-carboxylique
diméthyl-[(2-méthyl-1H-indol-3-yl)-phénylméthyl]-amine 3-(diméthylaminophénylméthyl)-1H-indol-4-ol
diméthyl-[(4-méthyl-1H-indol-3-yl)-phénylméthyl]-amine [(5-chloro-1H-indol-3-yl)-phénylméthyl]-diméthylamine [(5-benzyloxy-1H-indol-3-yl)-phénylméthyl]-diméthylamine
ester 3-(diméthylaminophénylméthyl)-1H-indol-4-ylique de l'acide acétique
{[2-(4-chlorophényl)-1H-indol-3-yl]-phénylméthyl}-diméthylamine
acide 5-benzyloxy-3-(diméthylaminophénylméthyl)-1H-indole-2-carboxylique
diméthyl-[(2-méthyl-5-nitro-1H-indol-3-yl)-phénylméthyl]-amine
diméthyl-[(2-méthyl-6-nitro-1H-indol-3-yl)-phénylméthyl]-amine
[(6-fluoro-2-méthyl-1H-indol-3-yl)-phénylméthyl]-diméthylamine
{[2-(4-fluorophényl)-1H-indol-3-yl]-phénylméthyl}-diméthylamine
{[2-(3-chloro-4-fluorophényl)-1H-indol-3-yl]-phénylméthyl}-diméthylamine
[(7-éthyl-1H-indol-3-yl)-phénylméthyl]-diméthylamine acide 3-(diméthylaminophénylméthyl)-1H-indole-6-carboxylique

diméthyl-[(1-méthyl-1H-indol-3-yl)-phénylméthyl]-amine
diméthyl-[(2-phényl-1H-indol-3-yl)-o-toluylméthyl]-amine
[(5-benzyloxy-1H-indol-3-yl)-o-toluylméthyl]-diméthylamine
{[1-(4-méthoxybenzyl)-1H-indol-3-yl]-phénylméthyl}-diméthylamine
[(1H-indol-3-yl)-phénylméthyl]-diméthylamine
[(5-bromo-2-fluorophényl)-(4-nitro-1H-indol-3-yl)-méthyl]-diméthylamine
[(5-bromo-2-fluorophényl)-(1-méthyl-1H-indol-3-yl)-méthyl]-diméthylamine
[(2-chloro-6-fluorophényl)-(2-phényl-1H-indol-3-yl)-méthyl]-diméthylamine
[(2-chloro-6-fluorophényl)-(1-éthyl-2-phényl-1H-indol-3-yl)-méthyl]-diméthylamine
[(2-chloro-6-fluorophényl)-(4-nitro-1H-indol-3-yl)-méthyl]-diméthylamine
[(2-chloro-6-fluorophényl)-(1-méthyl-1H-indol-3-yl)-méthyl]-diméthylamine
[(2-bromophényl)-(4-nitro-1H-indol-3-yl)-méthyl]-diméthylamine
[(2-bromophényl)-(1-méthyl-1H-indol-3-yl)-méthyl]-diméthylamine
[(3-bromophényl)-(7-éthyl-1H-indol-3-yl)-méthyl]-diméthylamine
[(3-bromophényl)-(1-méthyl-1H-indol-3-yl)-méthyl]-diméthylamine
[(4-tert-butylphényl)-(2-méthyl-1H-indol-3-yl-)-méthyl]-diméthylamine
[(4-tert-butylphényl)-(1-éthyl-2-phényl-1H-indol-3-yl)-méthyl]-diméthylamine
[(4-tert-butylphényl)-(1-méthyl-1H-indol-3-yl)-méthyl]-diméthylamine
[(2-chloro-4-fluorophényl)-(4-nitro-1H-indol-3-yl)-méthyl]-diméthylamine
[(2-chloro-4-fluorophényl)-(4-méthyl-1H-indol-3-yl)-méthyl]-diméthylamine
[(2-chloro-6-fluorophényl)-(2-phényl-1H-indol-3-yl)-méthyl]-diméthylamine
{(2-chloro-6-fluorophényl)-[2-(4-chlorophényl)-1H-indol-3-yl]-méthyl}-diméthylamine
[(2-chloro-6-fluorophényl)-(1-méthyl-1H-indol-3-yl)-méthyl]-diméthylamine
[(3-chlorophényl)-(1-méthyl-1H-indol-3-yl)-méthyl]-diméthylamine
[(2,3-dichlorophényl)-(1-méthyl-1H-indol-3-yl)-méthyl]-diméthylamine
[(2,4-dichlorophényl)-(1-méthyl-1H-indol-3-yl)-méthyl]-diméthylamine
[(4-tert-butylphényl)-(1-éthyl-2-phényl-1H-indol-3-yl)-méthyl]-diméthylamine
[(2-chloro-4-fluorophényl)-(2-méthyl-1H-indol-3-yl)-méthyl]-diméthylamine
[(2-chloro-6-fluorophényl)-(2-méthyl-1H-indol-3-yl)-méthyl]-diméthylamine
[(5-chloro-1H-indol-3-yl)-(2-fluorophényl)-méthyl]-diméthylamine
[(4-fluorophényl)-(4-méthyl-1H-indol-3-yl)-méthyl]-diméthylamine
diméthyl-[(2-méthyl-1H-indol-3-yl)-o-toluylméthyl]-amine
[(7-éthyl-1H-indol-3-yl)-o-toluylméthyl]-diméthylamine diméthyl-[(1-méthyl-1H-indol-3-yl)-o-toluylméthyl]-amine
[(5-chloro-1H-indol-3-yl)-o-toluylméthyl]-diméthylamine
[(5-chloro-1H-indol-3-yl)-m-toluylméthyl]-diméthylamine diméthyl-[(2-méthyl-1H-indol-3-yl)-p-toluylméthyl]-amine
[(5-chloro-1H-indol-3-yl)-p-toluylméthyl]-diméthylamine
[(5-chloro-1H-indol-3-yl)-(2-trifluorométhylphényl)-méthyl]-diméthylamine
diméthyl-[(2-méthyl-1H-indol-3-yl)-(4-trifluorométhylphényl)-méthyl]-amine.

**44.** Bases substituées de Mannich de type indole selon la revendication 1 :

1-méthyl-3-(morpholin-4-yl-phénylméthyl)-1H-indole
1-éthyl-2-phényl-3-(pyrrolidin-1-yl-o-toluylméthyl)-1H-indole
3-[(2-chlorophényl)-pipéridin-1-yl-méthyl]-1-éthyl-2-phényl-1H-indole
1-éthyl-2-phényl-3-(phénylpyrrolidin-1-yl-méthyl)-1H-indole
2-phényl-3-(pyrrolidin-1-yl-o-toluylméthyl)-1H-indole
2-phényl-3-(pipéridin-1-yl-o-toluylméthyl)-1H-indole
3-[(2-chlorophényl)-pipéridin-1-yl-méthyl]-2-phényl-1H-indole
2-phényl-3-(phénylpyrrolidin-1-yl-méthyl)-1H-indole
4-méthyl-3-(pipéridin-1-yl-o-toluylméthyl)-1H-indole
5-benzyloxy-3-(phénylpipéridin-1-yl-méthyl)-1H-indole
3-(phénylpipéridin-1-yl-méthyl)-1H-indole
1-méthyl-3-(phénylpipéridin-1-yl-méthyl)-1H-indole
3-(phénylpyrrolidin-1-yl-méthyl)-1H-indole
5-benzyloxy-3-(phénylpyrrolidin-1-yl-méthyl)-1H-indole.

**45.** Procédé pour la préparation de bases substituées de Mannich de type indole de formule générale I selon l'une quelconque des revendications 1 à 44, **caractérisé en ce qu'**on transforme des composés aromatiques de type aldéhyde de formule générale II,

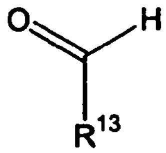

II

dans laquelle $R^{13}$ a la signification selon la formule générale I selon la revendication 1, en solution, en présence d'une base, à une température de préférence de -10 à +110°C avec des amines secondaires de formule générale III

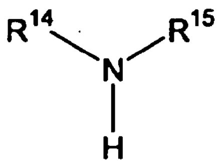

III,

dans laquelle les radicaux $R^{14}$ et $R^{15}$ ont la signification selon la formule générale I selon la revendication 1 en composés aminal de formule générale IV,

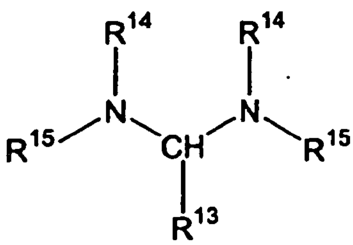

IV

et on transforme ces composés aminal de formule générale IV, sans autre purification, avec un chlorure d'acide, dans un solvant absolu, en sels d'iminium de formule générale V

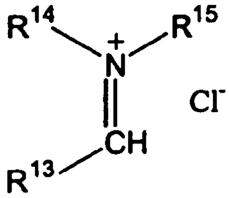

V

et on transforme ces sels d'iminium de formule générale V, sans autre purification, en solution, de préférence dans de l'acétonitrile et/ou du toluène avec de l'indole et/ou des composés substitués de type indole de formule générale VI,

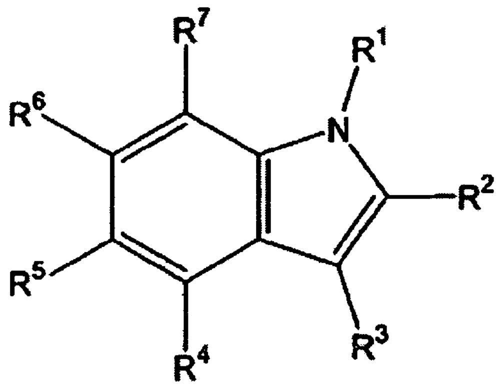

VI

dans laquelle le radical $R^3$ = H et les radicaux $R^1$, $R^2$ $R^4$ à $R^{12}$ et $R^{16}$ à $R^{21}$ ont la signification selon la formule générale I, et on purifie les bases substituées de Mannich de type indole ainsi obtenues de formule générale I selon l'une quelconque des revendications 1 à 44 par extraction et/ou lavage, de préférence par lavage avec de l'acétone et on les isole selon les procédés usuels.

**46.** Procédé pour la préparation de bases substituées de Mannich de type indole de formule générale I selon l'une quelconque des revendications 1 à 44, dans lequel le radical $R^1 \neq H$ et les radicaux $R^2$ à $R^{21}$ ont la signification selon la formule générale I, **caractérisé en ce qu'**on transforme des composés aromatiques de type aldéhyde de formule générale II,

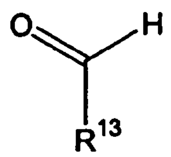

II

dans laquelle $R^{13}$ a la signification selon la formule générale I, en solution, en présence d'une base, à une température de préférence de -10 à +110°C avec des amines secondaires de formule générale III

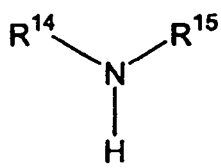

III

dans laquelle les radicaux $R^{14}$ et $R^{15}$ ont la signification selon la formule générale I,
en composés aminal de formule générale IV

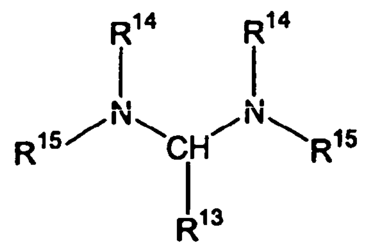

IV,

et on transforme ces composés aminal de formule générale IV, sans autre purification, avec un chlorure d'acide, dans un solvant absolu, en sels d'iminium de formule générale V

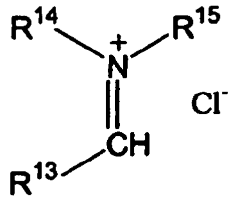

V

et on transforme ces sels d'iminium de formule générale V, sans autre purification, en solution, de préférence dans de l'acétonitrile et/ou du toluène avec de l'indole et/ou des composés substitués de type indole de formule générale VI,

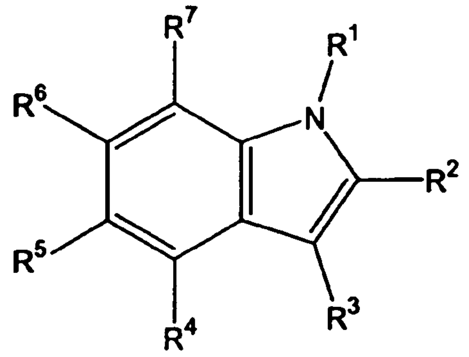

VI

dans laquelle les radicaux $R^1$ et $R^3$ sont à chaque fois = H et les radicaux $R^2$, $R^4$ à $R^{12}$ et $R^{16}$ à $R^{21}$ ont la signification selon la formule générale I, et on transforme les composés de formule générale VI ainsi obtenus, dans laquelle $R^1$ = H et les radicaux $R^2$ à $R^{21}$ ont la signification selon la formule générale I, en solution avec des composés de formule générale $XR^{22}$, dans laquelle $R^{22}$ représente un radical $C_{1-10}$-alkyle, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle, lié via un groupe $C_{1-6}$-alkylène, non substitué ou substitué par un radical halogène, CN, $CF_3$ ou OH et X = Cl, Br ou I, à une température de préférence de 10 à 150°C en présence d'une base et on purifie les composés de formule générale I ainsi obtenus, dans laquelle $R^1$ représente un radical $C_{1-10}$-alkyle, un radical phényle ou naphtyle lié via un groupe $C_{1-6}$-alkylène, non substitué ou au moins monosubstitué par

un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH et les autres radicaux $R^2$ à $R^{21}$ ont la signification selon la formule générale I, par filtration et on les isole selon les procédés usuels.

**47.** Procédé selon la revendication 46, **caractérisé en ce que** la transformation avec les composés de formule générale $XR^{22}$ est réalisée dans du diméthylsulfoxyde.

**48.** Procédé selon la revendication 46 ou 47, **caractérisé en ce que** X = Cl.

**49.** Procédé selon l'une quelconque des revendications 46 à 48, **caractérisé en ce que** le radical $R^{22}$ représente un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles.

**50.** Procédé selon l'une quelconque des revendications 46 à 49, **caractérisé en ce que** la transformation avec les composés de formule générale $XR^{22}$ est réalisée en présence d'hydroxyde de potassium comme base.

**51.** Procédé selon l'une quelconque des revendications 46 à 50, **caractérisé en ce que** les composés de formule générale I sont purifiés par filtration sur une résine piège, de préférence par filtration sur de la tris(2-aminoéthyl)-amine liée sur un polymère et/ou du 3-(3-mercaptophényl)propanamidométhylpolystyrène.

**52.** Procédé selon l'une quelconque des revendications 45 à 51, **caractérisé en ce que** les composés aromatiques de type aldéhyde de formule générale II sont transformés dans des solvants organiques, de préférence du toluène, avec des amines secondaires de formule générale III.

**53.** Procédé selon l'une quelconque des revendications 45 à 52, **caractérisé en ce que** les composés aromatiques de type aldéhyde de formule générale II sont transformés en présence de carbonate de potassium et/ou d'anhydride de l'acide borique comme base.

**54.** Procédé selon l'une quelconque des revendications 45 à 53, **caractérisé en ce que** les composés de type aminal de formule générale IV sont transformés avec du chlorure d'acétyle en sels d'iminium de formule générale V.

**55.** Procédé selon l'une quelconque des revendications 45 à 54, **caractérisé en ce que** les composés de type aminal de formule générale IV sont transformés dans du diéthyléther absolu en sels d'iminium de formule générale V.

**56.** Médicament contenant comme substance active au moins une base substituée de Mannich de type indole de formule générale I,

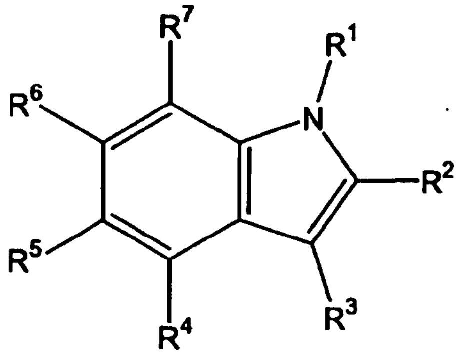

I

où

$R^1$ = H, un radical $C_{1-10}$-alkyle, un radical phényle ou naphtyle lié via un groupe $C_{1-6}$-alkylène, non substitué ou au

moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle, lié via un groupe $C_{1-6}$-alkylène, non substitué ou substitué par un radical halogène, CN, $CF_3$ ou OH, de préférence H, un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de manière particulièrement préférée H ou un radical $C_{1-2}$-alkyle,

$R^2$ = H, F, Cl, Br, $CF_3$, CN, $NO_2$, $NHR^8$, $SR^9$, $OR^{10}$, $SO_2NH_2$, $SO_2NHR^{21}$, CO ($OR^{11}$), $CH_2CO$ ($OR^{12}$), $COR^{19}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence = H, Cl, F, $NO_2$, $OR^{10}$, CO ($OR^{11}$), un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de manière particulièrement préférée H, un radical $C_{1-2}$-alkyle ou un radical phényle non substitué,

$R^3$ = CH ($R^{13}$) N ($R^{14}$) ($R^{15}$),

$R^4$, $R^5$, $R^6$, $R^7$ sont identiques ou différents et = H, F, Cl, Br, $CF_3$, CN, $NO_2$, $NHR^8$, $SR^9$, $OR^{10}$, $SO_2NH_2$, $SO_2NHR^{21}$, $CO(OR^{11})$, $CH_2CO(OR^{12})$, $COR^{16}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou un radical phényle, ledit radical phényle ou naphtyle étant le cas échéant condensé avec d'autres cycles, un radical thiophène, pyrrolyle ou furfuryle, non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles ou un radical thiophène, pyrrolyle, furfuryle, lié via un groupe $C_{1-6}$-alkylène, non substitué ou substitué par un radical halogène, CN, $CF_3$ ou OH, de préférence = H, Cl, F, $NO_2$, $OR^{10}$, CO ($OR^{11}$), un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de manière particulièrement préférée H, $NO_2$, un radical $C_{1-2}$-alkyle ou un radical phényle non substitué,

$R^8$ = H, $COR^{16}$, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence un radical $C_{1-6}$-alkyle,

$R^9$ = H, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence un radical $C_{1-6}$-alkyle ou phényle,

$R^{10}$ = H, $COR^{17}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence = H, un radical $C_{1-6}$-alkyle ou un radical phényle ou naphtyle lié via un groupe $C_{1-2}$-alkylène non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^{11}$ = H, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence = H ou un radical $C_{1-6}$-alkyle,

$R^{12}$ = H, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence un radical $C_{1-6}$-alkyle,

$R^{13}$ = un radical phényle ou naphtyle non substitué ou au moins monosubstitué par un radical OH, halogène, $CF_3$, CN, $C_{1-6}$-alkyle, $C_{1-6}$-alcoxy, -O-phényle ou phényle, ledit radical phényle ou naphtyle étant le cas échéant condensé

avec d'autres cycles, un radical thiophène, pyrrolyle ou furfuryle non substitué ou substitué par un radical halogène, CN, $CF_3$ ou OH ou un radical alkyle sans proton acide en position $\alpha$, de préférence un radical phényle non substitué ou un radical phényle, thiophène ou furfuryle au moins monosubstitué par un radical $C_{1-4}$-alkyle, $C_{1-3}$-alcoxy, halogène, $CF_3$, CN, O-phényle ou OH, de manière particulièrement préférée un radical phényle, thiophène ou furfuryle non substitué ou un radical 2-méthoxyphényle, 3-méthoxyphényle, 4-méthoxyphényle, 2-méthylphényle, 3-méthylphényle, 4-méthylphényle, 2-tert-butylphényle, 3-tert-butylphényle, 4-tert-butylphényle, 2-fluorophényle, 3-fluorophényle, 4-fluorophényle, 2-chlorophényle, 3-chlorophényle, 4-chlorophényle, 2-bromophényle, 3-bromophényle, 4-bromophényle, 5-bromo-2-fluorophényle, 2-chloro-4-fluorophényle, 2-chloro-5-fluorophényle, 2-chloro-6-fluorophényle, 4-bromo-2-fluorophényle, 3-bromo-4-fluorophényle, 3-bromo-2-fluorophényle, 2,3-dichlorophényle, 2,4-dichlorophényle, 2,5-dichlorophényle, 3,4-dichlorophényle, 2,3-diméthylphényle, 2,4-diméthylphényle, 2,5-diméthylphényle, 2,3-diméthoxyphényle, 2,4-diméthoxyphényle, 2,5-diméthoxyphényle, 3,4-diméthoxyphényle, 3,4,5-triméthoxyphényle, 2-trifluorométhylphényle, 3-trifluorométhylphényle ou 4-trifluorométhylphényle, de manière tout particulièrement préférée un radical phényle, thiophène ou furfuryle non substitué,

$R^{14}$, $R^{15}$, sont identiques ou différents et signifient un radical $C_{1-6}$-alkyle ramifié, non ramifié, saturé, insaturé, non substitué ou au moins monosubstitué ou un radical phényle, benzyle ou phénéthyle non substitué ou au moins monosubstitué, de préférence un radical $C_{1-6}$-alkyle saturé, non substitué ou au moins monosubstitué, de manière particulièrement préférée un radical $CH_3$,

ou $R^{14}$ et $R^{15}$ signifient ensemble $(CH_2)_n$, avec n = un nombre entier de 3 à 6 ou $(CH_2)_2O(CH_2)_2$, de préférence $(CH_2)_n$ avec n = 4 ou 5,

$R^{16}$ = un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle non substitué ou substitué par un radical halogène, CN, $CF_3$ ou OH, de préférence un radical $C_{1-6}$-alkyle

$R^{17}$ = un radical $C_{1-10}$-alkyle, de préférence un radical $C_{1-6}$-alkyle,

$R^{18}$ = un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle non substitué ou substitué par un radical halogène, CN, $CF_3$ ou OH, de préférence un radical $C_{1-6}$-alkyle,

$R^{19}$ = H, $NHNH_2$, $NHR^{20}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^{20}$ = H, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH, un radical phényle ou naphtyle lié via un radical $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^{21}$ = H, $COR^{17}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence = H, un radical $C_{1-6}$-alkyle ou un radical phényle ou naphtyle lié via un groupe $C_{1-2}$-alkylène,

non substitué ou substitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles
et/ou leurs racémates, énantiomères, diastéréo-isomères et/ou bases correspondantes et/ou sels correspondants d'acides physiologiquement acceptables et le cas échéant d'autres substances actives et/ou adjuvants.

**57.** Médicament selon la revendication 56 contenant au moins une base substituée de Mannich de type indole, choisie dans le groupe formé par

l'ester éthylique de l'acide 3-(diméthylaminophénylméthyl)-1H-indole-2-carboxylique
l'acide 3-(diméthylaminophénylméthyl)-1H-indole-2-carboxylique
la [(5-fluoro-1H-indol-3-yl)-phénylméthyl]-diméthylamine
la [(1-éthyl-2-phényl-1H-indol-3-yl)-phénylméthyl]-diméthylamine
la [(5-méthoxy-1H-indol-3-yl)-phénylméthyl]-diméthylamine
l'acide 3-(diméthylaminophénylméthyl)-5-hydroxy-1H-indole-2-carboxylique
la diméthyl-[(2-méthyl-1H-indol-3-yl)-phénylméthyl]-amine
le 3-(diméthylaminophénylméthyl)-1H-indol-4-ol
la diméthyl-[(4-méthyl-1H-indol-3-yl)-phénylméthyl]-amine
la [(5-chloro-1H-indol-3-yl)-phénylméthyl]-diméthylamine
la [(5-benzyloxy-1H-indol-3-yl)-phénylméthyl]-diméthylamine
l'ester 3-(diméthylaminophénylméthyl)-1H-indol-4-ylique de l'acide acétique
la {[2-(4-chlorophényl)-1H-indol-3-yl]-phénylméthyl}-diméthylamine
l'acide 5-benzyloxy-3-(diméthylaminophénylméthyl)-1H-indole-2-carboxylique
la diméthyl-[(2-méthyl-5-nitro-1H-indol-3-yl)-phénylméthyl]-amine
la diméthyl-[(2-méthyl-6-nitro-1H-indol-3-yl)-phénylméthyl]-amine
la [(6-fluoro-2-méthyl-1H-indol-3-yl)-phénylméthyl]-diméthylamine
la {[2-(4-fluorophényl)-1H-indol-3-yl]-phénylméthyl}-diméthylamine
la {[2-(3-chloro-4-fluorophényl)-1H-indol-3-yl]-phénylméthyl}-diméthylamine
la [(7-éthyl-1H-indol-3-yl)-phénylméthyl]-diméthylamine l'acide 3-(diméthylaminophénylméthyl)-1H-indole-6-carboxylique
la diméthyl-[(1-méthyl-1H-indol-3-yl)-phénylméthyl]-amine
la diméthyl-[(2-phényl-1H-indol-3-yl)-o-toluylméthyl]-amine
la [(5-benzyloxy-1H-indol-3-yl)-o-toluylméthyl]-diméthylamine
la [(2-méthoxyphényl)-(2-méthyl-1H-indol-3-yl)-méthyl]-diméthylamine
la {[1-(4-méthoxybenzyl)-1H-indol-3-yl]-phénylméthyl}-diméthylamine
la [(1H-indol-3-yl)-phénylméthyl]-diméthylamine
la [(5-bromo-2-fluorophényl)-(4-nitro-1H-indol-3-yl)-méthyl]-diméthylamine
la [(5-bromo-2-fluorophényl)-(1-méthyl-1H-indol-3-yl)-méthyl]-diméthylamine
la [(2-chloro-6-fluorophényl)-(2-phényl-1H-indol-3-yl)-méthyl]-diméthylamine
la [(2-chloro-6-fluorophényl)-(1-éthyl-2-phényl-1H-indol-3-yl)-méthyl]-diméthylamine
la [(2-chloro-6-fluorophényl)-(4-nitro-1H-indol-3-yl)-méthyl]-diméthylamine
la [(2-chloro-6-fluorophényl)-(1-méthyl-1H-indol-3-yl)-méthyl]-diméthylamine
la [(2-bromophényl)-(4-nitro-1H-indol-3-yl)-méthyl]-diméthylamine
la [(2-bromophényl)-(1-méthyl-1H-indol-3-yl)-méthyl]-diméthylamine
la [(3-bromophényl)-(7-éthyl-1H-indol-3-yl)-méthyl]-diméthylamine
la [(3-bromophényl)-(1-méthyl-1H-indol-3-yl)-méthyl]-diméthylamine
la [(4-tert-butylphényl)-(2-méthyl-1H-indol-3-yl-)-méthyl]-diméthylamine
la [(4-tert-butylphényl)-(1-éthyl-2-phényl-1H-indol-3-yl)-méthyl]-diméthylamine
la [(4-tert-butylphényl)-(1-méthyl-1H-indol-3-yl)-méthyl]-diméthylamine
la [(2-chloro-4-fluorophényl)-(4-nitro-1H-indol-3-yl)-méthyl]-diméthylamine
la [(2-chloro-4-fluorophényl)-(4-méthyl-1H-indol-3-yl)-méthyl]-diméthylamine
la [(2-chloro-6-fluorophényl)-(2-phényl-1H-indol-3-yl)-méthyl]-diméthylamine
la {(2-chloro-6-fluorophényl)-[2-(4-chlorophényl)-1H-indol-3-yl]-méthyl}-diméthylamine
la [(2-chloro-6-fluorophényl)-(1-méthyl-1H-indol-3-yl)-méthyl]-diméthylamine
la [(3-chlorophényl)-(1-méthyl-1H-indol-3-yl)-méthyl]-diméthylamine
la [(2,3-dichlorophényl)-(1-méthyl-1H-indol-3-yl)-méthyl]-diméthylamine
la [(2,4-dichlorophényl)-(1-méthyl-1H-indol-3-yl)-méthyl]-diméthylamine
la [(4-tert-butylphényl)-(1-éthyl-2-phényl-1H-indol-3-yl)-méthyl]-diméthylamine
la [(2-chloro-4-fluorophényl)-(2-méthyl-1H-indol-3-yl)-méthyl]-diméthylamine

la [(2-chloro-6-fluorophényl)-(2-méthyl-1H-indol-3-yl)-méthyl]-diméthylamine
la [(2,3-diméthoxyphényl)-(4-nitro-1H-indol-3-yl)-méthyl]-diméthylamine
l'acide 3-[(2,3-diméthoxyphényl)-diméthylaminométhyl]-4,7-dihydro-1H-indole-6-carboxylique
l'acide 3-[(2,3-diméthoxyphényl)-diméthylaminométhyl]-5-hydroxy-1H-indole-2-carboxylique
la [(3,4-diméthoxyphényl)-(2-méthyl-1H-indol-3-yl)-méthyl]-diméthylamine
la [[2-(4-chlorophényl)-1H-indol-3-yl]-(3,4-diméthoxyphényl)-méthyl]-diméthylamine
la [(3,4-diméthoxyphényl)-(1-éthyl-2-phényl-1H-indol-3-yl)-méthyl]-diméthylamine
la [(3,4-diméthoxyphényl)-(7-éthyl-1H-indol-3-yl)-méthyl]-diméthylamine
la [(3,4-diméthoxyphényl)-(1-méthyl-1H-indol-3-yl)-méthyl]-diméthylamine
la {(3,4-diméthoxyphényl)-[2-(4-fluorophényl)-1H-indol-3-yl]-méthyl}-diméthylamine
la [[2-(3-chloro-4-fluorophényl)-1H-indol-3-yl]-(3,4-diméthoxyphényl)-méthyl]-diméthylamine
la [(5-chloro-1H-indol-3-yl)-(2-fluorophényl)-méthyl]-diméthylamine
la [(4-fluorophényl)-(4-méthyl-1H-indol-3-yl)-méthyl]-diméthylamine
la [(7-éthyl-1H-indol-3-yl)-(2-méthoxyphényl)-méthyl]-diméthylamine
la diméthyl-[(2-méthyl-1H-indol-3-yl)-o-toluylméthyl]-amine
la [(7-éthyl-1H-indol-3-yl)-o-toluylméthyl]-diméthylamine
la diméthyl-[(1-méthyl-1H-indol-3-yl)-o-toluylméthyl]-amine
la [(5-chloro-1H-indol-3-yl)-o-toluylméthyl]-diméthylamine
la [(5-chloro-1H-indol-3-yl)-m-toluylméthyl]-diméthylamine
la diméthyl-[(2-méthyl-1H-indol-3-yl)-p-toluylméthyl]-amine
la [(5-chloro-1H-indol-3-yl)-p-toluylméthyl]-diméthylamine
la diméthyl-[(2-méthyl-1H-indol-3-yl)-(3-phénoxyphényl)-méthyl]-amine
la [(1-éthyl-2-phényl-1H-indol-3-yl)-(3-phénoxyphényl)-méthyl]-diméthylamine
la [(7-éthyl-1H-indol-3-yl)-(3-phénoxyphényl)-méthyl]-diméthylamine
la diméthyl-[(1-méthyl-1H-indol-3-yl)-(3-phénoxyphényl)-méthyl]-amine
la [[2-(4-fluorophényl)-1H-indol-3-yl]-(3-phénoxyphényl)-méthyl]-diméthylamine
la [[2-(3-chloro-4-fluorophényl)-1H-indol-3-yl]-(3-phénoxyphényl)-méthyl]-diméthylamine
la diméthyl-[(4-méthyl-1H-indol-3-yl)-(3-phénoxyphényl)-méthyl]-amine
la [(5-chloro-1H-indol-3-yl)-(2-trifluorométhylphényl)-méthyl]-diméthylamine
la diméthyl-[(2-méthyl-1H-indol-3-yl)-(4-trifluorométhylphényl)-méthyl]-amine.

**58.** Médicament selon la revendication 56 contenant au moins une base substituée de Mannich de type indole, choisie dans le groupe formé par

le 1-méthyl-3-(morpholin-4-yl-phénylméthyl)-1H-indole
le 3-[(2-méthoxyphényl)-pyrrolidin-1-yl-méthyl]-1-méthyl-1H-indole
le 1-éthyl-2-phényl-3-(pyrrolidin-1-yl-o-toluylméthyl)-1H-indole
le 3-[(2-chlorophényl)-pipéridin-1-yl-méthyl]-1-éthyl-2-phényl-1H-indole
le 1-éthyl-2-phényl-3-(phénylpyrrolidin-1-yl-méthyl)-1H-indole
le 1-éthyl-3-[(2-méthoxyphényl)-pyrrolidin-1-yl-méthyl]-2-phényl-1H-indole
le 2-phényl-3-(pyrrolidin-1-yl-o-toluylméthyl)-1H-indole
le 2-phényl-3-(pipéridin-1-yl-o-toluylméthyl)-1H-indole
le 3-[(2-chlorophényl)-pipéridin-1-yl-méthyl]-2-phényl-1H-indole
le 2-phényl-3-(phénylpyrrolidin-1-yl-méthyl)-1H-indole
le 3-[(2-méthoxyphényl)-pyrrolidin-1-yl-méthyl]-2-phényl-1H-indole
le 4-méthyl-3-(pipéridin-1-yl-o-toluylméthyl)-1H-indole
le 3-[(2-méthoxyphényl)-pyrrolidin-1-yl-méthyl]-4-méthyl-1H-indole
le 5-benzyloxy-3-(phénylpipéridin-1-yl-méthyl)-1H-indole
le 7-éthyl-3[(2-méthoxyphényl)-morpholin-4-yl-méthyl]-1H-indole
le 3-[(2-méthoxyphényl)-pipéridin-1-yl-méthyl]-2-phényl-1H-indole
le 5-chloro-3-[(2-méthoxyphényl)-pipéridin-1-yl-méthyl]-1H-indole
le 5-chloro-3-[(2-méthoxyphényl)-pyrrolidin-1-yl-méthyl]-1H-indole
le 5-benzyloxy-3-[(2-méthoxyphényl)-pyrrolidin-1-yl-méthyl]-1H-indole
le 3-(phénylpipéridin-1-yl-méthyl)-1H-indole
le 1-méthyl-3-(phénylpipéridin-1-yl-méthyl)-1H-indole
le 3-(phénylpyrrolidin-1-yl-méthyl)-1H-indole
le 5-benzyloxy-3-(phénylpyrrolidin-1-yl-méthyl)-1H-indole.

**59.** Médicament selon l'une quelconque des revendications 56 à 58, **caractérisé en ce qu'**il contient comme substance active un mélange d'énantiomères d'au moins une base substituée de Mannich de type indole de formule générale I selon l'une quelconque des revendications 56 à 58, le mélange présentant les énantiomères en des quantités non équimolaires.

**60.** Médicament selon la revendication 59, **caractérisé en ce que** la proportion relative d'un énantiomère dans le mélange est de 5 à 45% en mole, de préférence de 10 à 40% en mole, par rapport au mélange des énantiomères.

**61.** Médicament selon l'une quelconque des revendications 56 à 60 pour le traitement de/la lutte contre les douleurs, de préférence les douleurs chroniques, et/ou les réactions inflammatoires et/ou les réactions allergiques et/ou l'abus de drogues et/ou l'abus d'alcool et/ou la diarrhée et/ou la gastrite et/ou les ulcères et/ou les maladies cardiovasculaires et/ou l'incontinence urinaire et/ou les dépressions et/ou les états de choc et/ou les migraines et/ou la narcolepsie et/ou l'excès pondéral et/ou l'asthme et/ou le glaucome et/ou le syndrome d'hyperkinésie et/ou le manque d'énergie et/ou la boulimie et/ou l'anorexie et/ou la catalepsie et/ou pour l'anxiolyse et/ou pour l'augmentation de la vigilance et/ou pour l'augmentation de la libido.

**62.** Utilisation d'au moins un composé de formule générale I,

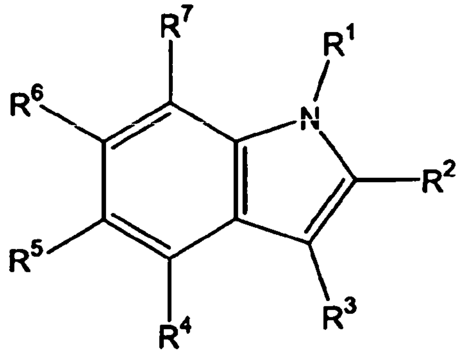

I

où

$R^1$ = H, un radical $C_{1-10}$-alkyle, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle, lié via un groupe $C_{1-6}$-alkylène, non substitué ou substitué par un radical halogène, CN, $CF_3$ ou OH, de préférence H, un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de manière particulièrement préférée H ou un radical $C_{1-2}$-alkyle,

$R^2$ = H, F, Cl, Br, $CF_3$, CN, $NO_2$, $NHR^8$, $SR^9$, $OR^{10}$, $SO_2NH_2$, $SO_2NHR^{21}$, CO ($OR^{11}$), $CH_2CO$ ($OR^{12}$), $COR^{19}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence = H, Cl, F, $NO_2$, $OR^{10}$, CO ($OR^{11}$), un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de manière particulièrement préférée H, un radical $C_{1-2}$-alkyle ou un radical phényle non substitué,

$R^3$ = $CH(R^{13})N(R^{14})(R^{15})$,

$R^4$, $R^5$, $R^6$, $R^7$, sont identiques ou différents et = H, F, Cl, Br, $CF_3$, CN, $NO_2$, $NHR^8$, $SR^9$, $OR^{10}$, $SO_2NH_2$, $SO_2NHR^{21}$,

$CO(OR^{11})$, $CH_2CO(OR^{12})$, $COR^{19}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou un radical phényle, ledit radical phényle ou naphtyle étant le cas échéant condensé avec d'autres cycles, un radical thiophène, pyrrolyle ou furfuryle, non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles ou un radical thiophène, pyrrolyle, furfuryle, lié via un groupe $C_{1-6}$-alkylène, non substitué ou substitué par un radical halogène, CN, $CF_3$ ou OH, de préférence = H, Cl, F, $NO_2$, $OR^{19}$, $CO(OR^{11})$, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de manière particulièrement préférée H, $NO_2$, un radical $C_{1-2}$-alkyle ou un radical phényle non substitué,

$R^8$ = H, $COR^{16}$, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence un radical $C_{1-6}$-alkyle,

$R^9$ = H, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence un radical $C_{1-6}$-alkyle ou phényle,

$R^{10}$ = H, $COR^{17}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence = H, un radical $C_{1-6}$-alkyle ou un radical phényle ou naphtyle lié via un groupe $C_{1-2}$-alkylène non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^{11}$ = H, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence = H ou un radical $C_{1-6}$-alkyle,

$R^{12}$ = H, un radical $C_{1-10}$-alkyle ou un radical phényle ou naphtyle non substitué ou au moins monosubstitué par un radical OH, halogène, $CF_3$, CN, $C_{1-6}$-alkyle, $C_{1-6}$-alcoxy ou phényle , ledit radical phényle ou naphtyle étant le cas échéant condensé avec d'autres cycles, de préférence un radical $C_{1-6}$-alkyle,

$R^{13}$ = un radical phényle ou naphtyle non substitué ou au moins monosubstitué par un radical OH, halogène, $CF_3$, CN, $C_{1-6}$-alkyle, $C_{1-6}$-alcoxy, -O-phényle ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH ou un radical alkyle sans proton acide en position $\alpha$, de préférence un radical phényle non substitué ou un radical phényle, thiophène ou furfuryle au moins monosubstitué par un radical $C_{1-4}$-alkyle, $C_{1-3}$-alcoxy, halogène, $CF_3$, CN, O-phényle ou OH, de manière particulièrement préférée un radical phényle, thiophène ou furfuryle non substitué

ou un radical 2-méthoxyphényle, 3-méthoxyphényle, 4-méthoxyphényle, 2-méthylphényle, 3-méthylphényle, 4-méthylphényle, 2-tert-butylphényle, 3-tert-butylphényle, 4-tert-butylphényle, 2-fluorophényle, 3-fluorophényle, 4-fluorophényle, 2-chlorophényle, 3-chlorophényle, 4-chlorophényle, 2-bromophényle, 3-bromophényle, 4-bromophényle, 5-bromo-2-fluorophényle, 2-chloro-4-fluorophényle, 2-chloro-5-fluorophényle, 2-chloro-6-fluorophényle, 4-bromo-2-fluorophényle, 3-bromo-4-fluorophényle, 3-bromo-2-fluorophényle, 2,3-dichlorophényle, 2,4-dichlorophényle, 2,5-dichlorophényle, 3,4-dichlorophényle, 2,3-diméthylphényle, 2,4-diméthylphényle, 2,5-diméthylphényle, 2,3-diméthoxyphényle, 2,4-diméthoxyphényle, 2,5-diméthoxyphényle, 3,4-diméthoxyphényle, 3,4,5-triméthoxyphényle, 2-trifluorométhylphényle, 3-trifluorométhylphényle ou 4-trifluorométhylphényle, de manière tout particulièrement préférée un radical phényle, thiophène ou furfuryle non substitué,

$R^{14}$, $R^{15}$ sont identiques ou différents et signifient un radical $C_{1-6}$-alkyle ramifié, non ramifié, saturé, insaturé, non substitué ou au moins monosubstitué ou un radical phényle, benzyle ou phénéthyle non substitué ou au moins monosubstitué, de préférence un radical $C_{1-6}$-alkyle saturé, non substitué ou au moins monosubstitué, de manière particulièrement préférée un radical $CH_3$,

ou $R^{14}$ et $R^{15}$ signifient ensemble $(CH_2)_n$, avec n = un nombre entier de 3 à 6 ou $(CH_2)_2O(CH_2)_2$, de préférence $(CH_2)_n$ avec n = 4 ou 5,

$R^{16}$ = un radical $C_{1-10}$-alkyle, un radical phényle ou naphtyle non substitué ou au moins monosubstitué par un radical

OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle non substitué ou substitué par un radical halogène, CN, $CF_3$ ou OH, de préférence un radical $C_{1-6}$-alkyle

$R^{17}$= un radical $C_{1-10}$-alkyle, de préférence un radical $C_{1-6}$-alkyle,

$R^{18}$ = un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle non substitué ou substitué par un radical halogène, CN, $CF_3$ ou OH, de préférence un radical $C_{1-6}$-alkyle,

$R^{19}$ = H, $NHNH_2$, $NHR^{20}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^{20}$ = H, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical thiophène, pyrrolyle ou furfuryle non substitué ou substitué par un radical halogène, CN, $CF_3$ ou OH, un radical phényle ou naphtyle lié via un radical $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, de préférence un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

$R^{21}$ = H, $COR^{17}$, un radical $C_{1-10}$-alkyle, un radical phényle ou naphtyle non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, un radical phényle ou naphtyle lié via un groupe $C_{1-6}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles, ou un radical thiophène, pyrrolyle ou furfuryle lié via un groupe $C_{1-6}$-alkylène, non substitué ou substitué par un radical halogène, CN, $CF_3$ ou OH, de préférence = H, un radical $C_{1-6}$-alkyle ou un radical phényle ou naphtyle lié via un groupe $C_{1-2}$-alkylène, non substitué ou au moins monosubstitué par un radical OH, halogène, $CF_3$, CN, $C_{1-6}$-alkyle, $C_{1-6}$-alcoxy ou phényle, ledit radical phényle ou naphtyle étant le cas échéant condensé avec d'autres cycles,

et/ou leurs racémates, énantiomères, diastéréo-isomères et/ou bases correspondantes et/ou sels correspondants d'acides physiologiquement acceptables,

pour la préparation d'un médicament pour lutter contre les douleurs, de préférence pour lutter contre les douleurs chroniques, pour le traitement de réactions inflammatoires, pour le traitement de réactions allergiques, pour le traitement de l'abus de drogues et/ou d'alcool, pour le traitement de la diarrhée, pour le traitement de la gastrite, pour le traitement d'ulcères, pour le traitement de maladies cardiovasculaires, pour le traitement de l'incontinence urinaire, pour le traitement de dépressions, pour le traitement d'états de choc, pour le traitement de migraines, pour le traitement de la narcolepsie, pour le traitement de l'excès pondéral, pour le traitement de l'asthme, pour le traitement de glaucomes, pour le traitement du syndrome d'hyperkinésie, pour le traitement du manque d'énergie, pour le traitement de la boulimie, pour le traitement de l'anorexie, pour le traitement lors de la catalepsie, pour l'anxiolyse, pour l'augmentation de la vigilance et pour l'augmentation de la libido.

**63.** Utilisation selon la revendication 62, **caractérisée en ce que** la base substituée de Mannich de type indole est choisie dans le groupe formé par

l'ester éthylique de l'acide 3-(diméthylaminophénylméthyl)-1H-indole-2-carboxylique
l'acide 3-(diméthylaminophénylméthyl)-1H-indole-2-carboxylique
la [(5-fluoro-1H-indol-3-yl)-phénylméthyl]-diméthylamine

la [(1-éthyl-2-phényl-1H-indol-3-yl)-phénylméthyl]-diméthylamine
la [(5-méthoxy-1H-indol-3-yl)-phénylméthyl]-diméthylamine
l'acide 3-(diméthylaminophénylméthyl)-5-hydroxy-1H-indole-2-carboxylique
la diméthyl-[(2-méthyl-1H-indol-3-yl)-phénylméthyl]-amine
le 3-(diméthylaminophénylméthyl)-1H-indol-4-ol
la diméthyl-[(4-méthyl-1H-indol-3-yl)-phénylméthyl]-amine
la [(5-chloro-1H-indol-3-yl)-phénylméthyl]-diméthylamine
la [(5-benzyloxy-1H-indol-3-yl)-phénylméthyl]-diméthylamine
l'ester 3-(diméthylaminophénylméthyl)-1H-indol-4-ylique de l'acide acétique
la {[2-(4-chlorophényl)-1H-indol-3-yl]-phénylméthyl}-diméthylamine
l'acide 5-benzyloxy-3-(diméthylaminophénylméthyl)-1H-indole-2-carboxylique
la diméthyl-[(2-méthyl-5-nitro-1H-indol-3-yl)-phénylméthyl]-amine
la diméthyl-[(2-méthyl-6-nitro-1H-indol-3-yl)-phénylméthyl]-amine
la [(6-fluoro-2-méthyl-1H-indol-3-yl)-phénylméthyl]-diméthylamine
la {[2-(4-fluorophényl)-1H-indol-3-yl]-phénylméthyl}-diméthylamine
la {[2-(3-chloro-4-fluorophényl)-1H-indol-3-yl]-phénylméthyl}-diméthylamine
la [(7-éthyl-1H-indol-3-yl)-phénylméthyl]-diméthylamine l'acide 3-(diméthylaminophénylméthyl)-1H-indole-6-carboxylique
la diméthyl-[(1-méthyl-1H-indol-3-yl)-phénylméthyl]-amine
la diméthyl-[(2-phényl-1H-indol-3-yl)-o-toluylméthyl]-amine
la [(5-benzyloxy-1H-indol-3-yl)-o-toluylméthyl]-diméthylamine
la [(2-méthoxyphényl)-(2-méthyl-1H-indol-3-yl)-méthyl]-diméthylamine
la {[1-(4-méthoxybenzyl)-1H-indol-3-yl]-phénylméthyl}-diméthylamine
la [(1H-indol-3-yl)-phénylméthyl]-diméthylamine
la [(5-bromo-2-fluorophényl)-(4-nitro-1H-indol-3-yl)-méthyl]-diméthylamine
la [(5-bromo-2-fluorophényl)-(1-méthyl-1H-indol-3-yl)-méthyl]-diméthylamine
la [(2-chloro-6-fluorophényl)-(2-phényl-1H-indol-3-yl)-méthyl]-diméthylamine
la [(2-chloro-6-fluorophényl)-(1-éthyl-2-phényl-1H-indol-3-yl)-méthyl]-diméthylamine
la [(2-chloro-6-fluorophényl)-(4-nitro-1H-indol-3-yl)-méthyl]-diméthylamine
la [(2-chloro-6-fluorophényl)-(1-méthyl-1H-indol-3-yl)-méthyl]-diméthylamine
la [(2-bromophényl)-(4-nitro-1H-indol-3-yl)-méthyl]-diméthylamine
la [(2-bromophényl)-(1-méthyl-1H-indol-3-yl)-méthyl]-diméthylamine
la [(3-bromophényl)-(7-éthyl-1H-indol-3-yl)-méthyl]-diméthylamine
la [(3-bromophényl)-(1-méthyl-1H-indol-3-yl)-méthyl]-diméthylamine
la [(4-tert-butylphényl)-(2-méthyl-1H-indol-3-yl-)-méthyl]-diméthylamine
la [(4-tert-butylphényl)-(1-éthyl-2-phényl-1H-indol-3-yl)-méthyl]-diméthylamine
la [(4-tert-butylphényl)-(1-méthyl-1H-indol-3-yl)-méthyl]-diméthylamine
la [(2-chloro-4-fluorophényl)-(4-nitro-1H-indol-3-yl)-méthyl]-diméthylamine
la [(2-chloro-4-fluorophényl)-(4-méthyl-1H-indol-3-yl)-méthyl]-diméthylamine
la [(2-chloro-6-fluorophényl)-(2-phényl-1H-indol-3-yl)-méthyl]-diméthylamine
la {(2-chloro-6-fluorophényl)-[2-(4-chlorophényl)-1H-indol-3-yl]-méthyl}-diméthylamine
la [(2-chloro-6-fluorophényl)-(1-méthyl-1H-indol-3-yl)-méthyl]-diméthylamine
la [(3-chlorophényl)-(1-méthyl-1H-indol-3-yl)-méthyl]-diméthylamine
la [(2,3-dichlorophényl)-(1-méthyl-1H-indol-3-yl)-méthyl]-diméthylamine
la [(2,4-dichlorophényl)-(1-méthyl-1H-indol-3-yl)-méthyl]-diméthylamine
la [(4-tert-butylphényl)-(1-éthyl-2-phényl-1H-indol-3-yl)-méthyl]-diméthylamine
la [(2-chloro-4-fluorophényl)-(2-méthyl-1H-indol-3-yl)-méthyl]-diméthylamine
la [(2-chloro-6-fluorophényl)-(2-méthyl-1H-indol-3-yl)-méthyl]-diméthylamine
la [(2,3-diméthoxyphényl)-(4-nitro-1H-indol-3-yl)-méthyl]-diméthylamine
l'acide 3-[(2,3-diméthoxyphényl)-diméthylaminométhyl]-4,7-dihydro-1H-indole-6-carboxylique
l'acide 3-[(2,3-diméthoxyphényl)-diméthylaminométhyl]-5-hydroxy-1H-indole-2-carboxylique
la [(3,4-diméthoxyphényl)-(2-méthyl-1H-indol-3-yl)-méthyl]-diméthylamine
la [[2-(4-chlorophényl)-1H-indol-3-yl]-(3,4-diméthoxyphényl)-méthyl]-diméthylamine
la [(3,4-diméthoxyphényl)-(1-éthyl-2-phényl-1H-indol-3-yl)-méthyl]-diméthylamine
la [(3,4-diméthoxyphényl)-(7-éthyl-1H-indol-3-yl)-méthyl]-diméthylamine
la [(3,4-diméthoxyphényl)-(1-méthyl-1H-indol-3-yl)-méthyl]-diméthylamine
la [(3,4-diméthoxyphényl)-[2-(4-fluorophényl)-1H-indol-3-yl]-méthyl]-diméthylamine
la [[2-(3-chloro-4-fluorophényl)-1H-indol-3-yl]-(3,4-diméthoxyphényl)-méthyl]-diméthylamine

la [(5-chloro-1H-indol-3-yl)-(2-fluorophényl)-méthyl]-diméthylamine
la [(4-fluorophényl)-(4-méthyl-1H-indol-3-yl)-méthyl]-diméthylamine
la [(7-éthyl-1H-indol-3-yl)-(2-méthoxyphényl)-méthyl]-diméthylamine
la diméthyl-[(2-méthyl-1H-indol-3-yl)-o-toluylméthyl]-amine
la [(7-éthyl-1H-indol-3-yl)-o-toluylméthyl]-diméthylamine
la diméthyl-[(1-méthyl-1H-indol-3-yl)-o-toluylméthyl]-amine
la [(5-chloro-1H-indol-3-yl)-o-toluylméthyl]-diméthylamine
la [(5-chloro-1H-indol-3-yl)-m-toluylméthyl]-diméthylamine
la diméthyl-[(2-méthyl-1H-indol-3-yl)-p-toluylméthyl]-amine
la [(5-chloro-1H-indol-3-yl)-p-toluylméthyl]-diméthylamine
la diméthyl-[(2-méthyl-1H-indol-3-yl)-(3-phénoxyphényl)-méthyl]-amine
la [(1-éthyl-2-phényl-1H-indol-3-yl)-(3-phénoxyphényl)-méthyl]-diméthylamine
la [(7-éthyl-1H-indol-3-yl)-(3-phénoxyphényl)-méthyl]-diméthylamine
la diméthyl-[(1-méthyl-1H-indol-3-yl)-(3-phénoxyphényl)-méthyl]-amine
la [[2-(4-fluorophényl)-1H-indol-3-yl]-(3-phénoxyphényl)-méthyl]-diméthylamine
la [[2-(3-chloro-4-fluorophényl)-1H-indol-3-yl]-(3-phénoxyphényl)-méthyl]-diméthylamine
la diméthyl-[(4-méthyl-1H-indol-3-yl)-(3-phénoxyphényl)-méthyl]-amine
la [(5-chloro-1H-indol-3-yl)-(2-trifluorométhylphényl)-méthyl]-diméthylamine
la diméthyl-[(2-méthyl-1H-indol-3-yl)-(4-trifluorométhylphényl)-méthyl]-amine.

**64.** Utilisation selon la revendication 62, **caractérisée en ce que** la base substituée de Mannich de type indole est choisie dans le groupe formé par

le 1-méthyl-3-(morpholin-4-yl-phénylméthyl)-1H-indole
le 3-[(2-méthoxyphényl)-pyrrolidin-1-yl-méthyl]-1-méthyl-1H-indole
le 1-éthyl-2-phényl-3-(pyrrolidin-1-yl-o-toluylméthyl)-1H-indole
le 3-[(2-chlorophényl)-pipéridin-1-yl-méthyl]-1-éthyl-2-phényl-1H-indole
le 1-éthyl-2-phényl-3-(phénylpyrrolidin-1-yl-méthyl)-1H-indole
le 1-éthyl-3-[(2-méthoxyphényl)-pyrrolidin-1-yl-méthyl]-2-phényl-1H-indole
le 2-phényl-3-(pyrrolidin-1-yl-o-toluylméthyl)-1H-indole
le 2-phényl-3-(pipéridin-1-yl-*o*-toluylméthyl)-1H-indole
le 3-[(2-chlorophényl)-pipéridin-1-yl-méthyl]-2-phényl-1H-indole
le 2-phényl-3-(phénylpyrrolidin-1-yl-méthyl)-1H-indole
le 3-[(2-méthoxyphényl)-pyrrolidin-1-yl-méthyl]-2-phényl-1H-indole
le 4-méthyl-3-(pipéridin-1-yl-o-toluylméthyl)-1H-indole
le 3[(2-méthoxyphényl)-pyrrolidin-1-yl-méthyl]-4-méthyl-1H-indole
le 5-benzyloxy-3-(phénylpipéridin-1-yl-méthyl)-1H-indole
le 7-éthyl-3[(2-méthoxyphényl)-morpholin-4-yl-méthyl]-1H-indole
le 3[(2-méthoxyphényl)-pipéridin-1-yl-méthyl]-2-phényl-1H-indole
le 5-chloro-3[(2-méthoxyphényl)-pipéridin-1-yl-méthyl]-1H-indole
le 5-chloro-3-[(2-méthoxyphényl)-pyrrolidin-1-yl-méthyl]-1H-indole
le 5-benzyloxy-3-[(2-méthoxyphényl)-pyrrolidin-1-yl-méthyl]-1H-indole
le 3-(phénylpipéridin-1-yl-méthyl)-1H-indole
le 1-méthyl-3-(phénylpipéridin-1-yl-méthyl)-1H-indole
le 3-(phénylpyrrolidin-1-yl-méthyl)-1H-indole
le 5-benzyloxy-3-(phénylpyrrolidin-1-yl-méthyl)-1H-indole.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3957288 A
- WO 9414770 A
- WO 9414771 A
- US 4855448 A
- EP 0768301 A


### In der Beschreibung aufgeführte Nicht-Patentliteratur


- *J. Pharmacol. Exptl. Ther,* 1993, vol. 267, 33
- *Khim. Geterotsikl. Soedin,* 1983, vol. 1, 49-54
- *J. Am. Chem. Soc.,* 1967, 243-251
- *Synthesis,* 1996, 883-887
- *Synthesis,* 1980, 728-730
- *J. Am. Chem. Soc.,* 1959, 2239-2242
- *Chem. Ber,* 1913, vol. 2144
- **TEZISY DOUL.** *Simp. Khim. Tekhnol. Geterotsihl. Soedin. Gorguch. Iskop,* 1973, 95-96
- **JOHN P. DEVLIN.** High Throughput Screening. Marcel Dekker Inc, 1997
- **M.CH. FRINK ; H.-H.-HENNIES ; W. ENGLBERGER ; M. HAURAND ; B. WILFFERT.** *Arzneim.-Forsch./Drug. Res,* 1996, vol. 46 (11), 1029-1036
- **E.G. GRAY ; V.P. WHITTAKER.** *J. Anat,* 1962, vol. 76, 79-88
- **I.C. HENDERSHOT ; J. FORSAITH.** *J. Pharmacol. Exp. Ther,* 1959, vol. 125, 237-240
- **JOHN P. DEVLIN.** High Throughput Screening. Marcel Dekker Inc, 1997, 307-316